(19) Europäisches Patentamt
European Patent Office
Office européen des brevets

(11) EP 1 579 866 A1

(12) **EUROPEAN PATENT APPLICATION**
published in accordance with Art. 158(3) EPC

(43) Date of publication:
**28.09.2005 Bulletin 2005/39**

(21) Application number: **03778795.9**

(22) Date of filing: **11.12.2003**

(51) Int Cl.[7]: **A61K 38/17**, A61K 31/7088, A61K 39/395, A61K 45/00, A61K 48/00, A61P 13/12

(86) International application number:
**PCT/JP2003/015836**

(87) International publication number:
**WO 2004/052391 (24.06.2004 Gazette 2004/26)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **12.12.2002 JP 2002361415**

(71) Applicant: **Takeda Pharmaceutical Company Limited**
**Osaka 541-0045 (JP)**

(72) Inventors:
- **HINUMA, Shuji**
  **Tsukuba-shi, Ibaraki 300-4293 (JP)**
- **MARUYAMA, Minoru**
  **Tsukuba-shi, Ibaraki 300-4293 (JP)**
- **FUJII, Ryo**
  **Tsukuba-shi, Ibaraki 300-4293 (JP)**

(74) Representative:
**Rickard, Timothy Mark Adrian et al**
**Takeda Euro IP Department,**
**11-12 Charles II Street**
**London SW1Y 4QU (GB)**

# (54) NOVEL USE OF EDG RECEPTORS

(57) By using a G protein-coupled receptor protein containing the same or substantially the same amino acid sequence as the amino acid sequence represented by SEQ ID NO: 1, SEQ ID NO: 5 or SEQ ID NO: 9 (EDG-2 receptor, EDG-3 receptor or EDG-5 receptor) or a salt thereof, prophylactic/therapeutic agents for diabetic nephropathy, etc. can be efficiently screened.

EP 1 579 866 A1

## Description

TECHNICAL FIELD

The present invention relates to endothelial differentiation gene (hereinafter abbreviated as EDG)-2 receptor, EDG-3 receptor or EDG-5 receptor, and use of a polynucleotide encoding these receptors.

BACKGROUND ART

EDG-2 receptors (Biochem. Biophys. Res. Commun., 1997 Feb. 24; 231(3): 619-22), EDG-3 receptors (Cell, 1999 Oct. 29; 99 (3): 301-12), EDG-5 receptors (J. Biol. Chem., 1999 Dec. 10; 274(50): 35343-50) are reported and these receptors are reportedly expressed in vascular tissue.

It is known that lysophosphatidic acid binds to receptors on kidney mesangial cells to activate MAPK together with PDGF and relates to proliferate mesangial cells and EDG-2 receptors and EDG-4 receptors are receptors for lysophosphatidic acid, and lysophosphatidic acid is involved in IgA nephropathy (Clinical Science, 96, 431-436 (1999)).

It is known that sphingosine-1-phosphate binds to EDG-3 receptors or EDG-5 receptors expressed in renal mesangial cells to proliferate mesangial cells and EDG-5 receptors increases in the kidney with IgA nephropathy (The Pharmacogenomics Journal (2001) 1, 211-217).

However, it was unknown that these receptors are involved in diabetic nephropathy.

It is a problem of the present invention to clarify the functions of EDG-2 receptors, EDG-3 receptors or EDG-5 receptors and provide a new use of these receptors.

DISCLOSURE OF THE INVENTION

In order to solve the foregoing problem, the present inventors have made extensive investigations and as a result, have found that a human EDG-2 receptor, a human EDG-3 receptor or a human EDG-5 receptor is overexpressed in human normal mesangial cells and further in the kidney of diabetic nephropathy model rat, an EDG-2 receptor, EDG-3 receptor or EDG-5 receptor is overexpressed. Diabetic nephropathy is nephropathy caused by chronic hyperglycemia, whereas IgA nephropathy is nephropathy induced by hyper-IgA-globulinemia which is induced by infection. Both are therefore clearly different in pathogenesis.

Based on these findings, the present inventors have made further studies and come to accomplish the present invention.

That is, the present invention provides the following features, and the like.

(1) A prophylactic/therapeutic agent for diabetic nephropathy, chronic renal failure, nephritis, glomerulonephritis, interstitial renal disease or renal edema, comprising an EDG-2 receptor comprising the same or substantially the same amino acid sequence as the amino acid sequence represented by SEQ ID NO: 1, an EDG-3 receptor comprising the same or substantially the same amino acid sequence as the amino acid sequence represented by SEQ ID NO: 5, an EDG-5 receptor comprising the same or substantially the same amino acid sequence as the amino acid sequence represented by SEQ ID NO: 9, its partial peptide, or a salt thereof.

(2) A prophylactic/therapeutic agent for diabetic nephropathy, chronic renal failure, nephritis, glomerulonephritis, interstitial renal disease or renal edema, comprising a polynucleotide comprising a polynucleotide encoding an EDG-2 receptor comprising the same or substantially the same amino acid sequence as the amino acid sequence represented by SEQ ID NO: 1, an EDG-3 receptor comprising the same or substantially the same amino acid sequence as the amino acid sequence represented by SEQ ID NO: 5, an EDG-5 receptor comprising the same or substantially the same amino acid sequence as the amino acid sequence represented by SEQ ID NO: 9, or a partial peptide thereof.

(3) A diagnostic agent for diabetic nephropathy, chronic renal failure, nephritis, glomerulonephritis, interstitial renal disease or renal edema, comprising a polynucleotide comprising a polynucleotide encoding an EDG-2 receptor comprising the same or substantially the same amino acid sequence as the amino acid sequence represented by SEQ ID NO: 1, an EDG-3 receptor comprising the same or substantially the same amino acid sequence as the amino acid sequence represented by SEQ ID NO: 5, an EDG-5 receptor comprising the same or substantially the same amino acid sequence as the amino acid sequence represented by SEQ ID NO: 9, or a partial peptide thereof.

(4) A prophylactic/therapeutic agent for diabetic nephropathy, chronic renal failure, nephritis, glomerulonephritis, interstitial renal disease or renal edema, comprising an antibody to an EDG-2 receptor comprising the same or substantially the same amino acid sequence as the amino acid sequence represented by SEQ ID NO: 1, an EDG-3 receptor comprising the same or substantially the same amino acid sequence as the amino acid sequence represented by SEQ ID NO: 5, an EDG-5 receptor comprising the same or substantially the same amino acid sequence as the amino acid sequence represented by SEQ ID NO: 9, its partial peptide, or a salt thereof.

(5) A diagnostic agent for diabetic nephropathy, chronic renal failure, nephritis, glomerulonephritis, interstitial renal disease or renal edema, comprising an antibody to an EDG-2 receptor comprising the same or substantially the same amino acid sequence as the amino acid sequence represented by SEQ ID NO: 1, an EDG-3 receptor comprising the same or substantially the same amino acid sequence as the amino acid sequence represented by SEQ ID NO: 5, an EDG-5 receptor comprising the same or substantially the same amino acid sequence as the amino acid sequence represented by SEQ ID NO: 9, its partial peptide, or a salt thereof.

(6) A prophylactic/therapeutic agent for diabetic nephropathy, chronic renal failure, nephritis, glomerulonephritis, interstitial renal disease or renal edema, comprising a polynucleotide comprising the entire or part of a base sequence complementary to a polynucleotide comprising a polynucleotide encoding an EDG-2 receptor comprising the same or substantially the same amino acid sequence as the amino acid sequence represented by SEQ ID NO: 1, an EDG-3 receptor comprising the same or substantially the same amino acid sequence as the amino acid sequence represented by SEQ ID NO: 5, an EDG-5 receptor comprising the same or substantially the same amino acid sequence as the amino acid sequence represented by SEQ ID NO: 9, or a partial peptide thereof.

(7) A diagnostic agent for diabetic nephropathy, chronic renal failure, nephritis, glomerulonephritis, interstitial renal disease or renal edema, comprising a polynucleotide comprising the entire or part of a base sequence complementary to a polynucleotide comprising a polynucleotide encoding an EDG-2 receptor comprising the same or substantially the same amino acid sequence as the amino acid sequence represented by SEQ ID NO: 1, an EDG-3 receptor comprising the same or substantially the same amino acid sequence as the amino acid sequence represented by SEQ ID NO: 5, an EDG-5 receptor comprising the same or substantially the same amino acid sequence as the amino acid sequence represented by SEQ ID NO: 9, or a partial peptide thereof.

(8) A method of screening a preventive/therapeutic drug for diabetic nephropathy, chronic renal failure, nephritis, glomerulonephritis, interstitial renal disease or renal edema, which comprises using (i) lysophosphatidic acid or a salt thereof, and (ii) an EDG-2 receptor comprising the same or substantially the same amino acid sequence as the amino acid sequence represented by SEQ ID NO: 1, its partial peptide, or a salt thereof, to screen a compound or a salt thereof that changes the binding property of lysophosphatidic acid or a salt thereof and said EDG-2 receptor or a salt thereof.

(9) A kit for screening a preventive/therapeutic drug for diabetic nephropathy, chronic renal failure, nephritis, glomerulonephritis, interstitial renal disease or renal edema, comprising (i) lysophosphatidic acid or a salt thereof, and (ii) an EDG-2 receptor comprising the same or substantially the same amino acid sequence as the amino acid sequence represented by SEQ ID NO: 1, its partial peptide, or a salt thereof.

(10) A method of screening a preventive/therapeutic drug for diabetic nephropathy, chronic renal failure, nephritis, glomerulonephritis, interstitial renal disease or renal edema, which comprises using (i) sphingosine-1-phosphate or a salt thereof, and (ii) an EDG-3 receptor comprising the same or substantially the same amino acid sequence as the amino acid sequence represented by SEQ ID NO: 5, its partial peptide, or a salt thereof, to screen a compound or a salt thereof that changes the binding property of sphingosine-1-phosphate or a salt thereof and said EDG-3 receptor or a salt thereof.

(11) A kit for screening a preventive/therapeutic drug for diabetic nephropathy, chronic renal failure, nephritis, glomerulonephritis, interstitial renal disease or renal edema, comprising (i) sphingosine-1-phosphate or a salt thereof, and (ii) an EDG-3 receptor comprising the same or substantially the same amino acid sequence as the amino acid sequence represented by SEQ ID NO: 5, its partial peptide, or a salt thereof.

(12) A method of screening a preventive/therapeutic drug for diabetic nephropathy, chronic renal failure, nephritis, glomerulonephritis, interstitial renal disease or renal edema, which comprises using (i) sphingosine-1-phosphate or a salt thereof, and (ii) an EDG-5 receptor comprising the same or substantially the same amino acid sequence as the amino acid sequence represented by SEQ ID NO: 9, its partial peptide, or a salt thereof, to screen a compound or a salt thereof that changes the binding property of sphingosine-1-phosphate or a salt thereof and said EDG-5 receptor or a salt thereof.

(13) A kit for screening a preventive/therapeutic drug for diabetic nephropathy, chronic renal failure, nephritis, glomerulonephritis, interstitial renal disease or renal edema, comprising (i) sphingosine-1-phosphate or a salt thereof, and (ii) an EDG-5 receptor comprising the same or substantially the same amino acid sequence as the amino acid sequence represented by SEQ ID NO: 9, its partial peptide, or a salt thereof.

(14) A prophylactic/therapeutic agent for diabetic nephropathy, chronic renal failure, nephritis, glomerulonephritis, interstitial renal disease or renal edema, comprising (i) lysophosphatidic acid or a salt thereof and (ii) a compound (especially, an antagonist) or a salt thereof that changes the binding property of an EDG-2 receptor comprising the same or substantially the same amino acid sequence as the amino acid sequence represented by SEQ ID NO: 1, its partial peptide, or a salt thereof.

(15) A prophylactic/therapeutic agent for diabetic nephropathy, chronic renal failure, nephritis, glomerulonephritis, interstitial renal disease or renal edema, comprising (i) sphingosine-1-phosphate or a salt thereof and (ii) a compound (especially, an antagonist) or a salt thereof that changes the binding property of an EDG-3 receptor comprising the same or substantially the same amino acid sequence as the amino acid sequence represented by SEQ ID NO: 5, its partial peptide, or a salt thereof.

(16) A prophylactic/therapeutic agent for diabetic nephropathy, chronic renal failure, nephritis, glomerulonephritis, interstitial renal disease or renal edema, comprising (i) sphingosine-1-phosphate or a salt thereof and (ii) a compound (especially, an antagonist) or a salt thereof that changes the binding property of an EDG-5 receptor comprising the same or substantially the same amino acid sequence as the amino acid sequence represented by SEQ ID NO: 9, its partial peptide, or a salt thereof.

(17) A method of screening a compound for preventing/treating diabetic nephropathy, chronic renal failure, nephritis, glomerulonephritis, interstitial renal disease or renal edema, or a salt thereof that changes an expression level of an EDG-2 receptor comprising the same or substantially the same amino acid sequence as the amino acid sequence represented by SEQ ID NO: 1, an EDG-3 receptor comprising the same or substantially the same amino acid sequence as the amino acid sequence represented by SEQ ID NO: 5, or an EDG-5 receptor comprising the same or substantially the same amino acid sequence as the amino acid sequence represented by SEQ ID NO: 9, which comprises using a polynucleotide comprising a polynucleotide encoding said EDG-2 receptor, EDG-3 receptor or EDG-5 receptor, or a partial peptide thereof.

(18) A kit for screening a compound for preventing/treating diabetic nephropathy, chronic renal failure, nephritis, glomerulonephritis, interstitial renal disease or renal edema, or a salt thereof that changes an expression level of an EDG-2 receptor comprising the same or substantially the same amino acid sequence as the amino acid sequence represented by SEQ ID NO: 1, an EDG-3 receptor comprising the same or substantially the same amino acid sequence as the amino acid sequence represented by SEQ ID NO: 5, or an EDG-5 receptor comprising the same or substantially the same amino acid sequence as the amino acid sequence represented by SEQ ID NO: 9, comprising a polynucleotide comprising a polynucleotide encoding said EDG-2 receptor, EDG-3 receptor or EDG-5 receptor, or a partial peptide thereof.

(19) A prophylactic/therapeutic agent for diabetic nephropathy, chronic renal failure, nephritis, glomerulonephritis, interstitial renal disease or renal edema, comprising a compound or its salt that changes an expression level (especially, a compound that decreases an expression level) of an EDG-2 receptor comprising the same or substantially the same amino acid sequence as the amino acid sequence represented by SEQ ID NO: 1, an EDG-3 receptor comprising the same or substantially the same amino acid sequence as the amino acid sequence represented by SEQ ID NO: 5, or an EDG-5 receptor comprising the same or substantially the same amino acid sequence as the amino acid sequence represented by SEQ ID NO: 9.

(20) A method for preventing/treating diabetic nephropathy, chronic renal failure, nephritis, glomerulonephritis, interstitial renal disease or renal edema, which comprises administering to a mammal an effective dose of (1) (i) lysophosphatidic acid or a salt thereof and (ii) a compound or a salt thereof that changes the binding property of an EDG-2 receptor comprising the same or substantially the same amino acid sequence as the amino acid sequence represented by SEQ ID NO: 1, its partial peptide, or a salt thereof (especially, an antagonist), (2) (i) sphingosine-1-phosphate or a salt thereof and (ii) a compound or a salt thereof that changes the binding property of an EDG-3 receptor comprising the same or substantially the same amino acid sequence as the amino acid sequence represented by SEQ ID NO: 5, its partial peptide, or a salt thereof (especially, an antagonist), (3) (i) sphingosine-1-phosphate or a salt thereof and (ii) a compound or a salt thereof that changes the binding property of an EDG-5 receptor comprising the same or substantially the same amino acid sequence as the amino acid sequence represented by SEQ ID NO: 9, its partial peptide, or a salt thereof (especially, an antagonist), or (4) a compound or its salt that changes an expression level (especially, a compound that decreases an expression level) of an EDG-2 receptor comprising the same or substantially the same amino acid sequence as the amino acid sequence represented by SEQ ID NO: 1, an EDG-3 receptor comprising the same or substantially the same amino acid sequence as the amino acid sequence represented by SEQ ID NO: 5, or EDG-5 receptor comprising the same or substantially the same amino acid sequence as the amino acid sequence represented by SEQ ID NO: 9.

(21) Use of (1) (i) lysophosphatidic acid or a salt thereof and (ii) a compound or a salt thereof that changes the binding property of EDG-2 receptor comprising the same or substantially the same amino acid sequence as the amino acid sequence represented by SEQ ID NO: 1, its partial peptide, or a salt thereof (especially, an antagonist), (2) (i) sphingosine-1-phosphate or a salt thereof and (ii) a compound or a salt thereof that changes the binding property of an EDG-3 receptor comprising the same or substantially the same amino acid sequence as the amino acid sequence represented by SEQ ID NO: 5, its partial peptide, or a salt thereof (especially, an antagonist), (3) (i) sphingosine-1-phosphate or a salt thereof and (ii) a compound or a salt thereof that changes the binding property of an EDG-5 receptor comprising the same or substantially the same amino acid sequence as the amino acid sequence represented by SEQ ID NO: 9, its partial peptide, or a salt thereof (especially, an antagonist), or (4) a compound or its salt that changes an expression level (especially, a compound that decreases an expression level) of an EDG-2 receptor comprising the same or substantially the same amino acid sequence as the amino acid sequence represented by SEQ ID NO: 1, an EDG-3 receptor comprising the same or substantially the same amino acid sequence as the amino acid sequence represented by SEQ ID NO: 5, or an EDG-5 receptor comprising the same or substantially the same amino acid sequence as the amino acid sequence represented by SEQ ID NO: 9, to manufacture a prophylactic/therapeutic agent for diabetic nephropathy, chronic renal failure, nephritis, glomerulonephritis, interstitial renal disease or renal edema.

BRIEF DESCRIPTION OF THE DRAWINGS

FIG. 1 shows expression of EDG-2 receptor mRNA in the kidney of diabetic nephropathy model rat (Wistar). On the abscissa, Lean, Fatty, 42 wks and 68 wks denote Wistar Lean, Wistar Fatty, 42 weeks old and 68 weeks old, respectively. The ordinate denotes a relative value of the expression level of EDG-2 receptor mRNA to the expression level of GAPDH mRNA.

FIG. 2 shows expression of EDG-3 receptor mRNA in the kidney of diabetic nephropathy model rat (Wistar). On the abscissa, Lean, Fatty, 42 wks and 68 wks denote Wistar Lean, Wistar Fatty, 42 weeks old and 68 weeks old, respectively. The ordinate denotes a relative value of the expression level of EDG-3 receptor mRNA to the expression level of GAPDH mRNA.

FIG. 3 shows expression of EDG-5 receptor mRNA in the kidney of diabetic nephropathy model rat (Wistar). On the abscissa, Lean, Fatty, 42 wks and 68 wks denote Wistar Lean, Wistar Fatty, 42 weeks old and 68 weeks old, respectively. The ordinate denotes a relative value of the expression level of EDG-5 receptor mRNA to the expression level of GAPDH mRNA.

BEST MODE FOR CARRYING OUT THE INVENTION

The EDG-2 receptor used in the present invention is a receptor protein comprising the same or substantially the same amino acid sequence as the amino acid sequence represented by SEQ ID NO: 1.

The EDG-3 receptor used in the present invention is a receptor protein comprising the same or substantially the same amino acid sequence as the amino acid sequence represented by SEQ ID NO: 5.

The EDG-5 receptor used in the present invention is a receptor protein comprising the same or substantially the same amino acid sequence as the amino acid sequence represented by SEQ ID NO: 9.

Hereinafter, the EDG-2 receptor, EDG-3 receptor and EDG-5 receptor are sometimes merely referred to as the EDG receptor.

The EDG receptor may be any protein derived from any cells of human and other mammals (e.g. guinea pig, rat, mouse, rabbit, swine, ovine, bovine, smian, etc.) [for example, retina cells, spleen cells, nerve cells, glial cells, β cells of pancreas, bone marrow cells, mesangial cells, Langerhans' cells, epidermic cells, epithelial cells, endothelial cells, fibroblasts, fibrocytes, myocytes, muscle cells, lipocytes, immune cell (e.g., macrophage, T cells, B cells, natural killer cells, mast cells, neutrophils, basophils, eosinophils, monocytes, leukocytes), megakaryocytes, synovial cells, chondrocytes, bone cells, osteoblasts, osteoclasts, mammary gland cells, hepatocytes or interstitial cells, or the corresponding precursor cells, stem cells, or cancer cells (e.g., breast cancer cell line (GI-101), colon cancer cell line (CX-1, GI-112), lung cancer cell line (LX-1, GI-117), ovary cancer cell line (GI-102), prostate cancer cell line, etc.), etc.], or any tissues where such cells are present, such as brain or any of brain regions (e.g., olfactory bulb, amygdaloid nucleus, basal ganglia, hippocampus, thalamus, hypothalamus, subthalamic nucleus, cerebral cortex, medulla oblongata, cerebellum, occipital lobes, frontal lobe, lateral lobe, putamen, caudate nucleus, corpus callosum, substantia nigra), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, prostate, testis, ovary, placenta, uterus, bone, joint, skeletal muscle, etc.; or proteins derived from hemocyte type cells or their cultured cells (e.g., MEL, M1, CTLL-2, HT-2, WEHI-3, HL-60, JOSK-1, K562, ML-1, MOLT-3, MOLT-4, MOLT-10, CCRF-CEM, TALL-1, Jurkat, CCRT-HSB-2, KE-37, SKW-3, HUT-78, HUT-102, H9, U937, THP-1, HEL, JK-1, CMK, KO-812, MEG-01, etc.); the proteins may also be synthetic proteins. The EDG receptor is overexpressed especially in the kidney or mesangial cells.

In the specification, the "amino acid sequence comprising substantially the same amino acid sequence" is used to mean an amino acid sequence having at least about 50% homology, preferably at least about 60% homology, more preferably at least about 70% homology, further more preferably at least about 80% homology, much more preferably at least about 90% homology and most preferably at least about 95% homology, to the amino acid sequence to be compared.

Homology of the amino acid sequence can be measured under the following conditions (an expectation value = 10; gaps are allowed; matrix = BLOSUM62; filtering = OFF) using a homology scoring algorithm NCBI BLAST (National Center for Biotechnology Information Basic Local Alignment Search Tool).

The EDG-2 receptor comprising substantially the same amino acid sequence as the amino acid sequence represented by SEQ ID NO: 1 preferably includes proteins comprising substantially the same amino acid sequence as the amino acid sequence represented by SEQ ID NO: 1 and having a property substantially equivalent to that of EDG-2 comprising the amino acid sequence represented by SEQ ID NO: 1, etc.

The EDG-3 receptor comprising substantially the same amino acid sequence as the amino acid sequence represented by SEQ ID NO: 5 preferably includes proteins comprising substantially the same amino acid sequence as the amino acid sequence represented by SEQ ID NO: 5 and having a property substantially equivalent to that of EDG-3 receptor comprising the amino acid sequence represented by SEQ ID NO: 5, etc.

The EDG-5 receptor comprising substantially the same amino acid sequence as the amino acid sequence represented by SEQ ID NO: 9 preferably includes proteins comprising substantially the same amino acid sequence as the amino acid sequence represented by SEQ ID NO: 9 and having a property substantially equivalent to that of EDG-5 receptor comprising the amino acid sequence represented by SEQ ID NO: 9, etc.

As the substantially equivalent properties, there are, for example, a ligand binding activity, a signal transduction activity, and the like. The substantially equivalent is used to mean that the nature of these properties is equivalent in terms of quality. Thus, the ligand binding activity, signal transduction activity, etc. is preferably equivalent (e.g., about 0.01 to 100 times, preferably about 0.5 to 20 times, and more preferably 0.5 to 2 times), but differences in degree such as a level of these activities, quantitative factors such as a molecular weight of the protein may be present and allowable.

The ligand binding activity, receptor binding activity, signal transduction activity, etc. can be assayed by publicly known methods with some modifications and can be determined, e.g., by the screening method later described.

Examples of the EDG receptor used include proteins comprising (1) the amino acid sequence represented by SEQ ID NO: 1, SEQ ID NO: 3, SEQ ID NO: 5, SEQ ID NO: 7, SEQ ID NO: 9 or SEQ ID NO: 11, of which at least 1 or 2 (e.g., preferably about 1 to about 30, more preferably about 1 to about 10 and most preferably several (1 to 5)) amino acids are deleted; (2) the amino acid sequence represented by SEQ ID NO: 1, SEQ ID NO: 3, SEQ ID NO: 5, SEQ ID NO: 7, SEQ ID NO: 9 or SEQ ID NO: 11, to which at least 1 or 2 (e.g., preferably about 1 to about 30, more preferably about 1 to about 10 and most preferably several (1 to 5)) amino acids are added; (3) the amino acid sequence represented by SEQ ID NO: 1, SEQ ID NO: 3, SEQ ID NO: 5, SEQ ID NO: 7, SEQ ID NO: 9 or SEQ ID NO: 11, in which at least 1 or 2 (e.g., preferably about 1 to about 30, more preferably about 1 to about 10 and most preferably several (1 to 5)) amino acids are substituted by other amino acids; or (4) a combination of these amino acid sequences; and the like.

Throughout the specification, the EDG receptor is represented in accordance with the conventional way of describing peptides, that is, the N-terminus (amino terminus) at the left hand and the C-terminus (carboxyl terminus) at the right hand. In the EDG receptor, the C-terminus may be in any form of a carboxyl group (-COOH), a carboxylate (-COO-), an amide ($-CONH_2$) and an ester (-COOR).

Herein, examples of the ester group shown by R include a $C_{1-6}$ alkyl group such as methyl, ethyl, n-propyl, isopropyl, n-butyl, etc.; a $C_{3-8}$ cycloalkyl group such as cyclopentyl, cyclohexyl, etc.; a $C_{6-12}$ aryl group such as phenyl, α-naphthyl, etc.; a $C_{7-14}$ aralkyl such as a phenyl-$C_{1-2}$ alkyl group, e.g., benzyl, phenethyl, etc.; an α-naphthy-$C_{1-2}$ alkyl group such as α-naphthylmethyl, etc.; pivaloyloxymethyl generally using as an oral ester and the like.

Where the EDG receptor contains a carboxyl group (or a carboxylate) at a position other than the C-terminus, the carboxyl group may be amidated or esterified and such an amide or ester is also included within the EDG receptor referred to in the present invention. Examples of the ester group in this case may be the C-terminal esters described above, etc.

Furthermore, examples of the EDG receptor include variants wherein the amino group at the N-terminal amino acid residues (e.g., methionine residue) is protected with a protecting group (e.g., a $C_{1-6}$ acyl group such as a $C_{2-6}$ alkanoyl group, e.g., formyl group, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a $C_{1-6}$ acyl group such as a $C_{2-6}$ alkanoyl group, e.g., formyl group, acetyl group, etc.), or conjugated proteins such as glycoproteins to which sugar chains are bound; etc.

Specific examples of the EDG-2 receptor are human EDG-2 receptor consisting of the amino acid sequence represented by SEQ ID NO: 1, rat EDG-2 receptor consisting of the amino acid sequence represented by SEQ ID NO: 3, etc.

Specific examples of the EDG-3 receptor are human EDG-3 receptor consisting of the amino acid sequence represented by SEQ ID NO: 5, rat EDG-3 receptor (fragment) consisting of the amino acid sequence represented by SEQ ID NO: 7, etc.

Specific examples of the EDG-5 receptor are human EDG-5 receptor consisting of the amino acid sequence represented by SEQ ID NO: 9, rat EDG-5 receptor consisting of the amino acid sequence represented by SEQ ID NO: 11, etc.

Human EDG-2 receptor is a known protein described in Genbank accession # U80811, Biochem. Biophys. Res. Commun. 1997 Feb 24; 231(3): 619-22).

Rat EDG-2 receptor is a known protein described in Genbank # NM 053936.

Human EDG-3 receptor is a known protein described in Cell 1999 Oct. 29; 99 (3): 301-12).

Rat EDG-3 receptor (fragment) is a known protein described in Genbank # AF184914).

Human EDG-5 receptor is a known protein described in J. Biol. Chem. 1999 Dec 10; 274 (50): 35343-50).

Rat EDG-5 receptor is a known protein described in Genbank # NM 017192.

The EDG receptor or its salt can be produced from human or mammalian cells or tissues described above by publicly known purification methods of receptor proteins. Alternatively, the EDG receptor may also be produced by culturing a transformant containing DNA encoding the EDG receptor, which will be later described. Furthermore, the EDG receptor may also be produced by the methods for protein synthesis or the methods based on them, which will be later described.

Where these receptors are produced from human or mammalian tissues or cells, human or mammalian tissues or cells are homogenized, extracted with an acid or the like, and the extract is purified and isolated by a combination of chromatography techniques such as reverse phase chromatography, ion exchange chromatography, and the like.

The partial peptides of the EDG receptor (hereinafter sometimes simply referred to as the "partial peptide") may be any peptide so long as it is a peptide having a part of the amino acid sequence of the EDG receptor described above. In the EDG receptor molecules, those being a region exposed outside cell membranes and having a receptor binding activity substantially equivalent to the EDG receptor are preferably used.

Specifically, the partial peptides of EDG-2 receptor consisting of the amino acid sequence represented by SEQ ID NO: 1, EDG-3 receptor consisting of the amino acid sequence represented by SEQ ID NO: 5 or EDG-5 receptor consisting of the amino acid sequence represented by SEQ ID NO: 9 are peptides containing the regions analyzed to be extracellular domains (hydrophilic regions) in the hydrophobic plotting analysis. A peptide which partly contains a hydrophobic region may be used as well. A peptide which separately contains each domain may be used, and the partial peptide which contains plural domains at the same time may be used as well.

In terms of the number of amino acids in the partial peptides, preferred peptides are those having at least 20, preferably at least 50, and more preferably at least 100 amino acids, in the amino acid sequence which constitutes the EDG receptor described above.

The amino acid sequence having substantially the same amino acid sequence includes an amino acid sequence having at least about 70% homology, preferably at least about 80% homology, more preferably at least about 90% homology and most preferably at least about 95% homology, to these amino acid sequences.

Homology of the amino acid sequence can be measured under the following conditions (an expectation value = 10; gaps are allowed; matrix = BLOSUM62; filtering = OFF) using a homology scoring algorithm NCBI BLAST (National Center for Biotechnology Information Basic Local Alignment Search Tool).

Herein, the term "receptor binding activity substantially equivalent" refers to the same significance as defined above. The "receptor binding activity substantially equivalent" can be assayed in the same manner as given above.

The partial peptide may contain an amino acid sequence, wherein at least 1 or 2 amino acids (preferably approximately 1 to 10 amino acids, more preferably several (1 to 5) amino acids) are deleted; to which at least 1 or 2 amino acids (preferably approximately 1 to 20 amino acids, more preferably approximately 1 to 10 amino acids, and most preferably several (1 to 5) amino acids) are added; or, in which at least 1 or 2 amino acids (preferably approximately 1 to 10 amino acids, more preferably several and most preferably approximately 1 to 5 amino acids) are substituted by other amino acids.

In the partial peptide, the C-terminus may be in any form of a carboxyl group (-COOH), carboxylate ($-COO^-$), an amide ($-CONH_2$) or an ester (-COOR).

As in the EDG receptor described above, the partial peptide further includes those in which the amino group of the amino acid residue of the N-terminal methionine residue is protected by a protecting group, those in which the N-terminal residue is cleaved in vivo and the produced glutamine residue is pyroglutaminated, those in which substituents on the side chains of amino acids in the molecule are protected by appropriate protecting groups, conjugated peptides such as so-called glycopeptides, to which sugar chains are bound, and the like.

For salts of the EDG receptor or its partial peptides, preferred are salts with physiologically acceptable acidsor bases, especially physiologically acceptable acid addition salts. Examples of the salts include salts with, for example, inorganic acids (e.g., hydrochloric acid, phosphoric acid, hydrobromic acid, sulfuric acid); salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The partial peptide or its salts of the EDG receptor can be manufactured by publicly known methods for peptide synthesis, or by cleaving the EDG receptor with an appropriate peptidase. In the methods for peptide synthesis, for example, either solid phase synthesis or liquid phase synthesis may be used. That is, the partial peptide or amino acids that can construct the EDG receptor are condensed with the remaining part. Where the product contains protecting groups, these protecting groups are removed to give the desired peptide. Publicly known methods for condensation and elimination of the protecting groups are described in a) through e) below.

a) M. Bodanszky & M.A. Ondetti: Peptide Synthesis, Interscience Publishers, New York (1966)
b) Schroeder & Luebke: The Peptide, Academic Press, New York (1965)
c) Nobuo Izumiya, et al.: *Peptide Gosei-no-Kiso to Jikken* (Basics and experiments of peptide synthesis), published by Maruzen Co. (1975)
d) Haruaki Yajima & Shunpei Sakakibara: *Seikagaku Jikken Koza* (Biochemical Experiment) 1, *Tanpakushitsu no Kagaku* (Chemistry of Proteins) IV, 205 (1977)
e) Haruaki Yajima, ed.: *Zoku Iyakuhin no Kaihatsu* (A sequel to Development of Pharmaceuticals), Vol. 14, Peptide Synthesis, published by Hirokawa Shoten

After completion of the reaction, the product may be purified and isolated by a combination of conventional purification methods such as solvent extraction, distillation, column chromatography, liquid chromatography, recrystallization and the like to give the partial peptide of the present invention. When the partial peptide obtained by the methods above is in a free form, the peptide can be converted into an appropriate salt by a publicly known method; conversely when the peptide is obtained in a salt form, it can be converted into a free form by a publicly known method.
The polynucleotide encoding the EDG receptor may be any polynucleotide so long as it contains the base sequence (DNA or RNA, preferably DNA) encoding the EDG receptor described above. Such a polynucleotide may also be any one of DNA, or RNA such as mRNA, etc. encoding the EDG receptor, and may be double-stranded or single-stranded. Where the polynucleotide is double-stranded, it may be double-stranded DNA, double-stranded RNA or DNA:RNA hybrid. Where the polynucleotide is single-stranded, it may be a sense strand (i.e., a coding strand) or an antisense strand (i.e., a non-coding strand).
Using the polynucleotide encoding the EDG receptor, mRNA of the EDG receptor can be quantified by, for example, the publicly known method published in separate volume of *Jikken Igaku* 15 (7) "New PCR and its application" (1997), or by its modifications.
The DNA encoding the EDG receptor may be any of genomic DNA, genomic DNA library, cDNA derived from the cells and tissues described above, cDNA library derived from the cells and tissues described above and synthetic DNA. The vector to be used for the library may be any of bacteriophage, plasmid, cosmid and phagemid. The DNA may also be directly amplified by reverse transcriptase polymerase chain reaction (hereinafter abbreviated as RT-PCR) using the total RNA or mRNA fraction prepared from the cells and tissues described above.
Specifically, the DNA encoding the EDG-2 receptor may be any DNA, so long as it is, for example, a DNA containing the base sequence represented by SEQ ID NO: 2 or SEQ ID NO: 4, or any DNA hybridizable to a DNA containing the base sequence represented by SEQ ID NO: 2 or SEQ ID NO: 4 under high stringent conditions and encoding a receptor protein which has the activity (e.g., the ligand binding activity, the signal transduction activity, etc.) substantially equivalent to that of the EDG-2 receptor consisting of the amino acid sequence represented by SEQ ID NO: 1 or SEQ ID NO: 3.
The DNA encoding the EDG-3 receptor may be any DNA, so long as it is, for example, a DNA containing the base sequence represented by SEQ ID NO: 6 or SEQ ID NO: 8, or any DNA hybridizable to a DNA containing the base sequence represented by SEQ ID NO: 6 or SEQ ID NO: 8 under high stringent conditions and encoding a receptor protein which has the activity (e.g., the ligand binding activity, the signal transduction activity, etc.) substantially equivalent to that of the EDG-3 receptor consisting of SEQ ID NO: 5 or SEQ ID NO: 7.
The DNA encoding the EDG-5 receptor may be any DNA, so long as it is, for example, a DNA containing the base sequence represented by SEQ ID NO: 10 or SEQ ID NO: 12, or any DNA hybridizable to a DNA containing the base sequence represented by SEQ ID NO: 10 or SEQ ID NO: 12 under high stringent conditions and encoding a receptor protein which has the activity substantially equivalent to that of the EDG-5 receptor consisting of SEQ ID NO: 9 or SEQ ID NO: 11 (e.g., the ligand binding activity, the signal transduction activity, etc.).
Examples of the DNA hybridizable to a DNA containing the base sequence represented by SEQ ID NO: 2, SEQ ID NO: 4, SEQ ID NO: 6, SEQ ID NO: 8, SEQ ID NO: 10 or SEQ ID NO: 12 under highly stringent conditions include a DNA containing a base sequence having at least about 70% homology, preferably at least about 80% homology, more preferably at least about 90% homology and most preferably at least about 95% homology, to the base sequence represented by SEQ ID NO: 4, SEQ ID NO: 6, SEQ ID NO: 8, SEQ ID NO: 10 or SEQ ID NO: 12.
Homology in the base sequence can be measured under the following conditions (an expectation value = 10; gaps are allowed; filtering = ON; match score = 1; mismatch score = -3) using the homology scoring algorithm NCBI BLAST (National Center for Biotechnology Information Basic Local Alignment Search Tool).
The hybridization can be carried out by publicly known methods or by modifications of these methods, for example, according to the method described in Molecular Cloning, 2nd (J. Sambrook et al., Cold Spring Harbor Lab. Press, 1989). A commercially available library may also be used according to the instructions of the attached manufacturer's protocol. Preferably, the hybridization can be carried out under highly stringent conditions.

The highly stringent conditions used herein are, for example, those in a sodium concentration at about 19 to 40 mM, preferably about 19 to 20 mM at a temperature of about 50 to 70°C, preferably about 60 to 65°C. In particular, hybridization conditions in a sodium concentration of about 19 mM at a temperature of about 65°C are most preferred.

More specifically, as the DNA encoding the human EDG-2 receptor consisting of the amino acid sequence represented by SEQ ID NO: 1, there may be employed a DNA consisting of the base sequence represented by SEQ ID NO: 2; etc.

As the DNA encoding the rat EDG-2 receptor consisting of the amino acid sequence represented by SEQ ID NO: 3, there may be employed a DNA consisting of the base sequence represented by SEQ ID NO: 4; etc.

As the DNA encoding the rat EDG-2 receptor consisting of the amino acid sequence represented by SEQ ID NO: 3, there may be employed a DNA consisting of the base sequence represented by SEQ ID NO: 4; etc.

As the DNA encoding the human EDG-3 receptor consisting of the amino acid sequence represented by SEQ ID NO: 5, there may be employed a DNA consisting of the base sequence represented by SEQ ID NO: 6; etc.

As the DNA encoding the rat EDG-3 receptor consisting of the amino acid sequence represented by SEQ ID NO: 7, there may be employed a DNA consisting of the base sequence represented by SEQ ID NO: 8; etc.

As the DNA encoding the human EDG-5 receptor consisting of the amino acid sequence represented by SEQ ID NO: 9, there may be employed a DNA consisting of the base sequence represented by SEQ ID NO: 10; etc.

As the DNA encoding the rat EDG-5 receptor consisting of the amino acid sequence represented by SEQ ID NO: 11, there may be employed a DNA consisting of the base sequence represented by SEQ ID NO: 12; etc.

The term "polynucleotide comprising a part of the base sequence of the DNA encoding the EDG receptor or a part of the base sequence complementary to the DNA is used to mean that the polynucleotide" embraces not only the DNA encoding the partial peptide of the EDG receptor described below but also RNA.

According to the present invention, antisense polynucleotides (nucleic acids) that can inhibit the replication or expression of a gene for the EDG receptor can be designed and synthesized based on the base sequence information of the cloned or determined DNA encoding the EDG receptor. Such a polynucleotide (nucleic acid) is capable of hybridizing to RNA of the EDG receptor gene to inhibit the synthesis or function of said RNA or capable of modulating or controlling the expression of the EDG receptor gene via interaction with the EDG receptor-associated RNA. Polynucleotides complementary to the selected sequences of RNA associated with the EDG receptor and polynucleotides specifically hybridizable to RNA associated with the EDG receptor are useful in modulating or controlling expression of the EDG receptor gene in vivo and in vitro, and useful for the treatment or diagnosis of diseases. The term "corresponding" is used to mean homologous to or complementary to a particular sequence of the nucleotide, base sequence or nucleic acid including the gene. The term "corresponding" between nucleotides, base sequences or nucleic acids and peptides (proteins) usually refer to amino acids of a peptide (protein) under the order derived from the sequence of nucleotides (nucleic acids) or their complements. In the genes for the receptor protein, the 5' end hairpin loop, 5' end 6-base-pair repeats, 5' end untranslated region, polypeptide translation initiation codon, protein coding region, ORF translation initiation codon, 3' end untranslated region, 3' end palindrome region, and 3' end hairpin loop, may be selected as preferred target regions, though any other region may be selected as a target in the genes for the receptor protein.

For cloning of the DNA that completely encodes the EDG receptor or its partial peptide (hereinafter sometimes collectively referred to as the EDG receptor), the DNA may be amplified by PCR using synthetic DNA primers containing a part of the base sequence of the EDG receptor, or the DNA inserted into an appropriate vector can be selected by hybridization with a labeled DNA fragment or synthetic DNA that encodes a part or entire region of the EDG receptor. The hybridization can be carried out, for example, according to the method described in Molecular Cloning, 2nd, J. Sambrook et al., Cold Spring Harbor Lab. Press, 1989. The hybridization may also be performed using commercially available library in accordance with the protocol described in the attached instructions. When the commercially available library is used, cloning may be performed according to the methods described in the protocols attached thereto.

(Antisense polynucleotide)

The relationship between the targeted nucleic acids and the polynucleotides complementary to at least a part of the target, specifically the relationship between the target and the polynucleotides hybridizable to the target, can be denoted to be "antisense." Examples of the antisense polynucleotides include polydeoxyribonucleotide containing 2-deoxy-D-ribose, polyribonucleotide containing D-ribose, any other type of polynucleotides which are N-glycosides of a purine or pyrimidine base, or other polymers containing non-nucleotide backbones (e.g., protein nucleic acids and synthetic sequence-specific nucleic acid polymers commercially available) or other polymers containing nonstandard linkages (provided that the polymers contain nucleotides having such a configuration that allows base pairing or base stacking, as is found in DNA or RNA), etc. They may be double-stranded DNA, single-stranded DNA, double-stranded RNA, single-stranded RNA or a DNA:RNA hybrid, and may further include unmodified polynucleotides (or unmodified oligonucleotides), those with publicly known types of modifications, for example, those with labels known in the art, those with caps, methylated polynucleotides, those with substitution of one or more naturally occurring nucleotides by their analogue, those with intramolecular modifications of nucleotides such as those with uncharged linkages (e.g., methyl phosphonates, phosphotriesters, phosphoramidates, carbamates, etc.) and those with charged linkages or sulfur-containing linkages (e.g., phosphorothioates, phosphorodithioates, etc.), those having side chain groups such as proteins (nucleases, nuclease inhibitors, toxins, antibodies, signal peptides, poly-L-lysine, etc.), saccharides (e.g., monosaccharides, etc.), those with intercalators (e.g., acridine, psoralen, etc.), those containing chelators (e.g., metals, radioactive metals, boron, oxidative metals, etc.), those containing alkylating agents, those with modified linkages (e.g., $\alpha$ anomeric nucleic acids, etc.), and the like. Herein the terms "nucleoside", "nucleotide" and "nucleic acid" are used to refer to moieties that contain not only the purine and pyrimidine bases, but also other heterocyclic bases, which have been modified. Such modifications may include methylated purines and pyrimidines, acylated purines and pyrimidines and other heterocyclic rings. Modified nucleotides and modified nucleotides also include modifications on the sugar moiety, wherein, for example, one or more hydroxyl groups may optionally be substituted with a halogen atom(s), an aliphatic group(s), etc., or may be converted into the corresponding functional groups such as ethers, amines, or the like.

The antisense polynucleotide (nucleic acid) of the present invention is RNA, DNA or a modified nucleic acid (RNA, DNA). Specific examples of the modified nucleic acid are, but not limited to, sulfur and thiophosphate derivatives of nucleic acids and those resistant to degradation of polynucleoside amides or oligonucleoside amides. The antisense nucleic acids of the present invention can be modified preferably based on the following design, that is, by increasing the intracellular stability of the antisense nucleic acid, increasing the cellular permeability of the antisense nucleic acid, increasing the affinity of the nucleic acid to the targeted sense strand to a higher level, or minimizing the toxicity, if any, of the antisense nucleic acid.

Many of such modifications are known in the art, as disclosed in J. Kawakami, et al., Pharm. Tech. Japan, Vol. 8, pp. 247, 1992; Vol. 8, pp. 395, 1992; S. T. Crooke, et al. ed., Antisense Research and Applications, CRC Press, 1993; etc.

The antisense nucleic acid of the present invention may contain changed or modified sugars, bases or linkages. The antisense nucleic acid may also be provided in a specialized form such as liposomes, microspheres, or may be applied to gene therapy, or may be provided in combination with attached moieties. Such attached moieties include polycations such as polylysine that acts as charge neutralizers of the phosphate backbone, or hydrophobic moieties such as lipids (e.g., phospholipids, cholesterols, etc.) that enhance the interaction with cell membranes or increase uptake of the nucleic acid. Preferred examples of the lipids to be attached are cholesterols or derivatives thereof (e.g., cholesteryl chloroformate, cholic acid, etc.). These moieties may be attached to the nucleic acid at the 3' or 5' ends thereof and may also be attached thereto through a base, sugar, or intramolecular nucleoside linkage. Other moieties may be capping groups specifically placed at the 3' or 5' ends of the nucleic acid to prevent degradation by nucleases such as exonuclease, RNase, etc. Such capping groups include, but are not limited to, hydroxyl protecting groups known in the art, including glycols such as polyethylene glycol, tetraethylene glycol and the like.

The inhibitory action of the antisense nucleic acid can be examined using the transformant of the present invention, the gene expression system of the present invention in vivo and in vitro, or the translation system of receptor proteins in vivo and in vitro. The nucleic acid can be applied to cells by a variety of publicly known methods.

The antisense polynucleotide of the present invention can suppress functions of the EDG receptor or the polynucleotide (e.g., DNA) of the present invention in the living body and are thus usable as an agent for preventing/treating diseases associated with dysfunction of, e.g., the EDG receptor. In addition, the antisense polynucleotides of the present invention can also be used as oligonucleotide probes for diagnosis to investigate the presence of the DNA of the present invention or the state of its expression in tissues or cells. Accordingly, the antisense polynucleotides can be used for diagnosis of diseases associated with dysfunction of the EDG receptor.

(siRNA)

The siRNA to the polynucleotide encoding the EDG receptor (hereinafter the siRNA of the present invention) is a double-stranded RNA containing a part of RNA encoding the EDG receptor and RNA complementary thereto.

The siRNA can be designed based on a sequence of the polynucleotide of the present invention and manufactured by modifications of publicly known methods (e.g., Nature, 411, p. 494, 2001).

The ribozyme containing a part of the RNA encoding the EDG receptor can be designed based on a sequence of the polynucleotide of the present invention and manufactured by modifications of publicly known methods (e.g., TRENDS in Molecular Medicine, 7, p. 221, 2001). For example, the ribozyme can be manufactured by replacing a part of the sequence of a publicly known ribozyme with a part of the RNA encoding the EDG receptor. Such a part of the RNA encoding the EDG receptor includes a sequence proximal to a consensus sequence NUX (wherein N represents all bases and X represents bases other than G), which may be cleaved by a publicly known ribozyme.

(DNA encoding the partial peptide)

The DNA encoding the partial peptide of the EDG receptor may be any DNA so long as it contains the base sequence encoding the partial peptide of the EDG receptor described above. The DNA may also be any of genomic DNA, genomic DNA library, cDNA derived from the cells and tissues described above, cDNA library derived from the cells and tissues described above and synthetic DNA. The vector to be used for the library may be any of bacteriophage, plasmid, cosmid and phagemid. The DNA may also be directly amplified by reverse transcriptase polymerase chain reaction (hereinafter abbreviated as RT-PCR) using mRNA fraction prepared from the cells and tissues described above.

Specific examples of the DNA encoding the partial peptide of EDG-2 receptor used include:

(1) a DNA having a partial base sequence of the DNA containing the base sequence represented by SEQ ID NO: 2 or SEQ ID NO: 4;
(2) a DNA having a partial base sequence of the DNA hybridizable to a DNA containing the base sequence represented by SEQ ID NO: 2 or SEQ ID NO: 4 under highly stringent conditions and encoding the receptor protein which has the activity (e.g., the ligand biding activity, the signal transduction activity, etc.) substantially equivalent to those of the EDG-2 receptor consinsting of the amino acid sequence represented by SEQ ID NO: 1 or SEQ ID NO: 3; and the like.

Examples of the DNA encoding the partial peptide of the EDG-3 receptor used include:

(1) a DNA having a partial base sequence of the DNA containing the base sequence represented by SEQ ID NO: 6 or SEQ ID NO: 8;
(2) a DNA having a partial base sequence of the DNA hybridizable to a DNA containing the base sequence represented by SEQ ID NO: 6 or SEQ ID NO: 8 under highly stringent conditions and encoding the receptor protein which has the activity (e.g., the ligand biding activity, the signal transduction activity, etc.) substantially equivalent to those of the EDG-3 receptor consinsting of the amino acid sequence represented by SEQ ID NO: 5 or SEQ ID NO: 7; and the like.

Examples of the DNA encoding the partial peptide of the EDG-5 receptor used include:

(1) a DNA having a partial base sequence of the DNA containing the base sequence represented by SEQ ID NO: 10 or SEQ ID NO: 12;
(2) a DNA having a partial base sequence of the DNA hybridizable to a DNA containing the base sequence represented by SEQ ID NO: 10 or SEQ ID NO: 12 under highly stringent conditions and encoding the receptor protein which has the activity (e.g., the ligand biding activity, the signal transduction activity, etc.) substantially equivalent to those of the EDG-5 receptor consinsting of the amino acid sequence represented by SEQ ID NO: 9 or SEQ ID NO: 11; and the like.

Examples of the DNA that is hybridizable to a DNA containing the base sequence represented by SEQ ID NO: 2, SEQ ID NO: 4, SEQ ID NO: 6, SEQ ID NO: 8, SEQ ID NO: 10 or SEQ ID NO: 12 under highly stringent conditions include a DNA containing a base sequence having at least about 70% homology, preferably at least about 80% homology, more preferably at least about 90% homology and most preferably at least about 95% homology, to the base sequence represented by SEQ ID NO: 2, SEQ ID NO: 4, SEQ ID NO: 6, SEQ ID NO: 8, SEQ ID NO: 10 or SEQ ID NO: 12.

Homology of the base sequences can be measured under the same conditions as described above using a homology scoring algorithm NCBI BLAST (National Center for Biotechnology Information Basic Local Alignment Search Tool).

Methods for the hybridization and the high stringent conditions are the same as those described above.

Antibodies to the EDG receptor, its partial peptides or salts thereof (hereinafter sometimes simply referred to as the EDG receptor) may be any of polyclonal antibodies and monoclonal antibodies, as long as they are capable of recognizing the EDG receptor or cells containing the EDG receptor.

The antibodies to the EDG receptor may be manufactured by publicly known methods for manufacturing antibodies or antisera, using the EDG receptor as an antigen.

[Preparation of monoclonal antibody]

(a) Preparation of monoclonal antibody-producing cells

The EDG receptor is administered to mammals either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once in every two to six weeks and 2 to 10 times in total. Examples of the applicable mammals are smian, rabbits, canine, guinea pigs, mice, rats, sheep and goats, with mice and rats being preferred.

In the preparation of monoclonal antibody-producing cells, warm-blooded animals, e.g., mice, immunized with an antigen wherein the antibody titer is noted is selected, then the spleen or lymph node is collected after 2 to 5 days from the final immunization and antibody-producing cells contained therein are fused with myeloma cells to give monoclonal antibody-producing hybridomas. Measurement of the antibody titer in antisera may be made, for example, by reacting a labeled form of the receptor protein, which will be described later, with the antiserum followed by assaying the binding activity of the labeling agent bound to the antibody. The fusion manipulation may be operated, for example, by the known Koehler and Milstein method [Nature, 256, p. 495 (1975)]. Examples of the fusion accelerator are polyethylene glycol (PEG), Sendai virus, etc., of which PEG is preferably employed.

Examples of the myeloma cells are NS-1, P3U1, SP2/0, etc. In particular, P3U1 is preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by incubating at about 20 to about 40°C, preferably at about 30 to about 37°C for about 1 to about 10 minutes, a cell fusion can be efficiently carried out.

Various methods can be used for screening of a monoclonal antibody-producing hybridoma. Examples of such methods include a method which comprises adding the supernatant of hybridoma to a solid phase (e.g., microplate) adsorbed with an antigen of the receptor protein directly or together with a carrier, adding an anti-immunoglobulin antibody (when mouse cells are used for the cell fusion, anti-mouse immunoglobulin antibody is used) labeled with a radioactive substance or an enzyme, or Protein A and detecting the monoclonal antibody bound to the solid phase, and a method which comprises adding the supernatant of hybridoma to a solid phase adsorbed with an anti-immunoglobulin antibody or Protein A, adding the receptor protein labeled with a radioactive substance or an enzyme and detecting the monoclonal antibody bound to the solid phase.

The monoclonal antibody can be selected by publicly known methods or by modifications of these methods. In general, the selection can be effected in a medium for animal cells supplemented with HAT (hypoxanthine, aminopterin and thymidine). Any selection and growth medium can be employed as far as the hybridoma can grow therein. For example, RPMI 1640 medium containing 1% to 20%, preferably 10% to 20% fetal bovine serum, GIT medium (Wako Pure Chemical Industries, Ltd.) containing 1% to 10% fetal bovine serum, a serum free medium for cultivation of a hybridoma (SFM-101, Nissui Seiyaku Co., Ltd.) and the like can be used for the selection and growth medium. The cultivation is carried out generally at 20°C to 40°C, preferably at about 37°C, for 5 days to 3 weeks, preferably 1 to 2 weeks. The cultivation can be conducted normally in 5% $CO_2$. The antibody titer of the culture supernatant of hybridomas can be determined as in the assay for the antibody titer in antisera described above.

(b) Purification of monoclonal antibody

Separation and purification of a monoclonal antibody can be carried out by methods applied to conventional separation and purification of immunoglobulins, as in the conventional methods for separation and purification of polyclonal antibodies [e.g., salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A, Protein G, etc. and dissociating the binding to obtain the antibody].

[Preparation of polyclonal antibody]

The polyclonal antibody of the present invention can be manufactured by publicly known methods or modifications thereof. For example, a complex of immunogen (EDG receptor, etc. as an antigen) and a carrier protein is prepared, and a mammal is immunized with the complex in a manner similar to the method described above for the manufacture of monoclonal antibodies. The product containing the antibody to the EDG receptor is collected from the immunized animal followed by separation and purification of the antibody.

In the complex of an immunogen and a carrier protein used to immunize a mammal, the type of carrier protein and the mixing ratio of a carrier to hapten may be any type and in any ratio, as long as the antibody is efficiently produced to the hapten immunized by crosslinking to the carrier. For example, bovine serum albumin, bovine thyroglobulins, keyhole limpet hemocyanin, etc. is coupled to hapten in a carrier-to-hapten weight ratio of approximately 0.1 to 20, preferably about 1 to about 5.

A variety of condensing agents can be used for the coupling of a carrier to hapten. Glutaraldehyde, carbodiimide, maleimide activated ester, activated ester reagents containing thiol group or dithiopyridyl group, etc. are used for the coupling.

The condensation product is administered to warm-blooded animals either solely or together with carriers or diluents to the site in which the antibody can be produced by the administration. In order to potentiate the antibody productivity upon the administration, complete Freund's adjuvant or incomplete Freund's adjuvant may be administered. The administration is usually made once approximately in every 2 to 6 weeks and about 3 to about 10 times in total.

The polyclonal antibody can be collected from the blood, ascites, etc., preferably from the blood of mammals immunized by the method described above.

The polyclonal antibody titer in antiserum can be assayed by the same procedure as that for the determination of serum antibody titer described above. The separation and purification of the polyclonal antibody can be carried out, following the method for the separation and purification of immunoglobulins performed as applied to the separation and purification of monoclonal antibodies described hereinabove.

[Application of the receptor protein, DNA, etc.]

The EDG receptor, the DNA encoding the EDG receptor (hereinafter sometimes referred to briefly as the DNA of the present invention), the antibody to the EDG receptor (hereinafter sometimes referred to briefly as the antibody of the present invention) and the antisense DNA to the DNA of the present invention (hereinafter sometimes merely referred to as the antisense DNA of the present invention) have the following applications.

(1) Agent for preventing/treating diseases associated with dysfunction of the EDG receptor

The EDG receptor is overexpressed in mesangial cells or diabetic nephropathy model rats. Accordingly, a) the EDG receptor or b) the DNA encoding the EDG receptor can be used as pharmaceuticals for preventing/treating diseases associated with dysfunction of the EDG receptor, especially for diabetic nephropathy, chronic renal failure, nephritis, glomerulonephritis, interstitial renal disease, renal edema, etc.

For example, when the physiological activity of ligand cannot be expected in a patient (deficiency of the EDG receptor) due to a decrease of the EDG receptor in the body, the amount of the EDG receptor can be increased in the body of the patient a) by administering the EDG receptor to the patient thereby to supplement the amount of the EDG receptor; or b) (i) by administering the DNA encoding the EDG receptor to the patient and expressing the same, or (ii) by inserting and expressing the DNA encoding the EDG receptor in the target cells and then transplanting the cells to the patient, thus increasing the amount of the EDG receptor in the body of the patient, whereby the activities of the ligand can be sufficiently exhibited.

That is, the EDG receptor or the DNA of the present inevention is useful as a safe and low toxic prophylactic/therapeutic agent for diseases associated with dysfunction of the EDG receptor, especially for diabetic nephropathy, chronic renal failure, nephritis, glomerulonephritis, interstitial renal disease or renal edema.

When the EDG receptor is used as the prophylactic/therapeutic agent described above, the EDG receptor can be prepared into a pharmaceutical composition in a conventional manner.

On the other hand, where the DNA of the present invention is used as the prophylactic/therapeutic agent described above, the DNA itself is administered; alternatively, the DNA is inserted into an appropriate vector such as retrovirus vector, adenovirus vector, adenovirus-associated virus vector, etc. and then administered in a conventional manner. The DNA of the present invention may also be administered as naked DNA, or with adjuvants to assist its uptake by gene gun or through a catheter such as a catheter with a hydrogel.

For example, a) the EDG receptor or b) the DNA of the present invention can be used orally, for example, in the form of tablets which may be sugar coated if necessary, capsules, elixirs, microcapsules etc., or parenterally in the form of injectable preparations such as a sterile solution and a suspension in water or with other pharmaceutically acceptable liquid. These preparations can be manufactured by mixing a) the EDG receptor or b) the DNA of the present invention with a physiologically acceptable known carrier, a flavoring agent, an excipient, a vehicle, an antiseptic agent, a stabilizer, a binder, etc. in a unit dosage form required in a generally accepted manner that is applied to making pharmaceutical preparations. The effective component in the preparation is controlled in such an amount that an appropriate dose is obtained within the specified range given.

Additives miscible with tablets, capsules, etc. include a binder such as gelatin, corn starch, tragacanth and gum arabic, an excipient such as crystalline cellulose, a swelling agent such as corn starch, gelatin and alginic acid, a lubricant such as magnesium stearate, a sweetening agent such as sucrose, lactose and saccharin, and a flavoring agent such as peppermint, akamono oil and cherry. When the unit dosage is in the form of capsules, liquid carriers such as oils and fats may further be used together with the additives described above. A sterile composition for injection may be formulated by conventional procedures used to make pharmaceutical compositions, e.g., by dissolving or suspending the active ingredients in a vehicle such as water for injection with a naturally occurring vegetable oil such as sesame oil and coconut oil, etc. to prepare the pharmaceutical composition. Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol or the like), a polyalcohol (e.g., propylene glycol and polyethylene glycol), a nonionic surfactant (e.g., polysorbate 80™ and HCO-50), etc. Examples of the oily medium include sesame oil and soybean oil, which may also be used in combination with a dissolution aid such as benzyl benzoate, benzyl alcohol, etc.

The prophylactic/therapeutic agents described above may further be formulated with a buffer (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus-prepared liquid for injection is normally filled in an appropriate ampoule.

Since the thus obtained pharmaceutical preparation is safe and low toxic, the preparation can be administered to human or mammals (e.g., rats, mice, rabbits, ovine, swine, bovine, feline, canine, simian, etc.).

The dose of the EDG receptor varies depending on subject to be administered, organs to be administered, conditions, methods for administration, etc.; in oral administration, e.g., for the patient with diabetic nephropathy, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, methods for administration, etc. but it is advantageous, e.g., for the patient with diabetic nephropathy, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

The dose of the DNA of the present invention varies depending on subject to be administered, organs to be administered, conditions, methods for administration, etc.; in oral administration, e.g., for the patient with diabetic nephropathy, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, methods for administration, etc. but it is advantageous, e.g., for the patient with diabetic nephropathy, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

(2) Gene diagnostic agent and method for diagnosis using the DNA or antisense DNA of the present invention

By using the DNA or antisense DNA of the present invention as probes, abnormalities (gene abnormalities) of the DNA or mRNA encoding the EDG receptor or its partial peptides in human or mammals (e.g., rats, mice, rabbits, ovine, swine, bovine, feline, canine, simian, etc.) can be detected, and the DNA and antisense DNA are thus useful as gene diagnostic agents for the damage against the DNA or mRNA, its mutation, or its reduced expression, or increased expression or overexpression of the DNA or mRNA.

For example, the DNA or antisense DNA of the present invention can be used as a diagnostic agent for diabetic nephropathy, chronic renal failure, nephritis, glomerulonephritis, interstitial renal disease or renal edema.

The gene diagnosis described above using the DNA or antisense DNA of the present invention can be performed by, for example, the publicly known Northern hybridization assay or the PCR-SSCP assay (Genomics, 5, 874-879 (1989); Proceedings of the National Academy of Sciences of the United States of America, 86, 2766-2770 (1989)), etc.

Where the reduced expression of overexpression of the EDG receptor is detected, e.g., by Northern hybridization, it can be diagnosed that one suffers from, for example, diseases caused by dysfunction or overexpression of the EDG receptor, especially diabetic nephropathy, chronic renal failure, nephritis, glomerulonephritis, interstitial renal disease, renal edema, etc., or it is highly likely to suffer from these disease in the future.

(3) Pharmaceuticals comprising the antisense DNA or siRNA of the present invention

The antisense DNA or siRNA of the present invention can be used as the prophylactic/therapeutic agent for diseases caused by overexpression of the EDG receptor, etc. (e.g., diabetic nephropathy, chronic renal failure, nephritis, glomerulonephritis, interstitial renal disease or renal edema) or the like.

Where the antisense DNA or siRNA is used, the antisense DNA or siRNA itself is administered; alternatively, the antisense DNA or siRNA is inserted into an appropriate vector such as retrovirus vector, adenovirus vector, adenovirus-associated virus vector, etc. and then administered in a conventional manner. The antisense DNA or siRNA may also be administered in an intact form, or with adjuvants to assist its uptake by gene gun or through a catheter such as a catheter with a hydrogel.

In addition, the antisense DNA can also be used as a diagnostic oligonucleotide probe to investigate presence of the DNA of the present invention in tissues or cells or the state of its expression.

(4) Method for screening the compound or its salt that changes the expression level of the EDG receptor

By using the DNA of the present invention as a probe, the DNA can be used for screening the compound or its salt that changes the expression level of the EDG receptor.

That is, the present invention provides a method for screening the compound or its salt that changes the expression level of the EDG receptor, which comprises measuring the amount of mRNA in the EDG receptor contained, for example, tissues or cells isolated from the in (i) (1) blood, (2) particular organs, (3) organs of non-human mammals or in (ii) transformants, etc.

The amount of mRNA in the EDG receptor can be specifically measured as follows.

(i) Normal or disease models of non-human mammals (e.g., mice, rats, rabbits, ovine, swine, bovine, feline, canine, simian, more specifically, tumor-bearing mice, etc.) are given a drug (e.g., an anticancer agent, etc.) or physical stress (e.g., soaking stress, electric shock, light and darkness, low temperature, etc.), and blood, particular organs (e.g., brain, lung, colon, prostate, etc.), or tissues or cells isolated from the organs are obtained after a specified period of time.

The mRNA in the protein of the present invention contained in the thus obtained cells is extracted from the cells, for example, in a conventional manner and quantified by means of, e.g., TaqManPCR, etc., or may also be analyzed by the Northern blot technique by publicly known methods.

(ii) Transformants that express the EDG receptor are prepared according to the methods described above, and the mRNA in the EDG receptor contained in the transformants can be quantified and analyzed, as described above.

The compound that changes the expression level of the EDG receptor can be screened by the following procedures.

(i) To normal or disease models of non-human mammals, a test compound is administered at a given period of time before (30 minutes to 24 hours before, preferably 30 minutes to 12 hours before, more preferably 1 hour to 6 hours before), at a given period of time after (30 minutes to 3 days after, preferably 1 hour to 2 days after, more preferably 1 hour to 24 hours after), or simultaneously with a drug or physical stress. At a given period of time (30 minute to 3 days, preferably 1 hour to 2 days, more preferably 1 hour to 24 hours) after administration of the test compound, the amount of mRNA in the EDG receptor contained in cells are quantified and analyzed.

(ii) Transformants are cultured in a conventional manner and a test compound is mixed in the culture medium. After a given period of time (after 1 day to 7 days, preferably after 1 day to 3 days, more preferably after 2 to 3 days), the amount of mRNA in the EDG receptor contained in the transformants can be quantified and analyzed.

The test compounds include, for example, peptides, proteins, non-peptide compounds, synthetic compounds, fermentation products, cell extracts, plant extracts, animal tissue extracts, plasma, etc. These compounds may be novel or known compounds.

The test compound may form salts and may be used in the form of salts with physiologically acceptable acids (e.g., inorganic acids, etc.) or bases (e.g., organic acids, etc.), preferably in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

The compound or its salt, which is obtained by the screening methods of the present invention, is the compound that changes the expression level of the EDG receptor. Specifically, it is (a) the compound or its salt that potentiates the cell stimulating activity mediated by the EDG receptor by increasing the expression level of the EDG receptor; and (b) the compound or its salt that attenuates the cell stimulating activity by decreasing the expression level of the EDG receptor.

As the cell stimulating activity, there are, for example, the activity that promotes or suppresses arachidonic acid release, acetylcholine release, intracellular $Ca^{2+}$ release, intracellular cAMP production, intracellular cGMP production, inositol phosphate production, changes in cell membrane potential, phosphorylation of intracellular proteins, activation of c-fos, cell growth, carbon monoxide production, chemotactic activity, activation of low molecular G protein Rho or Rac, phosphatidyl inositol (PI) 3 kinase activity, pH reduction, etc., preferably the activity that promotes these activities, etc.

The compound that increases the expression level of the EDG receptor to enhance the cell stimulating activity is useful as a safe and low toxic pharmaceutical to potentiate the physiological activity of the EDG receptor.

The compound that decreases the expression level of the EDG receptor to attenuate the cell stimulating activity is useful as a safe and low toxic pharmaceutical to decrease the physiological activity of the EDG receptor.

The compound or its salt that changes the expression level of the EDG receptor can be used as a prophylactic/therapeutic agent for diabetic nephropathy, chronic renal failure, nephritis, glomerulonephritis, interstitial renal disease, renal edema, etc.

(5) Agent for preventing/treating various diseases comprising the compound or its salt that changes the expression level of the EDG receptor.

The compound or its salt that changes the expression level of the EDG receptor can be used as a prophylactic/therapeutic agent for diseases associated with dysfunction of the EDG receptor.

Where the compound or its salt is used as a prophylactic/therapeutic agent for diseases associated with dysfunction of the EDG receptor described above, e.g., diabetic nephropathy, chronic renal failure, nephritis, glomerulonephritis, interstitial renal disease or renal edema, the compound or its salt can be formed into a pharmaceutical preparation in a conventional manner.

(6) Method for screening a compound (agonist, antagonist, etc.) or its salt that changes the binding property of the EDG receptor to its ligand and a screening kit

By using the EDG receptor, etc., or by constructing the expression system of a recombinant receptor protein, etc. and using the receptor-binding assay system via the expression system, the compound (e.g., a peptide, a protein, a non-peptide compound, a synthetic compound, a fermentation product, a cell extract, a plant extract, an animal tissue extract, plasma, etc.) or its salts that change the binding property of a ligand to the EDG receptor can be screened efficiently.

The ligand to the EDG-2 receptor used includes, for example, lysophosphatidic acid (LPA) or salts thereof, etc., so long as it binds to the EDG-2 receptor, but is not particularly limited thereto.

The ligand to the EDG-3 receptor used includes, for example, sphingosine-1-phosphate (S1P) or salts thereof, etc., so long as it binds to the EDG-3 receptor, but is not particularly limited thereto.

The ligand to the EDG-5 receptor used includes, for example, sphingosine-1-phosphate (S1P) or salts thereof, etc., so long as it binds to the EDG-5 receptor, but is not particularly limited thereto.

In addition, a compound (e.g., a low molecular synthetic agonist, etc.) or a salt thereof that changes the binding property of each of the EDG receptors to its ligand can also be used as the ligand. The compound or a salt thereof that changes the binding property of each of the EDG receptors to its ligand can be obtained by the screening method of the present invention later described, using as a ligand lysophosphatidic acid or its salt or sphingosine-1-phosphate or its salt.

Preferred examples of the compound or a salt thereof that changes the binding property of each EDG receptor to its ligand include FTY720 (2-amino-2-(2-[4-octylphenyl]ethyl)-1,3-propanediol hydrochloride) or its phosphate, the compounds described in WO 02/29001, the compounds described in WO 03/073986, the compounds described in WO 03/062248, the compounds described in WO 03/062252, the compounds described in Mol. Pharmacol. (2003) 64, 994-1005 (e.g., Ki1643), the compounds described in J. Med. Chem. (2002) 45m, 4629-4638 (e.g., 2-alkylthiazolidine-4-carboxylic acids, 2-(m- or p-heptylphenyl)-thiazolidine-4-carboxylic acid), the compounds described in WO 03/024402, the compounds described in US2003/0027800 and the like, and among them, preferably used are agonists such as FTY720 or its phosphate, the compounds described in WO 02/29001, the compounds described in WO 03/073986, the compounds described in WO 03/062248, the compounds described in WO 03/062252, the compounds described in WO 03/024402, the compounds described in US2003/0027800, and the like.

Hereinafter, the ligand to each receptor (including the compound or its salt that changes the binding property of the EDG receptor to its ligand) is briefly referred to as the ligand.

Examples of the compound that changes the binding property of the ligand to the EDG receptor include (a) a compound showing the cell stimulating activity mediated by the EDG receptor (a so-called agonist to the EDG receptor), (b) a compound having no such cell stimulating activity (a so-called antagonist to the EDG receptor), (c) a compound that potentiates the binding affinity of the ligand to the EDG receptor, or (d) a compound that decreases the binding affinity of the ligand to the EDG receptor, and the like.

That is, the present invention provides a method for screening a prophylactic/therapeutic drug for diabetic nephropathy, chronic renal failure, nephritis, glomerulonephritis, interstitial renal disease or renal edema, which comprises using the ligand and the EDG receptor or its partial peptide or a salt thereof (hereinafter the EDG receptor, etc.) thereby to screen a compound or its salt that changes the binding property of the EDG receptor to the ligand. Specifically, the present invention provides a method for screening a prophylactic/therapeutic drug for diabetic nephropathy, chronic renal failure, nephritis, glomerulonephritis, interstitial renal disease or renal edema, which comprises comparing (i) the case wherein the EDG receptor, etc. is brought in contact with the ligand, and (ii) the case wherein the EDG receptor, etc. is brought in contact with the ligand and a test compound, and screening the compound or its salt that changes the binding property of the ligand to the EDG receptor, etc.

According to the screening method of the present invention, the method is characterized by assaying, e.g., the binding amount of the ligand to the EDG receptor, etc., the cell stimulating activity, etc. in the cases (i) and (ii) and comparing them.

Examples of the cell stimulating activity include the activity that promotes or suppresses arachidonic acid release, acetylcholine release, intracellular $Ca^{2+}$ release, intracellular cAMP production, intracellular cGMP production, inositol phosphate production, changes in cell membrane potential, phosphorylation of intracellular proteins, activation of c-fos, cell growth, carbon monoxide production, chemotactic activity, activation of low molecular G protein Rho or Rac, phosphatidyl inositol (PI) 3 kinase activity, pH reduction, etc., preferably the activity that promotes these activities.

More specifically, the present invention provides the following methods.

(i) A method for screening a compound or a salt thereof that changes the binding property of the ligand to the EDG receptor, which comprises measuring the binding amount of a labeled form of the ligand to the EDG receptor, etc. in the case wherein the labeled ligand is brought in contact with the EDG receptor, etc. and in the case wherein the labeled ligand and a test compound are brought in contact with the EDG receptor, and comparing the cases.

(ii) A method for screening a compound or a salt thereof that changes the binding property of the ligand to the EDG receptor, which comprises measuring the binding amount of a labeled form of the ligand to a cell containing the EDG receptor or a membrane fraction of the cell, in the case wherein the labeled ligand is brought in contact with the cell containing the EDG receptor or the membrane fraction and in the case wherein the labeled ligand and a test compound are brought in contact with the cell containing the EDG receptor or its membrane fraction, and comparing these cases.

(iii) A method for screening a compound or a salt thereof that changes the binding property of the ligand to the EDG receptor, which comprises measuring the amount of a labeled form of the ligand bound to the EDG receptor, in the case wherein the labeled ligand is brought in contact with the EDG receptor expressed on a cell membrane by culturing a transformant containing the DNA of the present invention and in the case wherein the labeled ligand and a test compound are brought in contact with the EDG receptor expressed on the cell membrane by culturing a transformant containing the DNA of the present invention, and comparing the cases.

(iv) A method for screening a compound or its salt that changes the binding property of a ligand to the EDG receptor, which comprises assaying the cell stimulating activity mediated by the EDG receptor in the case wherein a compound or its salt (e.g., a ligand, etc.) that activates the EDG receptor is brought in contact with a cell containing the EDG receptor and in the case wherein the compound or its salt that activates the EDG receptor and a test compound are brought in contact with the cell containing the EDG receptor, and comparing the cell stimulating activity between the two cases.

(v) A method for screening a compound or a salt thereof that changes the binding property of the ligand to the EDG receptor, which comprises assaying the receptor protein-mediated cell stimulating activity in the case wherein a compound or its salt (e.g., a ligand, etc.) that activates the EDG receptor is brought in contact with the EDG receptor expressed on a cell membrane by culturing a transformant containing the DNA of the present invention and in the case wherein the compound or its salt that activates the EDG receptor and a test compound are brought in contact with the EDG receptor expressed on a cell membrane by culturing a transformant containing the DNA of the present invention, and comparing the cell stimulating activity between the two cases.

Hereinafter the screening method of the present invention will be described more specifically.

First, the EDG receptor, which is used for the screening method of the present invention, may be any receptor so long as it contains the EDG receptor described above, though cell membrane fractions from mammalian organs are preferably employed. However, since it is very difficult to obtain human-derived organs, human-derived receptor protein, and the like expressed abundantly by using recombinants are suitable as organs to be used for the screening.

In manufacturing the EDG receptor, the methods described above can be used, and the DNA of the present invention is preferably expressed on mammalian cells or insect cells. As the DNA fragment encoding the target protein region, a complementary DNA may be used but is not limited thereto. For example, gene fragments or a synthetic DNA may also be used. In order to introduce the DNA fragment encoding the EDG receptor into host animal cells and express the same efficiently, the DNA fragment is preferably incorporated into a polyhedron promoter of nuclear polyhedrosis virus (NPV) belonging to the Baculovirus, an SV40-derived promoter, a promoter of retrovirus, a metallothionein promoter, a human heat shock promoter, a cytomegalovirus promoter, SRα promoter, etc. at the downstream thereof. The quantity and quality of the thus expressed receptors can be examined by a publicly known method, for example, by the method described in the literature [Nambi, P. et al., J. Biol. Chem., 267, 19555-19559, 1992].

Accordingly, in the screening method of the present invention, the substance containing the EDG receptor may be the EDG receptor purified by publicly known methods, or a cell containing the EDG receptor or a membrane fraction of the cell containing the EDG receptor may be used as well.

Where the cell containing the EDG receptor is used in the screening method of the present invention, the cell may be fixed with glutaraldehyde, formalin, etc. The fixation may be carried out by a publicly known method.

The cell containing the EDG receptor refers to a host cell expressing the EDG receptor. Examples of such a host cell include Escherichia coli, Bacillus subtilis, yeast, insect cells, animal cells, etc.

The cell membrane fraction refers to a fraction abundant in cell membrane obtained by cell disruption and subsequent fractionation by publicly known methods. Cell disruption methods include cell squashing using a Potter-Elvehjem homogenizer, disruption using a Waring blender or Polytron (manufactured by Kinematica Inc.), disruption by ultrasonication, disruption by cell spraying through thin nozzles under an increased pressure using a French press, or the like. Cell membrane fractionation is effected mainly by fractionation using a centrifugal force, such as centrifugation for fractionation and density gradient centrifugation. For example, cell disruption fluid is centrifuged at a low speed (500 rpm to 3,000 rpm) for a short period of time (normally about 1 to about 10 minutes), the resulting supernatant is then centrifuged at a higher speed (15,000 rpm to 30,000 rpm) normally for 30 minutes to 2 hours. The precipitate thus obtained is used as the membrane fraction. The membrane fraction is rich in the EDG receptor expressed and membrane components such as cell-derived phospholipids, membrane proteins, etc.

The amount of the EDG receptor in a cell containing the EDG receptor or the cell membrane fraction is preferably $10^3$ to $10^8$ molecules per cell, more preferably $10^5$ to $10^7$ molecules per cell. As the level of expression increases, the ligand binding activity per unit of membrane fraction (specific activity) increases so that not only the highly sensitive screening system can be constructed but also large quantities of samples can be assayed with the same lot.

To perform (i) through (iii) above for screening the compound or its salt that changes the binding property of the ligand to the EDG receptor, an appropriate EDG receptor fraction and a labeled ligand are required.

The EDG receptor fraction is preferably a naturally occurring type EDG receptor fraction or a recombinant type EDG receptor fraction having an activity equivalent thereto. Herein, the equivalent activity is intended to mean the ligand binding activity or the signal transduction activity.

As a labeled form of the ligand, a labeled ligand, a labeled analogue compound and the like are employed. For example, there are used the ligand labeled with [$^3$H], [$^{125}$I], [$^{14}$C] [$^{35}$S], etc.

Specifically, the compound or its salt that changes the binding property of the ligand to the EDG receptor is screened by the following procedures. First, a preparation of the EDG receptor is prepared by suspending a cell or a membrane fraction of the cell containing the EDG receptor in a buffer appropriate for use in the screening method. Any buffer can be used so long as it does not interfere the binding affinity of the ligand to the EDG receptor, including a phosphate buffer or a Tris-HCl buffer, having pH of 4 to 10 (preferably pH of 6 to 8), etc. For the purpose of minimizing non-specific binding, a surfactant such as CHAPS, Tween-80™ (Kao-Atlas Inc.), digitonin, deoxycholate, etc., may optionally be added to the buffer. Further for the purpose of suppressing the degradation of the EDG receptor or ligand by a protease, a protease inhibitor such as PMSF, leupeptin, E-64 (manufactured by Peptide Institute, Inc.), pepstatin, etc. may also be added. A given amount (5,000 cpm to 500,000 cpm) of the labeled ligand is added to 0.01 ml to 10 ml of the EDG receptor solution, in which $10^{-4}$ M to $10^{-10}$ M of a test compound is co-present. To determine the amount of non-specific binding (NSB), a reaction tube containing unlabeled ligand in a large excess is also provided. The reaction is carried out at approximately 0°C to 50°C, preferably approximately 4°C to 37°C for about 20 minutes to about 24 hours, preferably about 30 minutes to 3 hours. After completion of the reaction, the reaction mixture is filtrated through glass fiber filter paper, etc. and washed with an appropriate volume of the same buffer. The residual radioactivity on the glass fiber filter paper is then measured by means of a liquid scintillation counter or γ-counter. When nonspecific binding (NSB) is subtracted from the count ($B_0$) where any antagonizing substance is absent and the resulting count ($B_0$ minus NSB) is made 100%, the test compound showing the specific binding amount (B minus NSB) of, e.g., 50% or less may be selected as a candidate compound.

The method (iv) or (v) described above for screening the compound or its salt that changes the binding property of the ligand to the EDG receptor can be performed as follows. For example, the cell stimulating activity mediated by the receptor protein can be determined by a publicly known method, or using an assay kit commercially available.

Specifically, the cells containing the EDG receptor are first cultured in a multiwell plate, etc. Prior to screening, the medium is replaced with fresh medium or with an appropriate non-cytotoxic buffer, followed by incubation for a given period of time in the presence of a test compound, etc. Subsequently, the cells are extracted or the supernatant is recovered and the resulting product is quantified by appropriate procedures. Where it is difficult to detect the production of the cell-stimulating activity indicator (e.g., arachidonic acid, cAMP, etc.) due to a degrading enzyme contained in the cells, an inhibitor against such as a degrading enzyme may be added prior to the assay. For the activity such as the cAMP production suppression activity, the baseline production in the cells is increased by forskolin or the like and the suppressing effect on the increased baseline production can be detected.

As the test compound, the same examples as described above are given.

The test compound which is preferably used is a compound designed to bind to the ligand-binding pocket, based on the atomic coordinate and the position of the ligand-binding pocket in the active site of the EDG receptor. The atomic coordinate and the position of the ligand-binding pocket in the active site of the EDG receptor can be determined by publicly known methods or modifications thereof.

By the following (1) or (2), it is determined that the compound is either an agonist or an antagonist to the EDG receptor.

(1) The screening methods of (i) to (iii) described above are performed to obtain the compound or its salt that changes the binding property of the ligand to the EDG receptor (especially inhibits the binding). Then, it is determined if the compound or its salt possesses the cell stimulating activity described above. The compound or its salt having the cell stimulating activity is an agonist to the EDG receptor, whereas the compound or its salt having no such activity is an antagonist to the EDG receptor.

(2)

(a) A test compound is brought in contact with a cell containing the EDG receptor to assay the cell stimulating activity described above. The test compound having the cell stimulating activity is an agonist to the EDG receptor.

(b) The cell stimulating activity mediated by the EDG receptor is assayed in the case wherein a compound or its salt (e.g., a ligand) that activates the EDG receptor is brought in contact with a cell containing the EDG receptor and in the case wherein a compound or its salt that activates the EDG receptor and a test compound are brought in contact with a cell containing the EDG receptor, and comparison is made therebetween. The compound or its salt capable of decreasing the cell stimulating activity by the compound that activates the EDG receptor is an antagonist to the EDG receptor.

The kit for screening the compound or its salt that changes the binding property of the ligand to the EDG receptor is the EDG receptor, the kit containing a cell or a membrane fraction of the cell containing the EDG receptor, and the like.

Examples of the screening kit of the present invention include the following.

1. Reagent for screening

(1) Assay buffer and wash buffer

Hanks' balanced salt solution (manufactured by Gibco, Inc.) supplemented with 0.05% bovine serum albumin (manufactured by Sigma, Inc.)

The solution is sterilized by filtration through a 0.45 μm filter, and stored at 4°C or may be prepared at use.

(2) Preparation of the EDG receptor

CHO cells wherein the EDG receptor has been expressed are passaged in a 12-well plate at a density of 5 x $10^5$ cells/well followed by culturing at 37°C under 5% $CO_2$ and 95% air for 2 days.

(3) The labeled ligand

An aqueous solution of the ligand labeled with commercially available [$^3$H], [$^{125}$I], [$^{14}$C], [$^{35}$S], etc. is stored at 4°C or -20°C, and diluted to 1 μM with the assay buffer upon use.

(4) Standard solution of the ligand

The ligand is dissolved in and adjusted to 1 mM with PBS containing 0.1% bovine serum albumin (manufactured by Sigma, Inc.) and stored at -20°C.

2. Assay method

(1) CHO cells wherein the EDG receptor has been expressed are cultured in a 12-well culture plate and washed twice with 1 ml of the assay buffer, and 490 μl of the assay buffer is added to each well.

(2) After adding 5 μl of $10^{-3}$ - $10^{-10}$ M test compound solution, 5 μl of the labeled ligand is added to the mixture, and the cells are incubated at room temperature for an hour. To determine the amount of the non-specific binding, 5 μl of the ligand is added in place of the test compound.

(3) The reaction solution is removed, and the wells are washed 3 times with the washing buffer. The labeled ligand bound to the cells is dissolved in 0.2N NaOH-1% SDS, and mixed with 4 ml of liquid scintillator A (manufactured by Wako Pure Chemical Industries, Ltd.)

(4) The radioactivity is measured using a liquid scintillation counter (manufactured by Beckman Co.), and the percent maximum binding (PMB) is calculated by the equation below.

$$PMB = [(B - NSB)/(B_0 - NSB)] \times 100$$

PMB : Percent maximum binding
B : Value obtained in the presence of a test compound
NSB : Non-specific binding
Bo : Maximum binding

The compound or its salt, which can be obtained by using the screening method or the screening kit of the present invention, is the compound that changes the binding property of the ligand to the EDG receptor. Specifically, the compound is: (1) a compound having the cell stimulating activity mediated by the EDG receptor (a so-called agonist to the EDG receptor); (2) a compound having no cell stimulating activity (a so-called antagonist to the EDG receptor); (3) a compound that potentiates the binding affinity of the ligand to the EDG receptor; or (4) a compound that reduces the binding affinity of the ligand to the EDG receptor.

These compounds, which are obtained by using the screening method or screening kit of the present invention, may be peptides, proteins, non-peptide compounds, synthetic compounds, fermentation products, cell extracts, plant extracts, animal tissue extracts, plasma, etc. These compounds may be novel or known compounds.

The compound obtained by the screening method may form salts and may be used in the form of salts with physiologically acceptable acids (e.g., inorganic acids, organic acids, etc.) or bases (e.g., alkali metal salts), preferably in the form of physiologically acceptable acid addition salts. Examples of such salts are salts with inorganic acids (e. g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

Since the agonist to the EDG receptor have the same physiological activity as in the ligand to the EDG receptor, the agonist is useful as a safe and low toxic pharmaceutical, correspondingly to the physiological activity possessed by the ligand.

Since the antagonist to the EDG receptor can suppress the physiological activity the ligand to the EDG receptor has, the antagonist is useful as a safe and low toxic pharmaceutical to suppress the physiological activity.

The compound or its salt that potentiates the binding affinity of the ligand to the EDG receptor can potentiate the physiological activity the ligand to the EDG receptor has is useful as a safe and low toxic pharmaceutical to potentiate the physiological activity the ligand to the EDG receptor has.

The compound that reduces the binding affinity of the ligand to the EDG receptor can reduce the physiological activity the ligand to the EDG receptor has is useful as a safe and low toxic pharmaceutical to suppress the physiological activity the ligand to the EDG receptor has.

As the compounds that change the binding property of the ligand to the EDG receptor, there are used, for example, FTY720 or its phosphate, the compounds described in WO 02/29001, the compounds described in WO 03/073986, the compounds described in WO 03/062248, the compounds described in WO 03/062252, the compounds described in Mol. Pharmacol. (2003) 64, 994-1005, the compounds described in J. Med. Chem. (2002) 45m, 4629-4638, the compounds described in WO 03/024402, the compounds described in US2003/0027800, and the like.

Among them, FTY720 or its phosphate, the compounds described in WO 02/29001, the compounds described in WO 03/073986, the compounds described in WO 03/062248, the compounds described in WO 03/062252, the compounds described in WO 03/024402, the compounds described in US2003/0027800, etc. are used as agonists to the EDG receptor.

As the antagonists, there are used the compounds described in WO 02/29001, the compounds described in Mol. Pharmacol. (2003) 64,994-1005, the compounds described in J. Med. Chem. (2002) 45m, 4629-4638, the compounds described in WO 03/024402, the compounds described in US2003/0027800, and the like.

(7) Agent for preventing/treating various diseases comprising the compound or its salt that changes the binding property of the EDG receptor to the ligand (the agonist, antagonist, etc.)

The compound or its salt that changes the binding property of the ligand to the EDG receptor (the agonist, antagonist, etc.) or the ligand, especially the antagonist can be used as an agent for preventing/treating diseases associated with dysfunction of the EDG receptor, e.g., diabetic nephropathy, chronic renal failure, nephritis, glomerulonephritis, interstitial renal disease or renal edema.
Where the compound or its salt or the ligand is used as an agent for preventing/treating diseases associated with dysfunction of the EDG receptor, the compound, etc. can be formed into a pharmaceutical preparation in a conventional manner as described above.

(8) Method for quantifying the protein, etc. using the antibody of the present invention, a diagnostic agent comprising the antibody of the present invention and a method for diagnosis using the diagnostic agent

The antibody of the present invention is capable of specifically recognizing the EDG receptor and can be used for quantification of the EDG receptor in a sample fluid, especially quantification by sandwich immunoassay, competitive method, immunometric method, nephrometry, etc.
For applying these immunological methods to the measurement methods of the present invention, any particular conditions or procedures are not required. The assay system for the EDG receptor is constructed by adding the usual technical consideration in the art to the conventional conditions and procedures. For the details of these general technical means, reference can be made to the following reviews and texts. For example, Hiroshi Irie, ed. "Radioimmunoassay" (Kodansha, published in 1974), Hiroshi Irie, ed. "Sequel to the Radioimmunoassay" (Kodansha, published in 1979), Eiji Ishikawa, et al. ed. "Enzyme immunoassay" (Igakushoin, published in 1978), Eiji Ishikawa, et al. ed. "Immunoenzyme assay" (2nd ed.) (Igakushoin, published in 1982), Eiji Ishikawa, et al. ed. "Immunoenzyme assay" (3rd ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies)) (all published by Academic Press Publishing).
As described above, the EDG receptor can be quantified with high sensitivity, using the antibody of the present invention.
In addition, various diseases associated with dysfunction of the EDG receptor can be diagnosed by quantifying the EDG receptor in the living body using the antibody of the present invention.
For example, where an increased or decreased level of the EDG receptor is detected by quantifying the EDG receptor level using the antibody of the present invention, it can be diagnosed that one suffers from, for example, diseases associated with dysfunction or overexpression of the EDG receptor, especially diabetic nephropathy, chronic renal failure, nephritis, glomerulonephritis, interstitial renal disease or renal edema, or it is highly likely to suffer from these disease in the future.
In addition, the antibody of the present invention can be used to specifically direct the EDG receptor present in a sample fluid such as body fluids, tissues, etc. Furthermore, the antibody may also be used for the preparation of an antibody column to purify the EDG receptor, detect the EDG receptor in each fraction upon purification, analysis of the behavior of the EDG receptor in the cells under investigation.

(9) Method for screening the compound or its salt that changes the amount of the EDG receptor on cell membrane

The antibody of the present invention is capable of specifically recognizing the EDG receptor and can be used for screening the compound or its salt that changes the amount of the EDG receptor on the cell membrane.
That is, the present invention provides the following methods:

(i) a method for screening the compound or its salt that changes the amount of the EDG receptor in the cell membrane, which comprises measuring the amount of the EDG receptor contained in a cell membrane fraction isolated after disrupting tissues or cells isolated from (a) blood, (b) a particular organ or (c) the organs of non-human mammals;
(ii) a method for screening the compound or its salt that changes the amount of the EDG receptor in the cell membrane, which comprises disrupting a transformant expressing EDG receptor, isolating the cell membrane fraction and quantifying the EDG receptor contained in the cell membrane fraction;

(iii) a method for screening the compound or its salt that changes the amount of the EDG receptor in the cell membrane, which comprises preparing a slice from the tissues or cells isolated from (a) blood, (b) a particular organ or (c) organs, using the immunostaining assay thereby and quantifying the staining intensity of the EDG receptor on the cell surface to confirm the EDG receptor on the cell membrane by; and,

(iv) a method for screening the compound or its salt that changes the amount of the EDG receptor in the cell membrane, which comprises preparing a slice of a transformant expressing the EDG receptor and quantifying the staining intensity of the EDG receptor on the cell surface using the immunostaining assay thereby to confirm the EDG receptor on the cell membrane.

Specifically, the EDG receptor contained in the cell membrane fraction can be assayed as follows.

(i) Normal or disease models of non-human mammals (e.g., mice, rats, rabbits, ovine, swine, bovine, feline, canine, simian, more specifically, tumor-bearing mice, etc.) are given a drug (e.g., an anticancer agent, etc.) or physical stress (e.g., soaking stress, electric shock, light and darkness, low temperature, etc.), and the blood, or particular organs (e.g., brain, lung, colon, prostate, etc.), or tissues or cells isolated from the organs are obtained after a specified period of time. The obtained organs, tissues, cells or the like are suspended in, for example, an appropriate buffer (e.g., Tris hydrochloride buffer, phosphate buffer, HEPES buffer, etc.), and the organs, tissues or cells are disrupted, and the cell membrane fraction is obtained using surfactants (e.g., Triton-X 100™, Tween 20™) and further using techniques such as centrifugal separation, filtration, column fractionation, etc.

The cell membrane fraction means a fraction abundant in cell membrane obtained by cell disruption and subsequent fractionation by publicly known methods. Cell disruption methods include cell squashing using a Potter-Elvehjem homogenizer, disruption using a Waring blender or Polytron (manufactured by Kinematica Inc.), disruption by ultrasonication, disruption by cell spraying through thin nozzles under an increased pressure using a French press, or the like. Cell membrane fractionation is effected mainly by fractionation using a centrifugal force, such as centrifugation for fractionation and density gradient centrifugation. For example, cell disruption fluid is centrifuged at a low speed (500 rpm to 3,000 rpm) for a short period of time (normally about 1 to about 10 minutes), the resulting supernatant is then centrifuged at a higher speed (15,000 rpm to 30,000 rpm) normally for 30 minutes to 2 hours. The precipitate thus obtained is used as the membrane fraction. The membrane fraction is rich in the EDG receptor expressed and membrane components such as cell-derived phospholipids, membrane proteins, etc.

The EDG receptor contained in the cell membrane fraction can be quantified by, for example, the sandwich immunoassay, western blot analysis, etc. using the antibody of the present invention.

The sandwich immunoassay can be performed as described above, and the western blot can be performed by publicly known methods.

(ii) A transformant expressing the EDG receptor is prepared by the method described above, and the EDG receptor contained in the cell membrane fraction can be quantified.

The compound or its salt that changes the amount of the EDG receptor on cell membranes can be screened as follows.

(i) A test compound is administered to normal or disease models of non-human mammals at a specified period of time before (30 minutes to 24 hours before, preferably 30 minutes to 12 hours before, more preferably 1 hour to 6 hours before), at a given period of time after (30 minutes to 3 days after, preferably 1 hour to 2 days after, more preferably 1 hour to 24 hours after), or simultaneously with a drug or physical stress. At a given period of time (30 minute to 3 days, preferably 1 hour to 2 days, more preferably 1 hour to 24 hours) after the administration, the amount of the EDG receptor in the cell membrane can be quantified.

(ii) When a transformant is cultured in a conventional manner, a test compound is mixed into the culture medium. After incubation for a given period of time (after 1 day to 7 days, preferably after 1 day to 3 days, more preferably after 2 to 3 days), the amount of the EDG receptor in the cell membrane can be quantified.

Specifically, the EDG receptor or partial peptide thereof contained on cell membrane fraction is confirmed as follows.

(iii) A drug (e.g., an anti-dementia agent, hypotensive agent, anticancer agent, antiobestic agent, etc.) or physical stress (e.g., soaking stress, electric shock, light and darkness, low temperature, etc.) or the like is given to normal or disease models of non-human mammals (e.g., mice, rats, rabbits, ovine, swine, bovine, feline, canine, simian, etc., more specifically, rats with dementia, obese mice, rabbits with arteriosclerosis, tumor-bearing mice, etc.), and blood or a particular organ (e.g., brain, lung, colon, etc.), or tissues or cells isolated from the organ are obtained after a specified period of time. A tissue section is prepared from the thus obtained organs, tissues, cells, etc. in a conventional manner followed by immunostaining using the antibody of the present invention. The staining intensity of the receptor protein on the cell surface is quantified to confirm the protein on the cell membrane, whereby the amount of the EDG receptor on the cell membrane can be confirmed quantitatively or qualitatively.
(iv) The confirmation can also be made by the similar procedure, using a transformant expressing the EDG receptor.

As the test compound, there are used the same examples given above.
The compound or its salt obtained by using the screening method of the present invention is the compound or its salt that has the action of changing the amount of the EDG receptor in the cell membranes. Specifically, the compound or its salt is (a) the compound or its salt that increases the amount of the EDG receptor in the cell membranes thereby to potentiate the cell stimulating activity mediated by the receptor and (b) the compound or its salt that decreases the amount of the EDG receptor in the cell membranes thereby to attenuate the cell stimulating activity.
The compound obtained by using the screening method of the present invention includes a peptide, a protein, a non-peptide compound, a synthetic compound, a fermentation product, a cell extract, a plant extract, an animal tissue extract, plasma, etc. The compound may be a novel or publicly known compound.
The compound obtained by the screening method may form salts and may be used in the form of salts with physiologically acceptable acids (e.g., inorganic acids, organic acids, etc.) or bases (e.g., alkali metal salts), preferably in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.
The compound or its salt that increases the amount of the EDG receptor in the cell membranes to enhance the cell stimulating activity is useful as a safe and low toxic pharmaceutical to potentiate the physiological activity of the EDG receptor.
The compound that decreases the amount of the EDG receptor in the cell membrane to attenuate the cell stimulating activity is useful as a safe and low toxic pharmaceutical to decrease the physiological activity of the EDG receptor.

(10) Compound or its salt that changes the amount of the EDG receptor in the cell membrane and agent for preventing/treating various diseases comprising the compound of its salt

As described above, the EDG receptor is considered to play a critical role in the living body. Therefore, the compound or its salt that changes the amount of the EDG receptor on the cell membrane can be used as an agent for preventing/treating diseases associated with dysfunction of the EDG receptor.
For example, the compound that changes the amount of the EDG receptor on the cell membrane, especially the compound that reduces the amount of the EDG receptor on the cell membrane can be used as an agent for preventing/treating diseases associated with dysfunction or overexpression of the EDG receptor (e.g., diabetic nephropathy, chronic renal failure, nephritis, glomerulonephritis, interstitial renal disease or renal edema).
Where the compound or its salt is used as an agent for preventing/treating diseases associated with dysfunction or overexpression of the EDG receptor, the compound or its salt can be prepared into pharmaceutical preparations in a conventional manner as described above.

(11) Pharmaceutical comprising the antibody of the present invention

The neutralizing antibody to the EDG receptor has an activity of inactivating the EDG receptor-related activity, e.g., the signal transduction function. Accordingly, when the antibody of the present invention has a neutralizing activity, the EDG receptor-related signal transduction, e.g., the EDG receptor-related cell stimulating activity can be inactivated. Such an antibody can thus be used for preventing/treating diseases caused by overexpression, etc. of the EDG receptor.
For example, the antibody to the EDG receptor (e.g., neutralizing antibody) can be used as an agent for preventing/treating diseases caused by overexpression, etc. of the EDG receptor (e.g., diabetic nephropathy, chronic renal failure, nephritis, glomerulonephritis, interstitial renal disease or renal edema).
The prophylactic/therapeutic agent of the present invention can be prepared into pharmaceutical preparations in a manner similar to the preparations comprising the EDG receptor described above.

(12) Preparation of DNA transgenic animal of the present invention

The present invention provides a non-human mammal bearing exogenous DNA of the present invention (hereinafter briefly referred to as the exogenous DNA of the present invention) or its variant DNA (sometimes briefly referred to as the exogenous variant DNA of the present invention).

That is, the present invention provides:

[1] a non-human mammal bearing the exogenous DNA of the present invention or its variant DNA;
[2] the mammal according to [1], wherein the non-human mammal is a rodent;
[3] the mammal according to [2], wherein the rodent is mouse or rat; and,
[4] a recombinant vector bearing the exogenous DNA of the present invention or its variant DNA and capable of expressing in a mammal.

The non-human mammal bearing the exogenous DNA of the present invention or its variant DNA (hereinafter briefly referred to as the DNA transgenic animal of the present invention) can be produced by transferring a desired DNA into an unfertilized egg, a fertilized egg, a spermatozoon, and a germinal cell containing a primordial germinal cell thereof, and the like, preferably in the embryogenic stage in the development of a non-human mammal (more preferably in the single cell or fertilized cell stage and generally before the 8-cell phase), by standard means, such as the calcium phosphate method, the electric pulse method, the lipofection method, the agglutination method, the microinjection method, the particle gun method, the DEAE-dextran method, etc. Also, it is possible to transfer the exogenous DNA of the present invention into a somatic cell, a living organ, a tissue cell, or the like by the DNA transfer, and utilize the transformant for cell culture, tissue culture, etc. In addition, these cells may be fused with the above-described germinal cell by a publicly known cell fusion method to prepare the DNA transgenic animal of the present invention.

Examples of the non-human mammal that can be used include bovine, swine, ovine, goat, rabbits, canine, feline, guinea pigs, hamsters, mice, rats, etc. Above all, preferred are rodents, especially mice (e.g., C57B1/6 strain, DBA2 strain, etc. for a pure line and for a cross line, B6C3$F_1$ strain, BD$F_1$ strain B6D2$F_1$ strain, BALB/c strain, ICR strain, etc.), rats (Wistar, SD, etc.) or the like, since they are relatively short in ontogeny and life cycle from a standpoint of producing model animals for human disease.

"Mammals" in a recombinant vector that can be expressed in the mammals include the aforesaid non-human mammals and human.

The exogenous DNA of the present invention refers to the DNA of the present invention that is once isolated and extracted from mammals, not the DNA of the present invention inherently possessed by the non-human mammals.

The mutant DNA of the present invention includes mutants resulting from variation (e.g., mutation, etc.) in the base sequence of the original DNA of the present invention, specifically DNAs resulting from base addition, deletion, substitution with other bases, etc. and further including abnormal DNA.

The abnormal DNA is intended to mean DNA that expresses abnormal EDG receptor and exemplified by the DNA that expresses EDG receptor, etc. for suppressing the function of normal EDG receptor.

The exogenous DNA of the present invention may be any one of those derived from a mammal of the same species as, or a different species from, the mammal as the target animal. In transferring the DNA of the present invention, it is generally advantageous to use the DNA as a DNA construct in which the DNA is ligated downstream a promoter capable of expressing the DNA in the target animal. For example, in the case of transferring the human DNA of the present invention, a DNA transgenic mammal that expresses the DNA of the present invention to a high level, can be prepared by microinjecting a DNA construct (e.g., vector, etc.) ligated with the human DNA of the present invention into a fertilized egg of the target non-human mammal downstream various promoters which are capable of expressing the DNA derived from various mammals (e.g., rabbits, canine, feline, guinea pigs, hamsters, rats, mice, etc.) bearing the DNA of the present invention highly homologous to the human DNA.

As expression vectors for the EDG receptor, there are Escherichia coli-derived plasmids, Bacillus subtilis-derived plasmids, yeast-derived plasmids, bacteriophages such as λ phage, retroviruses such as Moloney leukemia virus, etc., and animal viruses such as vaccinia virus, baculovirus, etc. Of these vectors, Escherichia coli-derived plasmids, Bacillus subtilis-derived plasmids, or yeast-derived plasmids, etc. are preferably used.

Examples of these promoters for regulating the DNA expression include (i) promoters for DNA derived from viruses (e.g., simian virus, cytomegalovirus, Moloney leukemia virus, JC virus, breast cancer virus, poliovirus, etc.), and (ii) promoters derived from various mammals (human, rabbits, canine, feline, guinea pigs, hamsters, rats, mice, etc.), for example, promoters of albumin, insulin II, uroplakin II, elastase, erythropoietin, endothelin, muscular creatine kinase, glial fibrillary acidic protein, glutathione S-transferase, platelet-derived growth factor β, keratins K1, K10 and K14, collagen types I and II, cyclic AMP-dependent protein kinase βI subunit, dystrophin, tartarate-resistant alkaline phosphatase, atrial natriuretic factor, endothelial receptor tyrosine kinase (generally abbreviated as Tie2), sodium-potassium adenosine triphosphorylase (Na,K-ATPase), neurofilament light chain, metallothioneins I and IIA, metalloproteinase I tissue inhibitor, MHC class I antigen (H-2L), H-ras, renin, dopamine β-hydroxylase, thyroid peroxidase (TPO), protein chain elongation factor 1α (EF-1α), β actin, α and β myosin heavy chains, myosin light chains 1 and 2, myelin base protein, thyroglobulins, Thy-1, immunoglobulins, H-chain variable region (VNP), serum amyloid component P, myoglobin, troponin C, smooth muscle α actin, preproencephalin A, vasopressin, etc. Among them, cytomegalovirus promoters, human protein elongation factor 1α (EF-1α) promoters, human and fowl β actin promoters, etc., which are capable of high expression in the whole body are preferred.

Preferably, the vectors described above have a sequence that terminates the transcription of the desired messenger RNA in the DNA transgenic animal (generally termed a terminator); for example, a sequence of each DNA derived from viruses and various mammals, and SV40 terminator of the simian virus and the like are preferably used.

In addition, for the purpose of increasing the expression of the desired exogenous DNA to a higher level, the splicing signal and enhancer region of each DNA, a portion of the intron of an eukaryotic DNA may also be ligated at the 5' upstream of the promoter region, or between the promoter region and the translational region, or at the 3' downstream of the translational region, depending upon purposes.

The translational region for normal EDG receptor can be obtained using as a starting material the entire genomic DNA or its portion of liver, kidney, thyroid cell or fibroblast origin from human or various mammals (e.g., rabbits, canine, feline, guinea pigs, hamsters, rats, mice, etc.) or of various commercially available genomic DNA libraries, or using cDNA prepared by a publicly known method from RNA of liver, kidney, thyroid cell or fibroblast origin as a starting material. Also, an exogenous abnormal DNA can produce the translational region through variation of the translational region of normal EDG receptor obtained from the cells or tissues described above by point mutagenesis.

The translational region can be prepared by a conventional DNA engineering technique, in which the DNA is ligated downstream the aforesaid promoter and if desired, upstream the translation termination site, as a DNA construct capable of being expressed in the transgenic animal.

The exogenous DNA of the present invention is transferred at the fertilized egg cell stage in a manner such that the DNA is certainly present in all the germinal cells and somatic cells of the target mammal. The fact that the exogenous DNA of the present invention is present in the germinal cells of the animal prepared by DNA transfer means that all offspring of the prepared animal will maintain the exogenous DNA of the present invention in all of the germinal cells and somatic cells thereof. The offspring of the animal that inherits the exogenous DNA of the present invention also have the exogenous DNA of the present invention in all of the germinal cells and somatic cells thereof.

The non-human mammal in which the normal exogenous DNA of the present invention has been transferred can be passaged as the DNA=bearing animal under ordinary rearing environment, by confirming that the exogenous DNA is stably retained by crossing.

By transfer of the exogenous DNA of the present invention at the fertilized egg cell stage, the DNA is retained to be excess in all of the germinal and somatic cells. The fact that the exogenous DNA of the present invention is excessively present in the germinal cells of the prepared animal after transfer means that the DNA of the present invention is excessively present in all of the germinal cells and somatic cells thereof. The offspring of the animal that inherits the exogenous DNA of the present invention have excessively the DNA of the present invention in all of the germinal cells and somatic cells thereof.

It is possible to obtain homozygous animals having the transferred DNA in both homologous chromosomes and breed male and female of the animal so that all the progeny have this DNA in excess.

In a non-human mammal bearing the normal DNA of the present invention, the normal DNA of the present invention has expressed at a high level, and may eventually develop hyperfunction in the function of the EDG receptor by accelerating the function of endogenous normal DNA. Therefore, the animal can be utilized as a pathologic model animal for such a disease. For example, using the normal DNA transgenic animal of the present invention, it is possible to elucidate the mechanism of hyperfunction in the function of the EDG receptor and the pathological mechanism of the disease associated with the EDG receptor and to investigate how to treat these diseases.

Furthermore, since a mammal transferred with the exogenous normal DNA of the present invention exhibits an increasing symptom of the EDG receptor liberated, the animal is usable for screening of a drug for the treatment of diseases associated with the EDG receptor.

On the other hand, a non-human mammal having the exogenous abnormal DNA of the present invention can be passaged under normal breeding conditions as the DNA-bearing animal by confirming stable retention of the exogenous DNA via crossing. Furthermore, the exogenous DNA of interest can be utilized as a starting material by inserting the DNA into the plasmid described above. The DNA construct with a promoter can be prepared by conventional DNA engineering techniques. The transfer of the abnormal DNA of the present invention at the fertilized egg cell stage is preserved to be present in all of the germinal and somatic cells of the target mammal. The fact that the abnormal DNA of the present invention is present in the germinal cells of the animal after DNA transfer means that all of the offspring of the prepared animal have the abnormal DNA of the present invention in all of the germinal and somatic cells. Such an offspring that passaged the exogenous DNA of the present invention will have the abnormal DNA of the present invention in all of the germinal and somatic cells. A homozygous animal having the introduced DNA on both of homologous chromosomes can be acquired, and by crossing these male and female animals, all the offspring can be bred to retain the DNA.

In a non-human mammal bearing the abnormal DNA of the present invention, the abnormal DNA of the present invention has expressed to a high level, and may eventually develop the function inactive type inadaptability to the EDG receptor by inhibiting the functions of endogenous normal DNA. Therefore, the animal can be utilized as a pathologic model animal for such a disease. For example, using the abnormal DNA transgenic animal of the present invention, it is possible to elucidate the mechanism of the function inactive type inadaptability to the EDG receptor and the pathological mechanism of the disease and to investigate how to treat the disease.

More specifically, the transgenic animal overexpressing the abnormal DNA of the present invention is expected to serve as an experimental model to elucidate the mechanism of the functional inhibition (dominant negative effect) of a normal receptor protein by the abnormal receptor protein of the present invention in the function inactive type inadaptability of the EDG receptor.

A mammal bearing the abnormal exogenous DNA of the present invention is also expected to serve for screening a candidate drug for the treatment of the function inactive type inadaptability of the EDG receptor, since a free form of the EDG receptor is increased in such an animal.

Other potential applications of two kinds of the DNA transgenic animals of the present invention described above further include, for example:

(a) use as a cell source for tissue culture;

(b) elucidation of the relation to the EDG receptor that is specifically expressed or activated by the EDG receptor, by direct analysis of DNA or RNA in tissues of the DNA transgenic animal of the present invention or by analysis of the EDG receptor tissues expressed by the DNA;

(c) research on the function of cells derived from tissues that are difficult to culture, using cells in tissues bearing the DNA cultured by a standard tissue culture technique;

(d) screening a drug that enhances the functions of cells using the cells described in (3) above; and,

(e) isolation and purification of the variant receptor protein of the present invention and preparation of an antibody thereto; etc.

Furthermore, clinical conditions of a disease associated wit the EDG receptor, including the function inactive type inadaptability to the EDG receptor can be determined by using the DNA transgenic animal of the present invention. Also, pathological findings on each organ in a disease model associated with the EDG receptor can be obtained in more detail, leading to the development of a new method for treatment as well as the research and therapy of any secondary diseases associated with the disease.

It is also possible to obtain a free DNA-transferred cell by withdrawing each organ from the DNA transgenic animal of the present invention, mincing the organ and degrading with a proteinase such as trypsin, etc., followed by establishing culturing or the line of cultured cells. Furthermore, the DNA transgenic animal of the present invention can serve to identify cells capable of producing the EDG receptor, and to study in association with apoptosis, differentiation or proliferation or on the mechanism of signal transduction in these properties to inspect any abnormality therein. Thus, the DNA transgenic animal can provide an effective research material for the EDG receptor and for investigation of the function and effect thereof.

To develop a therapeutic drug for diseases associated with the EDG receptor, including the function inactive type inadaptability to the EDG receptor, using the DNA transgenic animal of the present invention, an effective and rapid method for screening can be provided by using the method for inspection and the method for quantification, etc. described above. It is also possible to investigate and develop a method for DNA therapy for the treatment of diseases associated with the EDG receptor, using the DNA transgenic animal of the present invention or a vector capable of expressing the exogenous DNA of the present invention.

(13) Knockout animal

The present invention provides a non-human mammalian embryonic stem cell bearing the DNA of the present invention inactivated and a non-human mammal deficient in expressing the DNA of the present invention.

Thus, the present invention provides:

[1] a non-human mammalian embryonic stem cell in which the DNA of the present invention is inactivated;

[2] the embryonic stem cell according to [1], wherein the DNA is inactivated by introducing a reporter gene (e.g., β-galactosidase gene derived from *Escherichia coli);*

[3] the embryonic stem cell according to [1], which is resistant to neomycin;

[4] the embryonic stem cell according to [1], wherein the non-human mammal is a rodent;

[5] the embryonic stem cell according to [4], wherein the rodent is mouse;

[6] a non-human mammal deficient in expressing the DNA of the present invention, wherein the DNA is inactivated;

[7] the non-human mammal according to [6], wherein the DNA is inactivated by inserting a reporter gene (e.g., β-galactosidase derived from *Escherichia coli)* therein and the reporter gene is capable of being expressed under control of a promoter for the DNA of the present invention;

[8] the non-human mammal according to [6], which is a rodent;

[9] the non-human mammal according to [8], wherein the rodent is mouse; and,

[10] a method for screening a compound or its salt that promotes or inhibits the promoter activity to the DNA of the present invention, which comprises administering a test compound to the mammal of [7] and detecting expression of the reporter gene.

The non-human mammal embryonic stem cell in which the DNA of the present invention is inactivated refers to a non-human mammal embryonic stem cell that suppresses the ability of the non-human mammal to express the DNA by artificially mutating the DNA of the present invention, or the DNA has no substantial ability to express the EDG receptor (hereinafter sometimes referred to as the knockout DNA of the present invention) by substantially inactivating the activity of the EDG receptor encoded by the DNA (hereinafter merely referred to as ES cell).

As the non-human mammal, the same examples as described above are given.

Techniques for artificially mutating the DNA of the present invention include deletion of a part or all of the DNA sequence and insertion of or substitution with other DNA, by genetic engineering. By these variations, the knockout DNA of the present invention may be prepared, for example, by shifting the reading frame of a codon or by disrupting the function of a promoter or exon.

Specifically, the non-human mammal embryonic stem cell in which the DNA of the present invention is inactivated (hereinafter merely referred to as the ES cell with the DNA of the present invention inactivated or the knockout ES cell of the present invention) can be obtained by, for example, isolating the DNA of the present invention that the desired non-human mammal possesses, inserting a DNA fragment having a DNA sequence constructed by inserting a drug resistant gene such as a neomycin resistant gene or a hygromycin resistant gene, or a reporter gene such as lacZ (β-galactosidase gene) or cat (chloramphenicol acetyltransferase gene), etc. into its exon region thereby to disable the functions of exon, or integrating to a chromosome of the target animal by, e.g., homologous recombination, a DNA sequence that terminates gene transcription (e.g., polyA additional signal, etc.) in the intron between exons, thus inhibiting the synthesis of complete messenger RNA and eventually destroying the gene (hereinafter briefly referred to as a targeting vector). The thus-obtained ES cells to the southern hybridization analysis with a DNA sequence on or near the DNA of the present invention as a probe, or to PCR analysis with a DNA sequence on the targeting vector and another DNA sequence near the DNA of the present invention which is not included in the targeting vector as primers, to select the knockout ES cell of the present invention.

The parent ES cells to inactivate the DNA of the present invention by homologous recombination, etc. may be of a strain already established as described above, or may originally be established in accordance with a modification of the known method by Evans and Kaufman described above. For example, in the case of mouse ES cells, currently it is common practice to use ES cells of the 129 strain. However, since their immunological background is obscure, the C57BL/6 mouse or the $BDF_1$ mouse ($F_1$ hybrid between C57BL/6 and DBA/2), wherein the low ovum availability per C57BL/6 in the C57BL/6 mouse has been improved by crossing with DBA/2, may be preferably used, instead of obtaining a pure line of ES cells with the clear immunological genetic background and for other purposes. The $BDF_1$ mouse is advantageous in that, when a pathologic model mouse is generated using ES cells obtained therefrom, the genetic background can be changed to that of the C57BL/6 mouse by back-crossing with the C57BL/6 mouse, since its background is of the C57BL/6 mouse, as well as being advantageous in that ovum availability per animal is high and ova are robust.

In establishing ES cells, blastocytes at 3.5 days after fertilization are commonly used. In the present invention, embryos are preferably collected at the 8-cell stage, after culturing until the blastocyte stage, the embryos are used to efficiently obtain a large number of early stage embryos.

Although the ES cells used may be of either sex, male ES cells are generally more convenient for generation of a germ cell line chimera. It is also desirable that sexes are identified as soon as possible to save painstaking culture time.

Methods for sex identification of the ES cell include the method in which a gene in the sex-determining region on the Y-chromosome is amplified by the PCR process and detected. When this method is used, one colony of ES cells (about 50 cells) is sufficient for sex-determination analysis, which karyotype analysis requires about $10^6$ cells; therefore, the first selection of ES cells at the early stage of culture can be based on sex identification, and male cells can be selected early, which saves a significant amount of time at the early stage of culture.

Also, second selection can be achieved by, for example, confirmation of the number of chromosomes by the G-banding method. It is usually desirable that the chromosome number of the obtained ES cells be 100% of the normal number. However, when it is difficult to obtain the cells having the normal number of chromosomes due to physical operations, etc. in the cell establishment, it is desirable that the ES cell is again cloned to a normal cell (e.g., in a mouse cell having the number of chromosomes being 2n = 40) after knockout of the gene of the ES cells.

Although the embryonic stem cell line thus obtained shows a very high growth potential, it must be subcultured with great care, since it tends to lose its ontogenic capability. For example, the embryonic stem cell line is cultured at about 37°C in a carbon dioxide incubator (preferably 5% carbon dioxide and 95% air, or 5% oxygen, 5% carbon dioxide and 90% air) in the presence of LIF (1 to 10000 U/ml) on appropriate feeder cells such as STO fibroblasts, treated with a trypsin/EDTA solution (normally 0.001 to 0.5% trypsin/0.1 to about 5 mM EDTA, preferably about 0.1% trypsin/1 mM EDTA) at the time of passage to obtain separate single cells, which are then plated on freshly prepared feeder cells. This passage is normally conducted every 1 to 3 days; it is desirable that cells be observed at the passage and cells found to be morphologically abnormal in culture, if any, be abandoned.

Where ES cells are allowed to reach a high density in mono-layers or to form cell aggregates in suspension under appropriate conditions, it is possible to differentiate the ES cells to various cell types, for example, pariental and visceral muscles, cardiac muscle or the like [M. J. Evans and M. H. Kaufman, Nature, 292, 154, 1981; G. R. Martin, Proc. Natl. Acad. Sci. U. S.A., 78, 7634, 1981; T. C. Doetschman et al., Journal of Embryology Experimental Morphology, 87, 27, 1985]. The cells deficient in expression of the DNA of the present invention, which are obtained from the differentiated ES cells of the present invention, are useful for studying the EDG receptor in vitro or the EDG receptor cytologically.

The non-human mammal deficient in expression of the DNA of the present invention can be identified from a normal animal by measuring the mRNA level in the subject animal by a publicly known method, and indirectly comparing the degrees of expression.

As the non-human mammal, the same examples as described above are given.

With respect to the non-human mammal deficient in expression of the DNA of the present invention, the DNA of the present invention can be made knockout by transferring a targeting vector, prepared as described above, to mouse embryonic stem cells or mouse oocytes, and conducting homologous recombination in which a targeting vector DNA sequence, wherein the DNA of the present invention is inactivated by the transfer, is replaced with the DNA of the present invention on a chromosome of a mouse embryonic stem cell or mouse embryo.

The knockout cells with the disrupted DNA of the present invention can be identified by the southern hybridization analysis using as a probe a DNA fragment on or near the DNA of the present invention, or by the PCR analysis using as primers a DNA sequence on the targeting vector and another DNA sequence at the proximal region of other than the DNA of the present invention derived from mouse used in the targeting vector. When non-human mammal stem cells are used, a cell line wherein the DNA of the present invention is inactivated by homologous recombination is cloned; the resulting clones are injected to, e.g., a non-human mammalian embryo or blastocyst, at an appropriate stage such as the 8-cell stage. The resulting chimeric embryos are transplanted to the uterus of the pseudopregnant non-human mammal. The resulting animal is a chimeric animal constructed with both cells having the normal locus of the DNA of the present invention and those having an artificially mutated locus of the DNA of the present invention.

When some germ cells of the chimeric animal have a mutated locus of the DNA of the present invention, an individual, which entire tissue is composed of cells having a mutated locus of the DNA of the present invention can be selected from a series of offspring obtained by crossing between such a chimeric animal and a normal animal, e.g., by coat color identification, etc. The individuals thus obtained are normally deficient in heterozygous expression of the EDG receptor. The individuals deficient in homozygous expression of the EDG receptor can be obtained from offspring of the intercross between those deficient in heterozygous expression of the EDG receptor.

When an oocyte is used, a DNA solution may be injected, e.g., into the prenucleus by microinjection thereby to obtain a transgenic non-human mammal having a targeting vector introduced in its chromosome. From such transgenic non-human mammals, those having a mutation at the locus of the DNA of the present invention can be obtained by selection based on homologous recombination.

As described above, the individuals in which the DNA of the present invention is rendered knockout permit passage rearing under ordinary rearing conditions, after the individuals obtained by their crossing have proven to have been knockout.

Furthermore, the genital system may be obtained and retained by conventional methods. That is, by crossing male and female animals each having the inactivated DNA, homozygous animals having the inactivated DNA in both loci can be obtained. The homozygotes thus obtained may be reared so that one normal animal and two or more homozygotes are produced from a mother animal to efficiently obtain such homozygotes. By crossing male and female heterozygotes, homozygotes and heterozygotes having the inactivated DNA are proliferated and passaged.

The non-human mammalian embryonic stem cell, in which the DNA of the present invention is inactivated, is very useful for preparing a non-human mammal deficient in expression of the DNA of the present invention.

Since the non-human mammal, in which the DNA of the present invention is inactivated, lacks various biological activities derived from the EDG receptor, such an animal can be a disease model suspected of inactivated biological activities of the EDG receptor and thus, offers an effective study to investigate the causes for and therapy for these diseases.

(14a) Method for screening a compound having therapeutic/preventive effects on diseases caused by deficiency, damages, etc. of the DNA of the present invention

The non-human mammal deficient in expression of the DNA of the present invention can be employed for screening of a compound having therapeutic/preventive effects on diseases caused by deficiency, damages, etc. of the DNA of the present invention.

That is, the present invention provides a method for screening a compound or its salt having a therapeutic/preventive effect on diseases caused by deficiency, damages, etc. of the DNA of the present invention, which comprises administering a test compound to a non-human mammal deficient in expression of the DNA of the present invention and, observing and measuring a change occurred in the animal.

As the non-human mammal deficient in expression of the DNA of the present invention, which can be employed for the screening method, the same examples as given hereinabove apply.

As the test compound, the same examples as described above are given.

Specifically, the non-human mammal deficient in expression of the DNA of the present invention is treated with a test compound, comparison is made with an intact animal for control and a change in each organ, tissue, disease conditions, etc. of the animal is used as an indicator to assess the therapeutic/preventive effects of the test compound.

For treating an animal with a test compound, for example, oral administration, intravenous injection, etc. are applied, and the treatment can be appropriately selected depending on conditions of the test animal, properties of the test compound, etc. Furthermore, a dose of the test compound to be administered can be appropriately chosen depending on the administration methods, property of the test compound, etc.

When a test compound is administered to a test animal in the screening method and the disease condition of the test animal is improved by at least about 10%, preferably at least 30%, more preferably at least about 50%, the test compound can be selected to be a compound having a therapeutic/preventive effect on diabetic nephropathy, chronic renal failure, nephritis, glomerulonephritis, interstitial renal disease or renal edema.

The compound or is salt, which is obtained by using the above screening method, is a compound selected from the test compounds described above and can be used as a safe and low toxic drug such as a prophylactic/therapeutic drug for diseases caused by deficiencies, damages, etc. of the EDG receptor (e.g., diabetic nephropathy, chronic renal failure, nephritis, glomerulonephritis, interstitial renal disease or renal edema). In addition, compounds or salts thereof derived from the compound or its salt obtained by the screening described above can be used as well.

As salts of the compound obtained by the screening method, there are used salts with physiologically acceptable acids (e.g., inorganic acids, organic acids, etc.) or bases (e.g., alkali metal salts), preferably physiologically acceptable acid addition salts. Examples of such salts used are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

The pharmaceutical comprising the compound or its salt obtained by the above screening method can be manufactured in a manner similar to the method for preparing the pharmaceutical comprising the EDG receptor described above, comprising the compound that changes the binding property of the EDG receptor to the ligand.

Since the pharmaceutical preparation thus obtained is safe and low toxic, it can be administered to human or mammal (e.g., rat, mouse, guinea pig, rabbit, ovine, swine, bovine, equine, feline, canine, smian, etc.).

The dose of the compound or its salt may vary depending upon target disease, subject to be administered, route for administration, etc. For example, when the compound or its salt is orally administered, it is generally administered to the patient (as 60 kg body weight) with diabetic nephropathy in a daily dose of about 0.1 to about 100 mg, preferably about 1.0 to about 50 mg and, more preferably about 1.0 to about 20 mg. In parenteral administration, a single dose may vary depending upon target subject, target disease, condition, route for administration etc. When the compound or its salt is administered in the form of an injectable preparation, it is advantageous to administer the compound or its salt intravenously to the patient (as 60 kg body weight) with diabetic nephropathy in a daily dose of about 0.01 to about 30 mg, preferably about 0.1 to about 20 mg, and more preferably about 0.1 to about 10 mg. For other animal species, the corresponding dose as converted per 60 kg body weight can be administered.

(14b) Method for screening a compound or its salt that promotes or inhibits the activity of a promoter to the DNA of the present invention

The present invention provides a method for screening a compound or its salt that promotes or inhibits the activity of a promoter to the DNA of the present invention, which comprises administering a test compound to a non-human mammal deficient in expression of the DNA of the present invention and detecting the expression of the reporter gene.

In the screening method described above, an animal in which the DNA of the present invention is inactivated by introducing a reporter gene and the reporter gene is expressed under control of a promoter to the DNA of the present invention is used as the non-human mammal deficient in expression of the DNA of the present invention, which is selected from the aforesaid non-human mammals deficient in expression of the DNA of the present invention.

As the test compound, the same examples as described above are given.

As the reporter gene used, the same specific examples as described above are given. Preferably, the reporter gene is β-galactosidase (lacZ), soluble alkaline phosphatase gene, luciferase gene, etc.

Since the reporter gene is present under control of a promoter to the DNA of the present invention in the non-human mammal deficient in expression of the DNA of the present invention wherein the DNA of the present invention is substituted with the reporter gene, the activity of the promoter can be detected by tracing the expression of a substance encoded by the reporter gene.

When a part of the DNA region encoding the EDG receptor is substituted with, e.g., β-galactosidase gene (lacZ) derived from *Escherichia coli,* β-galactosidase is expressed in a tissue where the EDG receptor should originally be expressed, instead of the EDG receptor. Thus, the expression state of the EDG receptor can be readily observed in vivo of an animal by staining with a reagent, e.g., 5-bromo-4-chloro-3-indolyl-β-galactopyranoside (X-gal) which is substrate for β-galactosidase. Specifically, a mouse deficient in the EDG receptor, or its tissue section is fixed with glutaraldehyde, etc. After washing with phosphate buffered saline (PBS), the system is reacted with a staining solution containing X-gal at room temperature or about 37°C for approximately 30 minutes to an hour. After the β-galactosidase reaction is terminated by washing the tissue preparation with 1 mM EDTA/PBS solution, the color formed is observed. Alternatively, mRNA encoding lacZ may be detected in a conventional manner.

The compound or salts thereof obtained using the screening method described above are compounds that are selected from the test compounds described above and that promote or inhibit the promoter activity to the DNA of the present invention.

As salts of the compound obtained by the screening method, there are used salts with physiologically acceptable acids (e.g., inorganic acids, organic acids, etc.) or bases (e.g., alkali metal salts), preferably physiologically acceptable acid addition salts. Examples of such salts used are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

Since the compound or its salt that promotes the promoter activity to the DNA of the present invention can promote the expression of the EDG receptor and can promote the function of the EDG receptor, it is useful as a drug such as a prophylactic/therapeutic agent for diseases associated with dysfunction of the EDG receptor.

Since the compound or its salt that inhibits the promoter activity to the DNA of the present invention can inhibit the expression of the EDG receptor and can inhibit the function of the EDG receptor, the compound or its salt is useful as a drug such as a prophylactic/therapeutic agent for diseases associated with overexpression of the EDG receptor.

Specifically, the compound that promotes or inhibits the promoter activity to the DNA encoding the EDG receptor, especially the compound that inhibits the promoter activity to the DNA encoding the EDG receptor is useful as a prophylactic/therapeutic agent for, e.g., diabetic nephropathy, chronic renal failure, nephritis, glomerulonephritis, interstitial renal disease, renal edema, etc.

In addition, compounds derived from the compound or its salt obtained by the screening described above can be used as well.

The pharmaceutical comprising the compound or its salt obtained by the above screening method can be manufactured in a manner similar to the method for preparing the pharmaceutical comprising the compound or its salt that changes the binding property of the EDG receptor or its salt to the ligand described above.

Since the pharmaceutical preparation thus obtained is safe and low toxic, it can be administered to, for example, human or mammal (e.g., rat, mouse, guinea pig, rabbit, ovine, swine, bovine, equine, feline, canine, smian, etc.).

The dose of the compound or its salt may vary depending upon target disease, subject to be administered, route of administration, etc. For example, when the compound that promotes or inhibits the promoter activity to the DNA of the present invention is orally administered, it is generally administered to the patient (as 60 kg body weight) with diabetic nephropathy in a daily dose of about 0.1 to about 100 mg, preferably about 1.0 to about 50 mg and, more preferably about 1.0 to about 20 mg. In parenteral administration, a single dose may vary depending upon target subject, target disease, condition, route for administration, etc. When the compound or its salt is administered in the form of an injectable preparation, it is advantageous to administer the compound intravenously to the patient (as 60 kg body weight) with diabetic nephropathy in a daily dose of about 0.01 to about 30 mg, preferably about 0.1 to about 20 mg, and more preferably about 0.1 to about 10 mg. For other animal species, the corresponding dose as converted per 60 kg body weight can be administered.

As stated above, the non-human mammal deficient in expression of the DNA of the present invention is extremely useful for screening the compound or its salt that promotes or inhibits the promoter activity to the DNA of the present invention and can greatly contribute to elucidation of causes for various diseases caused by deficiency in expression of the DNA of the present invention and for the development of prophylactic/therapeutic drugs.

Also, a so-called transgenic animal (gene transferred animal) can be prepared by using a DNA containing the promoter region of the EDG receptor, ligating genes encoding various proteins at the downstream and injecting the same into oocyte of an animal. It is thus possible to synthesize the receptor protein therein specifically and study its activity in vivo. When an appropriate reporter gene is ligated to the promoter site described above and a cell line that expresses the gene is established, the resulting system can be utilized as the search system for a low molecular compound having the action of specifically promoting or inhibiting the in vivo productivity of the EDG receptor itself.

(15) Method for elucidation of the action mechanism of various drugs

By using the EDG receptor, it can be confirmed whether or not various drugs exhibit their pharmacological effects mediated by the EDG receptor.

That is, the present invention provides the following methods.

(i) A method of confirmation that a prophylactic/therapeutic drug for diabetic nephropathy, chronic renal failure, nephritis, glomerulonephritis, interstitial renal disease or renal edema binds to the EDG receptor, which comprises using the EDG receptor.

(ii) A method of confirmation that a prophylactic/therapeutic drug for diabetic nephropathy, chronic renal failure, nephritis, glomerulonephritis, interstitial renal disease or renal edema is an agonist to the EDG receptor, which comprises using the EDG receptor.

(iii) A method of confirmation that a prophylactic/therapeutic drug for diabetic nephropathy, chronic renal failure, nephritis, glomerulonephritis, interstitial renal disease or renal edema is an antagonist to the EDG receptor, which comprises using the EDG receptor.

(iv) The screening method according to (i) to (iii), wherein the binding amount of each drug to the EDG receptor is measured when the drug is brought in contact with the EDG receptor.

This confirmation method can be performed by using the drug described above in place of the test compound in the aforesaid method of screening the compound or its salt that changes the binding property of the ligand to the EDG receptor described above.

The kit used for the confirmation method of the present invention comprises the drug described above in place of the test compound, in the aforesaid kit for screening the compound or its salt that changes the binding property of the ligand to the EDG receptor described above.

By using the confirmation method of the present invention as such, it can be confirmed that various drugs commercially available or under development exhibit their pharmacological effects mediated by the EDG receptor.

(List of abbreviations)

In the specification, where bases, amino acids, compounds, etc. are expressed in abbreviations, they are denoted by abbreviations in accordance with the IUPAC-IUB Commission on Biochemical Nomenclature or by conventional abbreviations in the art, examples of which are shown below. For amino acids that may have the optical isomer, L form is presented unless otherwise indicated.

| | |
|---|---|
| DNA : | deoxyribonucleic acid |
| cDNA : | complementary deoxyribonucleic acid |
| a or A : | adenine |
| t or T : | thymine |
| g or G : | guanine |
| c or C : | cytosine |
| u or U : | uracil |
| RNA : | ribonucleic acid |
| mRNA : | messenger ribonucleic acid |
| dATP : | deoxyadenosine triphosphate |
| dTTP : | deoxythymidine triphosphate |
| dGTP : | deoxyguanosine triphosphate |
| dCTP : | deoxycytidine triphosphate |
| ATP : | adenosine triphosphate |
| Gly : | glycine |
| Ala : | alanine |

| | |
|---|---|
| Val : | valine |
| Leu : | leucine |
| Ile : | isoleucine |
| Ser : | serine |
| Thr : | threonine |
| Cys : | cysteine |
| Met : | methionine |
| Glu : | glutamic acid |
| Asp : | aspartic acid |
| Lys : | lysine |
| Arg : | arginine |
| His : | histidine |
| Phe : | phenylalanine |
| Tyr : | tyrosine |
| Trp : | tryptophan |
| Pro : | proline |
| Asn : | asparagine |
| Gln : | glutamine |
| pGlu : | pyroglutamic acid |

The following is sequences described in the sequence listing of the present specification.

[SEQ ID NO: 1]

This shows the amino acid sequence of human EDG-2 receptor.

[SEQ ID NO: 2]

This shows the base sequence of cDNA encoding the amino acid sequence of human EDG-2 receptor.

[SEQ ID NO: 3]

This shows the amino acid sequence of rat EDG-2 receptor.

[SEQ ID NO: 4]

This shows the base sequence of cDNA encoding the amino acid sequence of rat EDG-2 receptor.

[SEQ ID NO: 5]

This shows the amino acid sequence of human EDG-3 receptor.

[SEQ ID NO: 6]

This shows the base sequence of cDNA encoding the amino acid sequence of human EDG-3 receptor.

[SEQ ID NO: 7]

This shows the amino acid sequence of rat EDG-3 receptor.

[SEQ ID NO: 8]

This shows the base sequence of cDNA encoding the amino acid sequence of rat EDG-3 receptor.

[SEQ ID NO: 9]

This shows the amino acid sequence of human EDG-5 receptor.

[SEQ ID NO: 10]

This shows the base sequence of cDNA encoding the amino acid sequence of human EDG-5 receptor.

[SEQ ID NO: 11]

This shows the amino acid sequence of rat EDG-5 receptor.

[SEQ ID NO: 12]

This shows the base sequence of cDNA encoding the amino acid sequence of rat EDG-5 receptor.

[SEQ ID NO: 13]

This shows the base sequence of the primer for EDG-1 receptor used in EXAMPLE 1.

[SEQ ID NO: 14]

This shows the base sequence of the primer for EDG-1 receptor used in EXAMPLE 1.

[SEQ ID NO: 15]

This shows the base sequence of the probe for EDG-1 receptor used in EXAMPLE 1.

[SEQ ID NO: 16]

This shows the base sequence of the primer for EDG-2 receptor used in

[SEQ ID NO: 17]

This shows the base sequence of the primer for EDG-2 receptor used in EXAMPLE 1.

[SEQ ID NO: 18]

This shows the base sequence of the probe for EDG-2 receptor used in EXAMPLE 1.

[SEQ ID NO: 19]

This shows the base sequence of the primer for EDG-3 receptor used in EXAMPLE 1.

[SEQ ID NO: 20]

This shows the base sequence of the primer for EDG-3 receptor used in EXAMPLE 1.

[SEQ ID NO: 21]

This shows the base sequence of the probe for EDG-3 receptor used in EXAMPLE 1.

[SEQ ID NO: 22]

This shows the base sequence of the primer for EDG-4 receptor used in EXAMPLE 1.

[SEQ ID NO: 23]

This shows the base sequence of the primer for EDG-4 receptor used in EXAMPLE 1.

[SEQ ID NO: 24]

This shows the base sequence of the probe for EDG-4 receptor used in EXAMPLE 1.

[SEQ ID NO: 25]

This shows the base sequence of the primer for EDG-5 receptor used in EXAMPLE 1.

[SEQ ID NO: 26]

This shows the base sequence of the primer for EDG-5 receptor used in EXAMPLE 1.

[SEQ ID NO: 27]

This shows the base sequence of the probe for EDG-5 receptor used in EXAMPLE 1.

[SEQ ID NO: 28]

This shows the base sequence of the primer for EDG-6 receptor used in EXAMPLE 1.

[SEQ ID NO: 29]

This shows the base sequence of the primer for EDG-6 receptor used in EXAMPLE 1.

[SEQ ID NO: 30]

This shows the base sequence of the probe for EDG-6 receptor used in EXAMPLE 1.

[SEQ ID NO: 31]

This shows the base sequence of the primer for EDG-7 receptor used in EXAMPLE 1.

[SEQ ID NO: 32]

This shows the base sequence of the primer for EDG-7 receptor used in EXAMPLE 1.

[SEQ ID NO: 33]

This shows the base sequence of the probe for EDG-7 receptor used in EXAMPLE 1.

[SEQ ID NO: 34]

This shows the base sequence of the primer for EDG-8 receptor used in EXAMPLE 1.

[SEQ ID NO: 35]

This shows the base sequence of the primer for EDG-8 receptor used in EXAMPLE 1.

[SEQ ID NO: 36]

This shows the base sequence of the probe for EDG-8 receptor used in EXAMPLE 1.

[SEQ ID NO: 37]

This shows the base sequence of the primer for EDG-2 receptor used in EXAMPLE 2.

[SEQ ID NO: 38]

This shows the base sequence of the primer for EDG-2 receptor used in EXAMPLE 2.

[SEQ ID NO: 39]

This shows the base sequence of the probe for EDG-2 receptor used in EXAMPLE 2.

[SEQ ID NO: 40]

This shows the base sequence of the primer for EDG-3 receptor used in EXAMPLE 2.

[SEQ ID NO: 41]

This shows the base sequence of the primer for EDG-3 receptor used in EXAMPLE 2.

[SEQ ID NO: 42]

This shows the base sequence of the probe for EDG-3 receptor used in EXAMPLE 2.

[SEQ ID NO: 43]

This shows the base sequence of the primer for EDG-5 receptor used in EXAMPLE 2.

[SEQ ID NO: 44]

This shows the base sequence of the primer for EDG-5 receptor used in EXAMPLE 2.

[SEQ ID NO: 45]

This shows the base sequence of the probe for EDG-5 receptor used in EXAMPLE 2.

EXAMPLES

The present invention is described in more detail below with reference to EXAMPLES, but is not deemed to limit the scope of the present invention thereto.

REFERENCE EXAMPLE 1

Gene expression analysis

In EXAMPLES below, human adult normal brain-derived mRNA sample was used and examined by the TaqMan method if mRNA derived from a gene belonging to the family of target G protein-coupled receptors, tyrosine phosphatase-type receptors, ion channels, etc. is present or absent and if any, its production level was quantified to analyze the expression level of the gene belonging to the family of target G protein-coupled receptors, tyrosine phosphatase-type receptors, ion channels, etc. In this EXAMPLE, expression of a gene belonging to the family of target G protein-coupled receptors (354 receptors), tyrosine phosphatase -type receptors, ion channels, etc using 384-well plates. As a target GPCR gene, for example, ORL; $M_1$; $M_2$; $M_3$; $M_4$; $M_5$; $A_1$; $A_{2A}$; $A_{2B}$; $A_3$; α1A; α1B; α1D; α2A; α2B; α2C; β1; β2; β3; AT1; AT2; BB1; BB2; bb3; $B_1$; $B_2$; CB1; CB2; CCR1; CCR2; CCR3; CCR4; CCR5; CCR6; CCR7; CCR8; CCR9; CCR10; CXCR1; CXCR2; CXCR3; CXCR4; CXCR5; $CX_3CR1$; XCR1; C3a; C5a; fMLP; $CCK_1$; $CCK_2$; $CRF_1$; $CRF_2$; D1; D2; D3; D4; D5; $ET_A$; $ET_B$; GAL1; GAL2; GAL3; $mglu_1$; $mglu_2$; $mglu_3$; $mglu_4$; $mglu_5$; $mglu_6$; $mglu_7$; $mglu_8$; FSH; LSH; TSH; $H_1$; $H_2$; $H_3$; $H_4$; 5-$HT_{1A}$; 5-$HT_{1B}$; 5-$HT_{1D}$; 5-$ht_{1B}$; 5-$ht_{1F}$; 5-$HT_{2A}$; 5-$HT_{2F}$; 5-$HT_{2C}$; 5-$HT_3$; 5-$HT_4$; 5-$ht_{5A}$; 5-$ht_{5B}$; 5-$HT_6$; 5-$HT_7$; BLT: CysLT1; $CysLT_2$; edg1; edg2; edg3; edg4; $MC_1$; $MC_2$; $MC_3$; $MC_4$; $MC_5$; $MT_1$; $MT_2$; $MT_3$; $Y_1$; $Y_2$; $Y_4$; $Y_5$; $Y_6$; NTS1; NTS2; DOP; KOP; MOP; NOP; $P2Y_1$; $P2Y_2$; $P2Y_4$; $P2Y_6$; $P2Y_{11}$; $P2Y_{12}$; PPAR-α; PPAR-β; PPAR-γ; DP; FP; IP; TP; $EP_1$; $EP_2$; $EP_3$; $EP_4$; PAR1; PAR2; PAR3; PAR4; $sst_1$; $sst_2$; $sst_3$; $sst_4$; $sst_5$; $NK_1$; $NK_2$; $NK_3$; $TRH_1$; $TRH_2$; $VPAC_1$; $VPAC_2$; $PAC_1$; $V_{1a}$; $V_{1b}$; $V_2$; OT; $Na^+$ channels (type I; type II/type IIA; type III; SCL11/NaG; PN1; NaCh6; NaDRG; SkM1/μI, SkM2); $K^+$ channels (Kv; EAG; KQT; IRK; ROMK; GIRK; $K_{ATP}$, etc.); $Ca^{2+}$ channels (α1G; α1E; α1S: α1C; α1D; α1B; α1A; IP3; ryanodine receptor, etc.); $Cl^-$ channels ($GABA_A$; $GABA_C$;

glycine receptor; C1C0; C1C1; CFTR, etc.); nonselective cation channels (nAChR; 5-$HT_3$; NMDA; AMPA; $P_{2X}$ATP; CNG, etc.) and the like are selected.

EXAMPLE 1

Expression of mRNA of the G protein-coupled receptor EDG family in human normal mesangial cells

Following the manual of Isogen (Nippon Gene Co., Ltd.), total RNA was prepared from normal human mesangial cells (purchased from Asahi Techno Glass Corp.). Using SuperScriptII reverse transcriptase (GIBCO BRL) as a reverse transcriptase, 1 μg of this RNA was reacted at 42°C as instructed in the manual attached. After the reaction was completed, ethanol precipitation was performed and the precipitates were dissolved in TE (corresponding to 100 ng/μl of RNA). The expression level of mRNA of the EDG family was quantified using the Sequence Detection System Prism 7900HT system (Applied Biosystems, Inc.). To quantify the expression level of each receptor, TaqMan probe and primer capable of specifically recognizing the receptor were designed and synthesized using Primer Express (software made by PE Applied Biosystems, Inc.).

For detecting EDG-1 receptor, 5'- CCACCGACCCATGTACTATTTT -3' (SEQ ID NO: 13), 5'-TGTAGGCTACTCCTGCCAACAG -3' (SEQ ID NO: 14) and 5'-(Fam)-TTGGCAATCTGGCCCTCTCAGA -(Tamra)-3' (SEQ ID NO: 15) as a TaqMan probe were used.

For detecting the EDG-2 receptor, 5'- ACTGTCAGCACATGGCTCCTT -3' (SEQ ID NO: 16), 5'-ACCGTAATGTGCCTCTCGATT -3' (SEQ ID NO: 17) and 5'-(Fam)-ATTGACACCAGCCTGACGGCAT -(Tamra)-3' (SEQ ID NO: 18) as a TaqMan probe were used.

For detecting the EDG-3 receptor, 5'- CCGTGCTCTTCTTGGTCAT-3' (SEQ ID NO: 19), 5'- CCAGATGGCAATCAAAACC -3' (SEQ ID NO: 20) and 5'-(Fam)-TGCAGCTTCATCGTCTTGGAGAACCT -(Tamra)-3' (SEQ ID NO: 21) as a TaqMan probe were used.

For detecting the EDG-4 receptor, 5'-CCTGGTCAAGACTGTTGTCATC-3' (SEQ ID NO: 22), 5'-CAGGACATTGCAGGACTCA -3' (SEQ ID NO: 23) and 5'-(Fam)-TGGTACTGCTCCTGGATGGTTTAGGCT -(Tamra)-3' (SEQ ID NO: 24) as a TaqMan probe were used.

For detecting the EDG-5 receptor, 5'- CCAACAAGGTCCAGGAACA-3' (SEQ ID NO: 25), 5'- AGGTTTTCCACCACAATGG -3' (SEQ ID NO: 26) and 5'-(Fam)-AATTATACCAAGGAGACGCTGGAAACGC -(Tamra)-3' (SEQ ID NO: 27) as a TaqMan probe were used.

For detecting the EDG-6 receptor, 5'- GAACTGCCTGTGCGCCTTT-3' (SEQ ID NO: 28), 5'- CCATAGAGGCCCATGATGGT -3' (SEQ ID NO: 29) and 5'-(Fam)-TCTGCCCCTCTACTCCAAGCGCTACATC-(Tamra)-3' (SEQ ID NO: 30) as a TaqMan probe were used.

For detecting the EDG-7 receptor, 5'- TGACTGCTTCCCTCACCAA-3' (SEQ ID NO: 31), 5'- GCATCCTCATGATTGACATGTG -3' (SEQ ID NO: 32) and 5'-(Fam)-TTGCTGGTTATCGCCGTGGAGA-(Tamra)-3' (SEQ ID NO: 33) as a TaqMan probe were used.

For detecting the EDG-8 receptor, 5'- CTTGCTCCACTGTCTTGCC-3' (SEQ ID NO: 34), 5'- TAGAGTGCACAGATCGCGG -3' (SEQ ID NO: 35) and 5'-(Fam)-CTCTACGCCAAGGCCTACGTGCTCTTCT-(Tamra)-3' (SEQ ID NO: 36) as a TaqMan probe were used.

Following the manual of TaqMan Universal PCR Master Mix (Applied Biosystems, Inc.), a reaction solution for quantification was prepared by adding a primer (0.9 μM) and a probe (0.25 μM) for each G protein-coupled receptor to cDNA in each amount corresponding to 25 ng of total RNA. PCR was carried out by reacting at 50°C for 2 minutes and 95°C for 10 minutes, and then repeating 40 times the cycle set to include 95°C for 15 seconds and 60°C for 1 minute. In human normal mesangial cells, the EDG-1 receptors showed 34,420 copies/25 ng total RNA,, EDG-2 receptors showed 624,726 copies/25 ng total RNA,, EDG-3 receptors showed 176,531 copies/25 ng total RNA, EDG-4 receptors showed 396 copies/25 ng total RNA, EDG-5 receptors showed 16,468, copies/25 ng total RNA, EDG-6 receptors showed 28 copies/25 ng total RNA, EDG-7 receptors showed 722 copies/25 ng total RNA and EDG-8 receptors showed 4,480 copies/25 ng total RNA. This indicates that the EDG receptor family, especially the EDG-1, EDG-2, EDG-3 and EDG-5 receptors are involved in renal diseases including diabetic nephropathy mediated by regulating proliferation of mesangial cells, glucose uptake, apoptosis, chemotaxis, etc.

EXAMPLE 2

Analysis of expression of the EDG receptor mRNA in the kidney of diabetic nephropathy model rats

Kidneys were extracted from Wistar Fatty and Wistar Lean rats of 42 weeks old and 68 weeks old and the total RNA was prepared using Isogen (Nippon Gene Co., Ltd.) in accordance with the manual. The total RNA (1 μg) was reverse-transcribed using a random primer. The reaction was carried out by using reverse transcriptase Super-

ScriptII (GIBCO BRL) according to the protocol attached, followed by ethanol precipitation. The precipitate was dissolved in 40 μl of TE. For quantification of mRNA expression level, ABI PRISM 7900HT (Applied Biosystems, Inc.) was used. Primers and TaqMan probes for amplification and detection were designed by utilizing Primer Express (Applied Biosystems, Inc.). The sequences are shown below.

[For the EDG-2 receptor]

5'-TGTCCCTAGACCCAAGAGACTTTAG-3' (SEQ ID NO: 37),
5'-GGTCCCCTTCTCTTTTCCAAA-3' (SEQ ID NO: 38),
5'-(Fam)-ATGAACTTGCTTGGTAGCCCCCATCTTC-(Tamra)-3' (SEQ ID NO: 39).

[For the EDG-3 receptor]

5'-ATCTTGTACGCGCGCATCTA-3' (SEQ ID NO: 40),
5'-TGGATCTCTCGGAGTTGTGGTT-3' (SEQ ID NO: 41),
5'-(Fam)-TGGTCAAGTCCAGCAGCCGCAG-(Tamra)-3' (SEQ ID NO: 42).

[For the EDG-5 receptor]

5'-GTTTGCCCGAGAGGGTTCA-3' (SEQ ID NO: 43),
5'-CTTGTCTCTCGATGGCAATGG-3' (SEQ ID NO: 44),
5'-(Fam)-CTTCATCACGCTCTCTGCCTCGGTCTT-(Tamra)-3' (SEQ ID NO: 45).

Following the protocol of TaqMan Universal PCR Master Mix (Applied Biosystems, Inc.), a reaction solution for quantification was prepared by adding a primer (0.9 μM), a probe (0.25 μM) and 1 μl of sample cDNA to the reaction mixture to make the volume 20 μl/well. The reaction was carried out on ABI PRISM 7900HT by reacting at 50°C (2 minutes) and 95°C (10 minutes), and then repeating 40 times the cycle set to include 95°C (15 seconds) and 60°C (1 minute).
GAPDH mRNA was quantified in accordance with the protocol, using TaqMan Rodent GAPDH Control Reagents (VIC probe) (Applied Biosystems, Inc.).
The expression level of each EDG receptor, etc. mRNA obtained was calibrated. The results reveal that the expression levels of the EDG-2, EDG-3 and EDG-5 receptors were high in the kidney from diabetic nephropathy model Wistar Fatty rat.

INDUSTRIAL APPLICABILITY

Agents for preventing/treating diabetic nephropathy, etc. can be efficiently screened by using the G protein-coupled receptor protein (the EDG-2, EDG-3 or EDG-5 receptor) comprising the same or substantially the same amino acid sequence as the amino acid sequence represented by SEQ ID NO: 1, SEQ ID NO: 5 or SEQ ID NO: 9, or a salt thereof.

## Claims

1. A prophylactic/therapeutic agent for diabetic nephropathy, chronic renal failure, nephritis, glomerulonephritis, interstitial renal disease or renal edema, comprising an EDG-2 receptor comprising the same or substantially the same amino acid sequence as the amino acid sequence represented by SEQ ID NO: 1, an EDG-3 receptor comprising the same or substantially the same amino acid sequence as the amino acid sequence represented by SEQ ID NO: 5, an EDG-5 receptor comprising the same or substantially the same amino acid sequence as the amino acid sequence represented by SEQ ID NO: 9, its partial peptide, or a salt thereof.

2. A prophylactic/therapeutic agent for diabetic nephropathy, chronic renal failure, nephritis, glomerulonephritis, interstitial renal disease or renal edema, comprising a polynucleotide comprising a polynucleotide encoding an EDG-2 receptor comprising the same or substantially the same amino acid sequence as the amino acid sequence represented by SEQ ID NO: 1, an EDG-3 receptor comprising the same or substantially the same amino acid sequence as the amino acid sequence represented by SEQ ID NO: 5, an EDG-5 receptor comprising the same or substantially the same amino acid sequence as the amino acid sequence represented by SEQ ID NO: 9, or a partial peptide thereof.

3. A diagnostic agent for diabetic nephropathy, chronic renal failure, nephritis, glomerulonephritis, interstitial renal disease or renal edema, comprising a polynucleotide comprising a polynucleotide encoding an EDG-2 receptor comprising the same or substantially the same amino acid sequence as the amino acid sequence represented by SEQ ID NO: 1, an EDG-3 receptor comprising the same or substantially the same amino acid sequence as the amino acid sequence represented by SEQ ID NO: 5, an EDG-5 receptor comprising the same or substantially the same amino acid sequence as the amino acid sequence represented by SEQ ID NO: 9, or a partial peptide thereof.

4. A prophylactic/therapeutic agent for diabetic nephropathy, chronic renal failure, nephritis, glomerulonephritis, interstitial renal disease or renal edema, comprising an antibody to an EDG-2 receptor comprising the same or substantially the same amino acid sequence as the amino acid sequence represented by SEQ ID NO: 1, an EDG-3 receptor comprising the same or substantially the same amino acid sequence as the amino acid sequence represented by SEQ ID NO: 5, an EDG-5 receptor comprising the same or substantially the same amino acid sequence as the amino acid sequence represented by SEQ ID NO: 9, its partial peptide, or a salt thereof.

5. A diagnostic agent for diabetic nephropathy, chronic renal failure, nephritis, glomerulonephritis, interstitial renal disease or renal edema, comprising an antibody to an EDG-2 receptor comprising the same or substantially the same amino acid sequence as the amino acid sequence represented by SEQ ID NO: 1, an EDG-3 receptor comprising the same or substantially the same amino acid sequence as the amino acid sequence represented by SEQ ID NO: 5, an EDG-5 receptor comprising the same or substantially the same amino acid sequence as the amino acid sequence represented by SEQ ID NO: 9, its partial peptide, or a salt thereof.

6. A prophylactic/therapeutic agent for diabetic nephropathy, chronic renal failure, nephritis, glomerulonephritis, interstitial renal disease or renal edema, comprising a polynucleotide comprising the entire or part of a base sequence complementary to a polynucleotide comprising a polynucleotide encoding an EDG-2 receptor comprising the same or substantially the same amino acid sequence as the amino acid sequence represented by SEQ ID NO: 1, an EDG-3 receptor comprising the same or substantially the same amino acid sequence as the amino acid sequence represented by SEQ ID NO: 5, an EDG-5 receptor comprising the same or substantially the same amino acid sequence as the amino acid sequence represented by SEQ ID NO: 9, or a partial peptide thereof.

7. A diagnostic agent for diabetic nephropathy, chronic renal failure, nephritis, glomerulonephritis, interstitial renal disease or renal edema, comprising a polynucleotide comprising the entire or part of a base sequence complementary to a polynucleotide comprising a polynucleotide encoding an EDG-2 receptor comprising the same or substantially the same amino acid sequence as the amino acid sequence represented by SEQ ID NO: 1, an EDG-3 receptor comprising the same or substantially the same amino acid sequence as the amino acid sequence represented by SEQ ID NO: 5, an EDG-5 receptor comprising the same or substantially the same amino acid sequence as the amino acid sequence represented by SEQ ID NO: 9, or a partial peptide thereof.

**8.** A method of screening a preventive/therapeutic drug for diabetic nephropathy, chronic renal failure, nephritis, glomerulonephritis, interstitial renal disease or renal edema, which comprises using (i) lysophosphatidic acid or a salt thereof and (ii) an EDG-2 receptor comprising the same or substantially the same amino acid sequence as the amino acid sequence represented by SEQ ID NO: 1, its partial peptide, or a salt thereof, to screen a compound or a salt thereof that changes the binding property of lysophosphatidic acid or a salt thereof to said EDG-2 receptor or a salt thereof.

**9.** A kit for screening a preventive/therapeutic drug for diabetic nephropathy, chronic renal failure, nephritis, glomerulonephritis, interstitial renal disease or renal edema, comprising (i) lysophosphatidic acid or a salt thereof, and (ii) an EDG-2 receptor comprising the same or substantially the same amino acid sequence as the amino acid sequence represented by SEQ ID NO: 1, its partial peptide, or a salt thereof.

**10.** A method of screening a preventive/therapeutic drug for diabetic nephropathy, chronic renal failure, nephritis, glomerulonephritis, interstitial renal disease or renal edema, which comprises using (i) sphingosine-1-phosphate or a salt thereof, and (ii) an EDG-3 receptor comprising the same or substantially the same amino acid sequence as the amino acid sequence represented by SEQ ID NO: 5, its partial peptide, or a salt thereof, to screen a compound or a salt thereof that changes the binding property of sphingosine-1-phosphate or a salt thereof and said EDG-3 receptor or a salt thereof.

**11.** A kit for screening a preventive/therapeutic drug for diabetic nephropathy, chronic renal failure, nephritis, glomerulonephritis, interstitial renal disease or renal edema, comprising (i) sphingosine-1-phosphate or a salt thereof, and (ii) an EDG-3 receptor comprising the same or substantially the same amino acid sequence as the amino acid sequence represented by SEQ ID NO: 5, its partial peptide, or a salt thereof.

**12.** A method of screening a preventive/therapeutic drug for diabetic nephropathy, chronic renal failure, nephritis, glomerulonephritis, interstitial renal disease or renal edema, which comprises using (i) sphingosine-1-phosphate or a salt thereof, and (ii) an EDG-5 receptor comprising the same or substantially the same amino acid sequence as the amino acid sequence represented by SEQ ID NO: 9, its partial peptide, or a salt thereof, to screen a compound or a salt thereof that changes the binding property of sphingosine-1-phosphate or a salt thereof and said EDG-5 receptor or a salt thereof.

**13.** A kit for screening a preventive/therapeutic drug for diabetic nephropathy, chronic renal failure, nephritis, glomerulonephritis, interstitial renal disease or renal edema, comprising (i) sphingosine-1-phosphate or a salt thereof, and (ii) an EDG-5 receptor comprising the same or substantially the same amino acid sequence as the amino acid sequence represented by SEQ ID NO: 9, its partial peptide, or a salt thereof.

**14.** A prophylactic/therapeutic agent for diabetic nephropathy, chronic renal failure, nephritis, glomerulonephritis, interstitial renal disease or renal edema, comprising (i) lysophosphatidic acid or a salt thereof and (ii) a compound or a salt thereof that changes the binding property of an EDG-2 receptor comprising the same or substantially the same amino acid sequence as the amino acid sequence represented by SEQ ID NO: 1, its partial peptide, or a salt thereof.

**15.** A prophylactic/therapeutic agent for diabetic nephropathy, chronic renal failure, nephritis, glomerulonephritis, interstitial renal disease or renal edema, comprising (i) sphingosine-1-phosphate or a salt thereof and (ii) a compound or a salt thereof that changes the binding property of an EDG-3 receptor comprising the same or substantially the same amino acid sequence as the amino acid sequence represented by SEQ ID NO: 5, its partial peptide, or a salt thereof.

**16.** A prophylactic/therapeutic agent for diabetic nephropathy, chronic renal failure, nephritis, glomerulonephritis, interstitial renal disease or renal edema, comprising (i) sphingosine-1-phosphate or a salt thereof and (ii) a compound or a salt thereof that changes the binding property of an EDG-5 receptor comprising the same or substantially the same amino acid sequence as the amino acid sequence represented by SEQ ID NO: 9, its partial peptide, or a salt thereof.

**17.** A method of screening a compound for preventing/treating diabetic nephropathy, chronic renal failure, nephritis, glomerulonephritis, interstitial renal disease or renal edema, or a salt thereof that changes an expression level of an EDG-2 receptor comprising the same or substantially the same amino acid sequence as the amino acid sequence represented by SEQ ID NO: 1, an EDG-3 receptor comprising the same or substantially the same amino acid sequence as the amino acid sequence represented by SEQ ID NO: 5, or an EDG-5 receptor comprising the same or substantially the same amino acid sequence as the amino acid sequence represented by SEQ ID NO: 9, which comprises using a polynucleotide comprising a polynucleotide encoding said EDG-2 receptor, EDG-3 receptor or EDG-5 receptor, or a partial peptide thereof.

**18.** A kit for screening a compound for preventing/treating diabetic nephropathy, chronic renal failure, nephritis, glomerulonephritis, interstitial renal disease or renal edema, or a salt thereof that changes an expression level of an EDG-2 receptor comprising the same or substantially the same amino acid sequence as the amino acid sequence represented by SEQ ID NO: 1, an EDG-3 receptor comprising the same or substantially the same amino acid sequence as the amino acid sequence represented by SEQ ID NO: 5, or an EDG-5 receptor comprising the same or substantially the same amino acid sequence as the amino acid sequence represented by SEQ ID NO: 9, comprising a polynucleotide comprising a polynucleotide encoding said EDG-2 receptor, EDG-3 receptor or EDG-5 receptor, or a partial peptide thereof.

**19.** A prophylactic/therapeutic agent for diabetic nephropathy, chronic renal failure, nephritis, glomerulonephritis, interstitial renal disease or renal edema, comprising a compound or its salt that changes an expression level of an EDG-2 receptor comprising the same or substantially the same amino acid sequence as the amino acid sequence represented by SEQ ID NO: 1, an EDG-3 receptor comprising the same or substantially the same amino acid sequence as the amino acid sequence represented by SEQ ID NO: 5, or an EDG-5 receptor comprising the same or substantially the same amino acid sequence as the amino acid sequence represented by SEQ ID NO: 9.

**20.** A method for preventing/treating diabetic nephropathy, chronic renal failure, nephritis, glomerulonephritis, interstitial renal disease or renal edema, which comprises administering to a mammal an effective dose of (1) (i) lysophosphatidic acid or a salt thereof and (ii) a compound or a salt thereof that changes the binding property of an EDG-2 receptor comprising the same or substantially the same amino acid sequence as the amino acid sequence represented by SEQ ID NO: 1, its partial peptide, or a salt thereof, (2) (i) sphingosine-1-phosphate or a salt thereof and (ii) a compound or a salt thereof that changes the binding property of an EDG-3 receptor comprising the same or substantially the same amino acid sequence as the amino acid sequence represented by SEQ ID NO: 5, its partial peptide, or a salt thereof, (3) (i) sphingosine-1-phosphate or a salt thereof and (ii) a compound or a salt thereof that changes the binding property of an EDG-5 receptor comprising the same or substantially the same amino acid sequence as the amino acid sequence represented by SEQ ID NO: 9, its partial peptide, or a salt thereof, or (4) a compound or its salt that changes an expression level of an EDG-2 receptor comprising the same or substantially the same amino acid sequence as the amino acid sequence represented by SEQ ID NO: 1, an EDG-3 receptor comprising the same or substantially the same amino acid sequence as the amino acid sequence represented by SEQ ID NO: 5, or an EDG-5 receptor comprising the same or substantially the same amino acid sequence as the amino acid sequence represented by SEQ ID NO: 9.

**21.** Use of (1) (i) lysophosphatidic acid or a salt thereof and (ii) a compound or a salt thereof that changes the binding property of an EDG-2 receptor comprising the same or substantially the same amino acid sequence as the amino acid sequence represented by SEQ ID NO: 1, its partial peptide, or a salt thereof, (2) (i) sphingosine-1-phosphate or a salt thereof and (ii) a compound or a salt thereof that changes the binding property of an EDG-3 receptor comprising the same or substantially the same amino acid sequence as the amino acid sequence represented by SEQ ID NO: 5, its partial peptide, or a salt thereof, (3) (i) sphingosine-1-phosphate or a salt thereof and (ii) a compound or a salt thereof that changes the binding property of an EDG-5 receptor comprising the same or substantially the same amino acid sequence as the amino acid sequence represented by SEQ ID NO: 9, its partial peptide, or a salt thereof, or (4) a compound or its salt that changes an expression level of an EDG-2 receptor comprising the same or substantially the same amino acid sequence as the amino acid sequence represented by SEQ ID NO: 1, an EDG-3 receptor comprising the same or substantially the same amino acid sequence as the amino acid sequence represented by SEQ ID NO: 5, or an EDG-5 receptor comprising the same or substantially the same amino acid sequence as the amino acid sequence represented by SEQ ID NO: 9, to manufacture a prophylactic/therapeutic agent for diabetic nephropathy, chronic renal failure, nephritis, glomerulonephritis, interstitial renal disease or renal edema.

FIG. 1

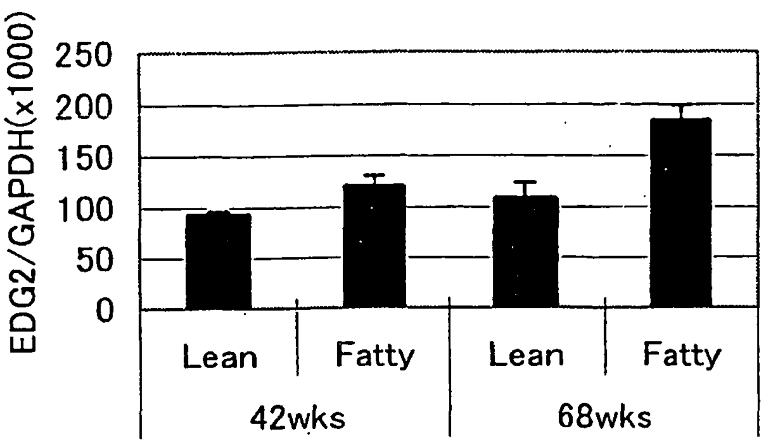
EDG2/GAPDH(x1000)
250
200
150
100
50
0
Lean
Fatty
Lean
Fatty
42wks
68wks

FIG. 2

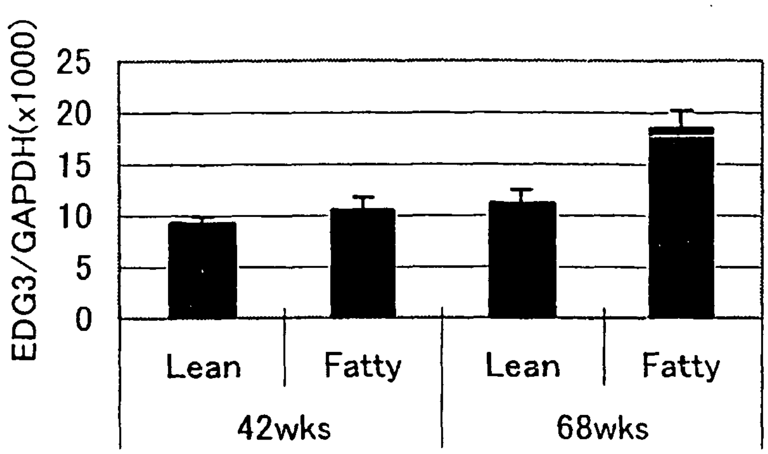
EDG3/GAPDH(x1000)
25
20
15
10
5
0
Lean
Fatty
Lean
Fatty
42wks
68wks

FIG.3

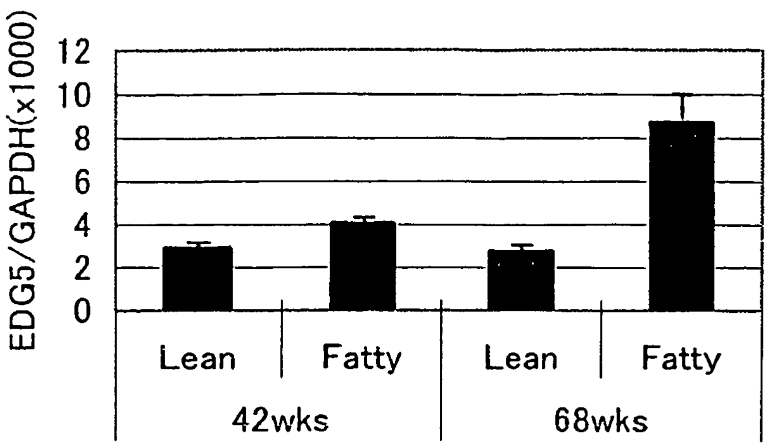
EDG5/GAPDH(x1000)
12
10
8
6
4
2
0
Lean
Fatty
Lean
Fatty
42wks
68wks

## INTERNATIONAL SEARCH REPORT

International application No.
PCT/JP03/15836

A. CLASSIFICATION OF SUBJECT MATTER
Int.Cl[7] A61K38/17, 31/7088, 39/395, 45/00, 48/00, A61P13/12

According to International Patent Classification (IPC) or to both national classification and IPC

B. FIELDS SEARCHED

Minimum documentation searched (classification system followed by classification symbols)
Int.Cl[7] A61K38/17, 31/7088, 39/395, 45/00, 48/00, A61P13/12

Documentation searched other than minimum documentation to the extent that such documents are included in the fields searched

Electronic data base consulted during the international search (name of data base and, where practicable, search terms used)
MEDLINE, CAPLUS, EMBASE, BIOSIS(STN), JMEDPLUS(JOIS)

C. DOCUMENTS CONSIDERED TO BE RELEVANT

| Category* | Citation of document, with indication, where appropriate, of the relevant passages | Relevant to claim No. |
|---|---|---|
| X | WO 02/077642 A1 (Nippon Shinyaku Co., Ltd.), 02 October, 2002 (02.10.02), Full text; Figs. 1 to 5 (Family: none) | 1 |
| X | Inoue CN. et al., 'Lysophosphatidic acid and mesangial cells: implications for renal diseases.', Clin.Sci.(Lond)., 1999 April; 96(4): 431-6. | 1 |
| X | Katsuma S. et al., 'Genomic analysis of a mouse model of immunoglobulin A nephropathy reveals an enhanced PDGF-EDG5 cascade.', Pharmacogenomics J.2001; 1(3):211-7. | 1 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 19 March, 2004 (19.03.04) | 06 April, 2004 (06.04.04) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP03/15836

C (Continuation). DOCUMENTS CONSIDERED TO BE RELEVANT

| Category* | Citation of document, with indication, where appropriate, of the relevant passages | Relevant to claim No. |
|---|---|---|
| X | Hanafusa N. et al., 'Sphingosine 1-phosphate stimulates rat mesangial cell proliferation from outside the cells.', Nephrol.Dial.Transplant. 2002 April; 17(4):580-6. | 1 |
| A | EP 1195165 A1 (ONO PHARMACEUTICAL CO.), 10 April, 2002 (10.04.02), Full text & WO 01/03739 A1 | 1 |
| A | WO 99/33972 A1 (ALLELIX BIOPHARMA), 08 July, 1999 (08.07.99), Full text & JP 2002-500008 A | 1 |
| P,X | JP 2002-360118 A (Nippon Shinyaku Co., Ltd.), 17 December, 2002 (17.12.02), Full text (Family: none) | 1 |
| E,A | WO 03/051876 A1 (Japan Tobacco Inc.), 26 June, 2003 (26.06.03), (Family: none) | 1 |
| E,A | WO 2004/002430 A1 (ONO PHARMACEUTICAL CO.), 08 January, 2004 (08.01.04), (Family: none) | 1 |
| E,A | WO 03/099765 A1 (ONO PHARMACEUTICAL CO.), 08 December, 2003 (08.12.03), (Family: none) | 1 |

Form PCT/ISA/210 (continuation of second sheet) (July 1998)

**INTERNATIONAL SEARCH REPORT**

International application No.
PCT/JP03/15836

**Box I Observations where certain claims were found unsearchable (Continuation of item 2 of first sheet)**

This international search report has not been established in respect of certain claims under Article 17(2)(a) for the following reasons:

1. [X] Claims Nos.: 20
because they relate to subject matter not required to be searched by this Authority, namely:
Claim 20 pertains to methods for treatment of the human body by therapy and thus relates to a subject matter which this International Searching Authority is not required, under the provisions of Article 17(2)(a)(i) of the PCT and Rule 39.1(iv) of the Regulations under the PCT, to search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:
(See extra sheet.)

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [X] No required additional search fees were timely paid by the applicant. Consequently, this international search report is restricted to the invention first mentioned in the claims; it is covered by claims Nos.:
The part using EDG-5 receptor in claim 1.

**Remark on Protest** [ ] The additional search fees were accompanied by the applicant's protest.
[ ] No protest accompanied the payment of additional search fees.

Form PCT/ISA/210 (continuation of first sheet (1)) (July 1998)

**INTERNATIONAL SEARCH REPORT**

International application No. PCT/JP03/15836

Continuation of Box No. II of continuation of first sheet(1)

Claims 1 to 19 and 21

It is recognized that the following 18 groups of inventions are described in the above claims.

(1) The part using EDG-2 receptor in claim 1.
(2) The part using EDG-3 receptor in claim 1.
(3) The part using EDG-5 receptor in claim 1.
(4) The parts relating to EDG-2 receptor in claims 2 and 3.
(5) The parts relating to EDG-3 receptor in claims 2 and 3.
(6) The parts relating to EDG-5 receptor in claims 2 and 3.
(7) The parts relating to EDG-2 receptor in claims 4 and 5.
(8) The parts relating to EDG-3 receptor in claims 4 and 5.
(9) The parts relating to EDG-5 receptor in claims 4 and 5.
(10) The parts relating to EDG-2 receptor in claims 6 and 7.
(11) The parts relating to EDG-3 receptor in claims 6 and 7.
(12) The parts relating to EDG-5 receptor in claims 6 and 7.
(13) The parts relating to (1) in claim 21 in claims 8, 9 and 14.
(14) The parts relating to (2) in claim 21 in claims 10, 11 and 15.
(15) The parts relating to (3) in claim 21 in claims 12, 13 and 16.
(16) The parts relating to EDG-2 receptor in claims 17 to 19 and the parts relating to (4) EDG-2 receptor in claim 21.
(17) The parts relating to EDG-3 receptor in claims 17 to 19 and the parts relating to (4) EDG-3 receptor in claim 21.
(18) The parts relating to EDG-5 receptor in claims 17 to 19 and the parts relating to (4) EDG-5 receptor in claim 21.

(1) to (3)

The invention according to claim 1 relates to a preventive/remedy for diabetic nephropathy, chronic renal failure and so on containing EDG-2 receptor, EDG-3 receptor or EDG-5 receptor or its peptide fragment or its salt.

As stated in the description of the present application (p. 12, 1.27 to p.13, 1. 12), the above-described receptors are each publicly known. Moreover, it seems that these receptors do not always have a property or an activity in common in the cause of using for medicinal purposes, referring to WO 02/077642 A1, etc. Such being the case, it does not appear that there is any technical relationship involving "a special technical feature" among (1) to (3) and thus these groups of inventions do not comply with the requirement of unity of invention.

(4) to (12)

"An amino acid sequence, its peptide fragment or its salt" (claim 1), "a polynucleotide" (claims 2 and 3), "an antibody" (claims 4 and 5) and "a polynucleotide having a complementary base sequence or a part thereof" (claims 6 and 7) are different substances from each other. Thus, it does not appear that there is any technical relationship involving "a special technical feature" among preventives/diagnostics or remedies comprising the same and thus these groups of inventions do not comply with the requirement of unity of invention.

(13) to (18)

Considering that WO 02/077642 A1 has been publicly known, it does not appear that there is any technical relationship involving "a special technical feature" among (1) to (3) and (13) to (18) and thus these groups of inventions do not comply with the requirement of unity of invention.

Form PCT/ISA/210 (extra sheet) (July 1998)

SEQUENCE LISTING
<110> Takeda Chemical Industries, Ltd.

<120> Novel Use of EDG Receptor

<130> 3127WOOP

<150> JP 2002-361415
<151> 2002-12-12

<160> 45

<210> 1
<211> 364
<212> PRT
<213> Homo sapiens

<400> 1
Met Ala Ala Ile Ser Thr Ser Ile Pro Val Ile Ser Gln Pro Gln Phe
5 10 15
Thr Ala Met Asn Glu Pro Gln Cys Phe Tyr Asn Glu Ser Ile Ala Phe
20 25 30
Phe Tyr Asn Arg Ser Gly Lys His Leu Ala Thr Glu Trp Asn Thr Val
35 40 45
Ser Lys Leu Val Met Gly Leu Gly Ile Thr Val Cys Ile Phe Ile Met
50 55 60
Leu Ala Asn Leu Leu Val Met Val Ala Ile Tyr Val Asn Arg Arg Phe
65 70 75 80
His Phe Pro Ile Tyr Tyr Leu Met Ala Asn Leu Ala Ala Ala Asp Phe
85 90 95
Phe Ala Gly Leu Ala Tyr Phe Tyr Leu Met Phe Asn Thr Gly Pro Asn
100 105 110
Thr Arg Arg Leu Thr Val Ser Thr Trp Leu Leu Arg Gln Gly Leu Ile
115 120 125
Asp Thr Ser Leu Thr Ala Ser Val Ala Asn Leu Leu Ala Ile Ala Ile
130 135 140
Glu Arg His Ile Thr Val Phe Arg Met Gln Leu His Thr Arg Met Ser
145 150 155 160
Asn Arg Arg Val Val Val Val Ile Val Val Ile Trp Thr Met Ala Ile
165 170 175
Val Met Gly Ala Ile Pro Ser Val Gly Trp Asn Cys Ile Cys Asp Ile
180 185 190
Glu Asn Cys Ser Asn Met Ala Pro Leu Tyr Ser Asp Ser Tyr Leu Val
195 200 205
Phe Trp Ala Ile Phe Asn Leu Val Thr Phe Val Val Met Val Val Leu
210 215 220
Tyr Ala His Ile Phe Gly Tyr Val Arg Gln Arg Thr Met Arg Met Ser
225 230 235 240
Arg His Ser Ser Gly Pro Arg Arg Asn Arg Asp Thr Met Met Ser Leu
245 250 255
Leu Lys Thr Val Val Ile Val Leu Gly Ala Phe Ile Ile Cys Trp Thr
260 265 270
Pro Gly Leu Val Leu Leu Leu Leu Asp Val Cys Cys Pro Gln Cys Asp
275 280 285
Val Leu Ala Tyr Glu Lys Phe Phe Leu Leu Leu Ala Glu Phe Asn Ser
290 295 300
Ala Met Asn Pro Ile Ile Tyr Ser Tyr Arg Asp Lys Glu Met Ser Ala
305 310 315 320
Thr Phe Arg Gln Ile Leu Cys Cys Gln Arg Ser Glu Asn Pro Thr Gly
325 330 335
Pro Thr Glu Gly Ser Asp Arg Ser Ala Ser Ser Leu Asn His Thr Ile
340 345 350
Leu Ala Gly Val His Ser Asn Asp His Ser Val Val
355 360

<210> 2
<211> 1092
<212> DNA

<213> Homo sapiens

<400> 2
atggctgcca tctctacttc catccctgta atttcacagc cccagttcac agccatgaat 60
gaaccacagt gcttctacaa cgagtccatt gccttctttt ataaccgaag tggaaagcat 120
cttgccacag aatggaacac agtcagcaag ctggtgatgg gacttggaat cactgtttgt 180
atcttcatca tgttggccaa cctattggtc atggtggcaa tctatgtcaa ccgccgcttc 240
cattttccta tttattacct aatggctaat ctggctgctg cagacttctt tgctgggttg 300
gcctacttct atctcatgtt caacacagga cccaatactc ggagactgac tgttagcaca 360
tggctcctgc gtcagggcct cattgacacc agcctgacgg catctgtggc caacttactg 420
gctattgcaa tcgagaggca cattacggtt ttccgcatgc agctccacac acggatgagc 480
aaccggcggg tagtggtggt cattgtggtc atctggacta tggccatcgt tatgggtgct 540
atacccagtg tgggctggaa ctgtatctgt gatattgaaa attgttccaa catggcaccc 600
ctctacagtg actcttactt agtcttctgg gccattttca acttggtgac ctttgtggta 660
atggtggttc tctatgctca catctttggc tatgttcgcc agaggactat gagaatgtct 720
cggcatagtt ctggaccccg gcggaatcgg gataccatga tgagtcttct gaagactgtg 780
gtcattgtgc ttggggcctt tatcatctgc tggactcctg gattggtttt gttacttcta 840
gacgtgtgct gtccacagtg cgacgtgctg gcctatgaga aattcttcct tctccttgct 900
gaattcaact ctgccatgaa ccccatcatt tactcctacc gcgacaaaga aatgagcgcc 960
acctttaggc agatcctctg ctgccagcgc agtgagaacc ccaccggccc cacagaaggc 1020
tcagaccgct cggcttcctc cctcaaccac accatcttgg ctggagttca cagcaatgac 1080
cactctgtgg tt 1092

<210> 3
<211> 364
<212> PRT
<213> Rattus spp

<400> 3
Met Ala Ala Ala Ser Thr Ser Ser Pro Val Ile Ser Gln Pro Gln Phe
1 5 10 15
Thr Ala Met Asn Glu Gln Gln Cys Phe Tyr Asn Glu Ser Ile Ala Phe
20 25 30
Phe Tyr Asn Arg Ser Gly Lys Tyr Leu Ala Thr Glu Trp Asn Thr Val
35 40 45
Ser Lys Leu Val Met Gly Leu Gly Ile Thr Val Cys Val Phe Ile Met
50 55 60
Leu Ala Asn Leu Leu Val Met Val Ala Ile Tyr Val Asn Arg Arg Phe
65 70 75 80
His Phe Pro Ile Tyr Tyr Leu Met Ala Asn Leu Ala Ala Ala Asp Phe
85 90 95
Phe Ala Gly Leu Ala Tyr Phe Tyr Leu Met Phe Asn Thr Gly Pro Asn
100 105 110
Thr Arg Arg Leu Thr Val Ser Thr Trp Leu Leu Arg Gln Gly Leu Ile
115 120 125
Asp Thr Ser Leu Thr Ala Ser Val Ala Asn Leu Leu Ala Ile Ala Ile
130 135 140
Glu Arg His Ile Thr Val Phe Arg Met Gln Leu His Thr Arg Met Ser
145 150 155 160
Asn Arg Arg Val Val Val Val Ile Val Val Ile Trp Thr Met Ala Ile
165 170 175
Val Met Gly Ala Ile Pro Ser Val Gly Trp Asn Cys Ile Cys Asp Ile
180 185 190
Asp His Cys Ser Asn Met Ala Pro Leu Tyr Ser Asp Ser Tyr Leu Val
195 200 205
Phe Trp Ala Ile Phe Asn Leu Val Thr Phe Val Val Met Val Val Leu
210 215 220
Tyr Ala His Ile Phe Gly Tyr Val Arg Gln Arg Thr Met Arg Met Ser
225 230 235 240
Arg His Ser Ser Gly Pro Arg Arg Asn Arg Asp Thr Met Met Ser Leu
245 250 255
Leu Lys Thr Val Val Ile Val Leu Gly Ala Phe Ile Val Cys Trp Thr
260 265 270
Pro Gly Leu Val Leu Leu Leu Leu Asp Val Cys Cys Pro Gln Cys Asp
275 280 285
Val Leu Ala Tyr Glu Lys Phe Phe Leu Leu Leu Ala Glu Phe Asn Ser
290 295 300
Ala Met Asn Pro Ile Ile Tyr Ser Tyr Arg Asp Lys Glu Met Ser Ala

```
305                 310                 315                 320
Thr Phe Arg Gln Ile Leu Cys Cys Gln Arg Asn Glu Asn Pro Asn Gly
                325                 330                 335
Pro Thr Glu Gly Ser Asp Arg Ser Ala Ser Ser Leu Asn His Thr Ile
            340                 345                 350
Leu Ala Gly Val His Ser Asn Asp His Ser Val Val
        355                 360
```

<210> 4
<211> 1092
<212> DNA
<213> Rattus spp

<400> 4

```
atggcagctg cctctacttc cagccctgtg atttcacagc cccagttcac agccatgaac     60
gaacaacagt gcttctacaa cgagtctatc gccttcttct ataaccggag tggaaagtat    120
ctagccacag aatggaacac tgtgagcaag ctggtgatgg gactgggcat cactgtctgc    180
gtgttcatca tgctggccaa tctactggtc atggtggcaa tttacgtcaa ccgccgcttc    240
catttcccta tttattactt gatggccaac ctggctgctg cagacttctt cgctggactg    300
gcctacttct acctgatgtt caacacggga cctaataccc ggagactgac cgtgagcaca    360
tggcttctcc ggcagggcct catcgacacc agcctgacgg cttctgtggc caacctgctg    420
gccattgcca tcgagaggca catcacagtt ttccgaatgc agctccatac acgaatgagc    480
aaccgacgtg tggtggtggt gattgtagtc atctggacta tggccattgt gatgggtgcc    540
atacccagtg tgggctggaa ctgcatctgt gatatcgatc attgttccaa catggcgccc    600
ctctacagtg actcctactt agtcttctgg gccattttca acctggtgac ctttgtggtc    660
atggtggttc tctacgctca catctttggc tatgttcgcc agaggactat gagaatgtcc    720
cggcatagtt ctggacccag gaggaatcgg gacaccatga tgagccttct gaagactgtg    780
gtcattgtgc tgggtgcctt tattgtctgc tggactccgg gattggtctt gctactgctc    840
gatgtgtgtt gcccgcagtg cgatgtcctg gcctatgaga agttcttcct cctcctggcc    900
gagttcaact ctgctatgaa ccccatcatc tactcctacc gcgacaaaga gatgagcgcc    960
accttcaggc agatcctgtg ttgccagcgc aacgagaacc ccaacggccc cacggaaggc   1020
tctgaccgct cggcctcctc cctcaaccac actattctgg ctggagttca cagcaatgac   1080
cactctgtgg tt                                                       1092
```

<210> 5
<211> 378
<212> PRT
<213> Homo sapiens

<400> 5

```
Met Ala Thr Ala Leu Pro Pro Arg Leu Gln Pro Val Arg Gly Asn Glu
1               5                   10                  15
Thr Leu Arg Glu His Tyr Gln Tyr Val Gly Lys Leu Ala Gly Arg Leu
            20                  25                  30
Lys Glu Ala Ser Glu Gly Ser Thr Leu Thr Thr Val Leu Phe Leu Val
        35                  40                  45
Ile Cys Ser Phe Ile Val Leu Glu Asn Leu Met Val Leu Ile Ala Ile
    50                  55                  60
Trp Lys Asn Asn Lys Phe His Asn Arg Met Tyr Phe Phe Ile Gly Asn
65                  70                  75                  80
Leu Ala Leu Cys Asp Leu Leu Ala Gly Ile Ala Tyr Lys Val Asn Ile
                85                  90                  95
Leu Met Ser Gly Lys Lys Thr Phe Ser Leu Ser Pro Thr Val Trp Phe
            100                 105                 110
Leu Arg Glu Gly Ser Met Phe Val Ala Leu Gly Ala Ser Thr Cys Ser
        115                 120                 125
Leu Leu Ala Ile Ala Ile Glu Arg His Leu Thr Met Ile Lys Met Arg
    130                 135                 140
Pro Tyr Asp Ala Asn Lys Arg His Arg Val Phe Leu Leu Ile Gly Met
145                 150                 155                 160
Cys Trp Leu Ile Ala Phe Thr Leu Gly Ala Leu Pro Ile Leu Gly Trp
                165                 170                 175
Asn Cys Leu His Asn Leu Pro Asp Cys Ser Thr Ile Leu Pro Leu Tyr
            180                 185                 190
Ser Lys Lys Tyr Ile Ala Phe Cys Ile Ser Ile Phe Thr Ala Ile Leu
        195                 200                 205
Val Thr Ile Val Ile Leu Tyr Ala Arg Ile Tyr Phe Leu Val Lys Ser
    210                 215                 220
```

Ser Ser Arg Lys Val Ala Asn His Asn Asn Ser Glu Arg Ser Met Ala
225 230 235 240
Leu Leu Arg Thr Val Val Ile Val Val Ser Val Phe Ile Ala Cys Trp
245 250 255
Ser Pro Leu Phe Ile Leu Phe Leu Ile Asp Val Ala Cys Arg Val Gln
260 265 270
Ala Cys Pro Ile Leu Phe Lys Ala Gln Trp Phe Ile Val Leu Ala Val
275 280 285
Leu Asn Ser Ala Met Asn Pro Val Ile Tyr Thr Leu Ala Ser Lys Glu
290 295 300
Met Arg Arg Ala Phe Phe Arg Leu Val Cys Asn Cys Leu Val Arg Gly
305 310 315 320
Arg Gly Ala Arg Ala Ser Pro Ile Gln Pro Ala Leu Asp Pro Ser Arg
325 330 335
Ser Lys Ser Ser Ser Ser Asn Asn Ser Ser His Ser Pro Lys Val Lys
340 345 350
Glu Asp Leu Pro His Thr Asp Pro Ser Ser Cys Ile Met Asp Lys Asn
355 360 365
Ala Ala Leu Gln Asn Gly Ile Phe Cys Asn
370 375

<210> 6
<211> 1134
<212> DNA
<213> Homo sapiens

<400> 6
atggcaactg ccctcccgcc gcgtctccag ccggtgcggg ggaacgagac cctgcgggag 60
cattaccagt acgtggggaa gttggcgggc aggctgaagg aggcctccga gggcagcacg 120
ctcaccaccg tgctcttctt ggtcatctgc agcttcatcg tcttggagaa cctgatggtt 180
ttgattgcca tctggaaaaa caataaattt cacaaccgca tgtacttttt cattggcaac 240
ctggctctct gcgacctgct ggccggcatc gcttacaagg tcaacattct gatgtctggc 300
aagaagacgt tcagcctgtc tcccacggtc tggttcctca gggagggcag tatgttcgtg 360
gcccttgggg cgtccacctg cagcttactg gccatcgcca tcgagcggca cttgacaatg 420
atcaaaatga ggccttacga cgccaacaag aggcaccgcg tcttcctcct gatcgggatg 480
tgctggctca ttgccttcac gctgggcgcc ctgcccattc tgggctggaa ctgcctgcac 540
aatctccctg actgctctac catcctgccc ctctactcca agaagtacat tgccttctgc 600
atcagcatct tcacggccat cctggtgacc atcgtgatcc tctacgcacg catctacttc 660
ctggtgaagt ccagcagccg taaggtggcc aaccacaaca actcggagcg gtccatggca 720
ctgctgcgga ccgtggtgat tgtggtgagc gtgttcatcg cctgctggtc cccactcttc 780
atcctcttcc tcattgatgt ggcctgcagg gtgcaggcgt gccccatcct cttcaaggct 840
cagtggttca tcgtgttggc tgtgctcaac tccgccatga acccggtcat ctacacgctg 900
gccagcaagg agatgcggcg ggccttcttc cgtctggtct gcaactgcct ggtcagggga 960
cggggggccc gcgcctcacc catccagcct gcgctcgacc caagcagaag taaatcaagc 1020
agcagcaaca atagcagcca ctctccgaag gtcaaggaag acctgcccca cacagacccc 1080
tcatcctgca tcatggacaa gaacgcagca cttcagaatg ggatcttctg caac 1134

<210> 7
<211> 222
<212> PRT
<213> Rattus spp

<400> 7
Arg Met Tyr Phe Phe Ile Gly Asn Leu Ala Leu Cys Asp Leu Leu Ala
5 10 15
Gly Ile Ala Tyr Lys Val Asn Ile Leu Met Ser Gly Arg Lys Thr Phe
20 25 30
Ser Leu Ser Pro Thr Val Trp Phe Leu Arg Glu Gly Ser Met Phe Val
35 40 45
Ala Leu Gly Ala Ser Thr Cys Ser Leu Leu Ala Ile Ala Ile Glu Arg
50 55 60
His Leu Thr Met Ile Lys Met Arg Pro Tyr Asp Ala Asn Lys Lys His
65 70 75 80
Arg Val Phe Leu Leu Ile Gly Met Cys Trp Leu Ile Ala Phe Ser Leu
85 90 95
Gly Ala Leu Pro Ile Leu Gly Trp Asn Cys Leu Glu Asn Phe Pro Asp
100 105 110
Cys Ser Thr Ile Leu Pro Leu Tyr Ser Lys Lys Tyr Ile Ala Phe Leu

```
        115                 120                 125
Ile Ser Ile Phe Thr Ala Ile Leu Val Thr Ile Val Ile Leu Tyr Ala
    130                 135                 140
Arg Ile Tyr Phe Leu Val Lys Ser Ser Ser Arg Arg Val Ala Asn His
145                 150                 155                 160
Asn Ser Glu Arg Ser Met Ala Leu Leu Arg Thr Val Val Ile Val Val
                165                 170                 175
Ser Val Phe Ile Ala Cys Trp Ser Pro Leu Phe Ile Leu Phe Leu Ile
            180                 185                 190
Asp Val Ala Cys Arg Ala Lys Glu Cys Ser Ile Leu Phe Lys Ser Gln
        195                 200                 205
Trp Phe Ile Met Leu Ala Val Leu Asn Ser Ala Met Asn Pro
    210                 215                 220

<210> 8
<211> 666
<212> DNA
<213> Rattus spp

<400> 8
cgcatgtact ttttcattgg caacttggct ctctgcgacc tgctggccgg catagcctac      60
aaggtcaaca ttctgatgtc cggtaggaag acgttcagcc tgtctccaac agtgtggttc     120
ctcagggagg gcagtatgtt cgtagccctg ggcgcatcca catgcagctt attggccatt     180
gccattgagc ggcacctgac catgatcaag atgaggccgt acgacgccaa caagaagcac     240
cgcgtgttcc ttctgattgg gatgtgctgg ctaattgcct tctcgctggg tgccctgccc     300
atcctgggct ggaactgcct ggagaacttt cccgactgct ctaccatctt gcccctctac     360
tccaagaaat acattgcctt tctcatcagc atcttcacag ccattctggt gaccatcgtc     420
atcttgtacg cgcgcatcta cttcctggtc aagtccagca gccgcagggt ggccaaccac     480
aactccgaga gatccatggc ccttctgcgg accgtagtga tcgtggtgag cgtgttcatc     540
gcctgttggt ccccccttt catcctcttc ctcatcgatg tggcctgcag ggcgaaggag      600
tgctccatcc tcttcaagag tcagtggttc atcatgctgg ctgtcctcaa ctccgccatg     660
aaccca                                                                666

<210> 9
<211> 353
<212> PRT
<213> Homo sapiens

<400> 9
Met Gly Ser Leu Tyr Ser Glu Tyr Leu Asn Pro Asn Lys Val Gln Glu
                5                   10                  15
His Tyr Asn Tyr Thr Lys Glu Thr Leu Glu Thr Gln Glu Thr Thr Ser
            20                  25                  30
Arg Gln Val Ala Ser Ala Phe Ile Val Ile Leu Cys Cys Ala Ile Val
        35                  40                  45
Val Glu Asn Leu Leu Val Leu Ile Ala Val Ala Arg Asn Ser Lys Phe
    50                  55                  60
His Ser Ala Met Tyr Leu Phe Leu Gly Asn Leu Ala Ala Ser Asp Leu
65                  70                  75                  80
Leu Ala Gly Val Ala Phe Val Ala Asn Thr Leu Leu Ser Gly Ser Val
                85                  90                  95
Thr Leu Arg Leu Thr Pro Val Gln Trp Phe Ala Arg Glu Gly Ser Ala
            100                 105                 110
Ser Ile Thr Leu Ser Ala Ser Val Phe Ser Leu Leu Ala Ile Ala Ile
        115                 120                 125
Glu Arg His Val Ala Ile Ala Lys Val Lys Leu Tyr Gly Ser Asp Lys
    130                 135                 140
Ser Cys Arg Met Leu Leu Leu Ile Gly Ala Ser Trp Leu Ile Ser Leu
145                 150                 155                 160
Val Leu Gly Gly Leu Pro Ile Leu Gly Trp Asn Cys Leu Gly His Leu
                165                 170                 175
Glu Ala Cys Ser Thr Val Leu Pro Leu Tyr Ala Lys His Tyr Val Leu
            180                 185                 190
Cys Val Val Thr Ile Phe Ser Ile Ile Leu Leu Ala Ile Val Ala Leu
        195                 200                 205
Tyr Val Arg Ile Tyr Cys Val Val Arg Ser Ser His Ala Asp Met Ala
    210                 215                 220
Ala Pro Gln Thr Leu Ala Leu Leu Lys Thr Val Thr Ile Val Leu Gly
```

```
225                 230                 235                 240
Val Phe Ile Val Cys Trp Leu Pro Ala Phe Ser Ile Leu Leu Leu Asp
                245                 250                 255
Tyr Ala Cys Pro Val His Ser Cys Pro Ile Leu Tyr Lys Ala His Tyr
            260                 265                 270
Phe Phe Ala Val Ser Thr Leu Asn Ser Leu Leu Asn Pro Val Ile Tyr
        275                 280                 285
Thr Trp Arg Ser Arg Asp Leu Arg Arg Glu Val Leu Arg Pro Leu Gln
    290                 295                 300
Cys Trp Arg Pro Gly Val Gly Val Gln Gly Arg Arg Arg Val Gly Thr
305                 310                 315                 320
Pro Gly His His Leu Leu Pro Leu Arg Ser Ser Ser Ser Leu Glu Arg
                325                 330                 335
Gly Met His Met Pro Thr Ser Pro Thr Phe Leu Glu Gly Asn Thr Val
            340                 345                 350
Val
```

<210> 10
<211> 1059
<212> DNA
<213> Homo sapiens

<400> 10

```
atgggcagct tgtactcgga gtacctgaac cccaacaagg tccaggaaca ctataattat      60
accaaggaga cgctggaaac gcaggagacg acctcccgcc aggtggcctc ggccttcatc     120
gtcatcctct gttgcgccat tgtggtggaa aaccttctgg tgctcattgc ggtggcccga     180
aacagcaagt tccactcggc aatgtacctg tttctgggca acctggccgc ctccgatcta     240
ctggcaggcg tggccttcgt agccaatacc ttgctctctg gctctgtcac gctgaggctg     300
acgcctgtgc agtggtttgc ccgggagggc tctgcctcca tcacgctctc ggcctctgtc     360
ttcagcctcc tggccatcgc cattgagcgc cacgtggcca ttgccaaggt caagctgtat     420
ggcagcgaca agagctgccg catgcttctg ctcatcgggg cctcgtggct catctcgctg     480
gtcctcggtg gcctgcccat ccttggctgg aactgcctgg gccacctcga ggcctgctcc     540
actgtcctgc ctctctacgc caagcattat gtgctgtgcg tggtgaccat cttctccatc     600
atcctgttgg ccatcgtggc cctgtacgtg cgcatctact gcgtggtccg ctcaagccac     660
gctgacatgg ccgccccgca gacgctagcc ctgctcaaga cggtcaccat cgtgctaggc     720
gtctttatcg tctgctggct gcccgccttc agcatcctcc ttctggacta tgcctgtccc     780
gtccactcct gcccgatcct ctacaaagcc cactactttt tcgccgtctc caccctgaat     840
tccctgctca accccgtcat ctacacgtgg cgcagccggg acctgcggcg ggaggtgctt     900
cggccgctgc agtgctggcg gccgggggtg ggggtgcaag gacggaggcg ggtcggggacc     960
ccgggccacc acctcctgcc actccgcagc tccagctccc tggagagggg catgcacatg    1020
cccacgtcac ccacgtttct ggagggcaac acggtggtc                           1059
```

<210> 11
<211> 352
<212> PRT
<213> Rattus spp

<400> 11

```
Met Gly Gly Leu Tyr Ser Glu Tyr Leu Asn Pro Glu Lys Val Gln Glu
                5                   10                  15
His Tyr Asn Tyr Thr Lys Glu Thr Leu Asp Met Gln Glu Thr Pro Ser
            20                  25                  30
Arg Lys Val Ala Ser Ala Phe Ile Ile Ile Leu Cys Cys Ala Ile Val
        35                  40                  45
Val Glu Asn Leu Leu Val Leu Ile Ala Val Ala Arg Asn Ser Lys Phe
    50                  55                  60
His Ser Ala Met Tyr Leu Phe Leu Gly Asn Leu Ala Ala Ser Asp Leu
65                  70                  75                  80
Leu Ala Gly Val Ala Phe Val Ala Asn Thr Leu Leu Ser Gly Pro Val
                85                  90                  95
Thr Leu Ser Leu Thr Pro Leu Gln Trp Phe Ala Arg Glu Gly Ser Ala
            100                 105                 110
Phe Ile Thr Leu Ser Ala Ser Val Phe Ser Leu Leu Ala Ile Ala Ile
        115                 120                 125
Glu Arg Gln Val Ala Ile Ala Lys Val Lys Leu Tyr Gly Ser Asp Lys
    130                 135                 140
Ser Cys Arg Met Leu Met Leu Ile Gly Ala Ser Trp Leu Ile Ser Leu
145                 150                 155                 160
```

```
Ile Leu Gly Gly Leu Pro Ile Leu Gly Trp Asn Cys Leu Asp His Leu
                165                 170                 175
Glu Ala Cys Ser Thr Val Leu Pro Leu Tyr Ala Lys His Tyr Val Leu
            180                 185                 190
Cys Val Val Thr Ile Phe Ser Val Ile Leu Leu Ala Ile Val Ala Leu
        195                 200                 205
Tyr Val Arg Ile Tyr Phe Val Val Arg Ser Ser His Ala Asp Val Ala
    210                 215                 220
Gly Pro Gln Thr Leu Ala Leu Leu Lys Thr Val Thr Ile Val Leu Gly
225                 230                 235                 240
Val Phe Ile Ile Cys Trp Leu Pro Ala Phe Ser Ile Leu Leu Leu Asp
                245                 250                 255
Ser Thr Cys Pro Val Arg Ala Cys Pro Val Leu Tyr Lys Ala His Tyr
            260                 265                 270
Phe Phe Ala Phe Ala Thr Leu Asn Ser Leu Leu Asn Pro Val Ile Tyr
        275                 280                 285
Thr Trp Arg Ser Arg Asp Leu Arg Arg Glu Val Leu Arg Pro Leu Leu
    290                 295                 300
Cys Trp Arg Gln Gly Lys Gly Ala Thr Gly Arg Arg Gly Gly Asn Pro
305                 310                 315                 320
Gly His Arg Leu Leu Pro Leu Arg Ser Ser Ser Ser Leu Glu Arg Gly
                325                 330                 335
Leu His Met Pro Thr Ser Pro Thr Phe Leu Glu Gly Asn Thr Val Val
            340                 345                 350

<210> 12
<211> 1056
<212> DNA
<213> Rattus spp

<400> 12
atgggcggtt tatactcaga gtacctcaat cctgagaagg ttcaggaaca ctacaattac      60
accaaggaga cgctggacat gcaggagacg ccctcccgca aggtggcctc cgccttcatc     120
atcattttat gctgtgccat cgtggtggag aaccttctgg tgctaatcgc agtggccagg     180
aacagcaagt tccactcagc catgtacctg ttcctcggca acctggcagc ctccgacctg     240
ctggcaggcg tggccttcgt ggccaacacc ttgctctccg gacctgtcac cctgtcctta     300
actcccttgc agtggtttgc ccgagagggt tcagccttca tcacgctctc tgcctcggtc     360
ttcagcctcc tggccattgc catcgagaga caagtggcca tcgccaaggt caagctctac     420
ggcagtgaca aaagctgtcg aatgttgatg ctcattgggg cctcttggct gatatcgctg     480
attctgggtg gcttgcccat cctgggctgg aattgtctgg accatctgga ggcttgctcc     540
actgtgctgc ccctctatgc taagcactat gtgctctgcg tggtcaccat cttctctgtc     600
atcttactgg ctatcgtggc cttgtacgtc cgaatctact tcgtagtccg ctcaagccat     660
gcggacgttg ctggtcctca gacgctggcc ctgctcaaga cagtcaccat cgtactgggt     720
gttttcatca tctgctggct gccggctttt agcatccttc tcttagactc tacctgtccc     780
gtccgggcct gtcctgtcct ctacaaagcc cattatttct ttgccttcgc caccctcaac     840
tctctgctca accctgtcat ctatacatgg cgtagccggg accttcggag ggaggtactg     900
aggcccctgc tgtgctggcg gcaggggaag ggagcaacag ggcgcagagg tgggaaccct     960
ggtcaccgac tcctgcccct ccgcagctcc agctccctgg agagaggctt gcatatgcct    1020
acatcgccaa catttctgga gggcaacaca gtggtc                              1056

<210> 13
<211> 22
<212> DNA
<213> Artificial Sequence

<220>
<223> Primer

<400> 13
ccaccgaccc atgtactatt tt                                                22

<210> 14
<211> 22
<212> DNA
<213> Artificial Sequence

<220>
<223> Primer
```

<400> 14
tgtaggctac tcctgccaac ag 22

<210> 15
<211> 22
<212> DNA
<213> Artificial Sequence

<220>
<223> Probe, labeled 5'-terminal with FAM and 3'-terminal with TAMRA

<400> 15
ttggcaatct ggccctctca ga 22

<210> 16
<211> 21
<212> DNA
<213> Artificial Sequence

<220>
<223> Primer

<400> 16
actgtcagca catggctcct t 21

<210> 17
<211> 21
<212> DNA
<213> Artificial Sequence

<220>
<223> Primer

<400> 17
accgtaatgt gcctctcgat t 21

<210> 18
<211> 22
<212> DNA
<213> Artificial Sequence

<220>
<223> Probe, labeled 5'-terminal with FAM and 3'-terminal with TAMRA

<400> 18
attgacacca gcctgacggc at 22

<210> 19
<211> 19
<212> DNA
<213> Artificial Sequence

<220>
<223> Primer

<400> 19
ccgtgctctt cttggtcat 19

<210> 20
<211> 19
<212> DNA
<213> Artificial Sequence

<220>
<223> Primer

<400> 20

ccagatggca atcaaaacc 19

<210> 21
<211> 26
<212> DNA
<213> Artificial Sequence

<220>
<223> Probe, labeled 5'-terminal with FAM and 3'-terminal with TAMRA

<400> 21
tgcagcttca tcgtcttgga gaacct 26

<210> 22
<211> 22
<212> DNA
<213> Artificial Sequence

<220>
<223> Primer

<400> 22
cctggtcaag actgttgtca tc 22

<210> 23
<211> 19
<212> DNA
<213> Artificial Sequence

<220>
<223> Primer

<400> 23
caggacattg caggactca 19

<210> 24
<211> 27
<212> DNA
<213> Artificial Sequence

<220>
<223> Probe, labeled 5'-terminal with FAM and 3'-terminal with TAMRA

<400> 24
tggtactgct cctggatggt ttaggct 27

<210> 25
<211> 19
<212> DNA
<213> Artificial Sequence

<220>
<223> Primer

<400> 25
ccaacaaggt ccaggaaca 19

<210> 26
<211> 19
<212> DNA
<213> Artificial Sequence

<220>
<223> Primer

<400> 26
aggttttcca ccacaatgg 19

<210> 27
<211> 28
<212> DNA
<213> Artificial Sequence

<220>
<223> Probe, labeled 5'-terminal with FAM and 3'-terminal with TAMRA

<400> 27
aattatacca aggagacgct ggaaacgc 28

<210> 28
<211> 19
<212> DNA
<213> Artificial Sequence

<220>
<223> Primer

<400> 28
gaactgcctg tgcgccttt 19

<210> 29
<211> 20
<212> DNA
<213> Artificial Sequence

<220>
<223> Primer

<400> 29
ccatagaggc ccatgatggt 20

<210> 30
<211> 28
<212> DNA
<213> Artificial Sequence

<220>
<223> Probe, labeled 5'-terminal with FAM and 3'-terminal with TAMRA

<400> 30
tctgcccctc tactccaagc gctacatc 28

<210> 31
<211> 19
<212> DNA
<213> Artificial Sequence

<220>
<223> Primer

<400> 31
tgactgcttc cctcaccaa 19

<210> 32
<211> 22
<212> DNA
<213> Artificial Sequence

<220>
<223> Primer

<400> 32
gcatcctcat gattgacatg tg 22

<210> 33
<211> 22

<212> DNA
<213> Artificial Sequence

<220>
<223> Probe, labeled 5'-terminal with FAM and 3'-terminal with TAMRA

<400> 33
ttgctggtta tcgccgtgga ga 22

<210> 34
<211> 19
<212> DNA
<213> Artificial Sequence

<220>
<223> Primer

<400> 34
cttgctccac tgtcttgcc 19

<210> 35
<211> 19
<212> DNA
<213> Artificial Sequence

<220>
<223> Primer

<400> 35
tagagtgcac agatcgcgg 19

<210> 36
<211> 28
<212> DNA
<213> Artificial Sequence

<220>
<223> Probe, labeled 5'-terminal with FAM and 3'-terminal with TAMRA

<400> 36
ctctacgcca aggcctacgt gctcttct 28

<210> 37
<211> 25
<212> DNA
<213> Artificial Sequence

<220>
<223> Primer

<400> 37
tgtccctaga cccaagagac tttag 25

<210> 38
<211> 21
<212> DNA
<213> Artificial Sequence

<220>
<223> Primer

<400> 38
ggtccccttc tcttttccaa a 21

<210> 39
<211> 28
<212> DNA
<213> Artificial Sequence

<220>
<223> Probe, labeled 5'-terminal with FAM and 3'-terminal with TAMRA

<400> 39
atgaacttgc ttggtagccc ccatcttc 28

<210> 40
<211> 20
<212> DNA
<213> Artificial Sequence

<220>
<223> Primer

<400> 40
atcttgtacg cgcgcatcta 20

<210> 41
<211> 22
<212> DNA
<213> Artificial Sequence

<220>
<223> Primer

<400> 41
tggatctctc ggagttgtgg tt 22

<210> 42
<211> 22
<212> DNA
<213> Artificial Sequence

<220>
<223> Probe, labeled 5'-terminal with FAM and 3'-terminal with TAMRA

<400> 42
tggtcaagtc cagcagccgc ag 22

<210> 43
<211> 19
<212> DNA
<213> Artificial Sequence

<220>
<223> Primer

<400> 43
gtttgcccga gagggttca 19

<210> 44
<211> 21
<212> DNA
<213> Artificial Sequence

<220>
<223> Primer

<400> 44
cttgtctctc gatggcaatg g 21

<210> 45
<211> 27
<212> DNA
<213> Artificial Sequence

<220>

```
<223> Probe, labeled 5'-terminal with FAM and 3'-terminal with TAMRA

<400> 45
cttcatcacg ctctctgcct cggtctt                        27
```